(19) **Europäisches Patentamt European Patent Office Office européen des brevets**

(11) **EP 2 441 464 B1**

(12) **EUROPEAN PATENT SPECIFICATION**

(45) Date of publication and mention of the grant of the patent:
**09.04.2014 Bulletin 2014/15**

(51) Int Cl.:
*A61K 38/12* (2006.01) *A61K 31/282* (2006.01)
*A61K 31/704* (2006.01) *A61K 31/519* (2006.01)
*A61K 31/475* (2006.01) *A61K 39/395* (2006.01)
*A61K 45/06* (2006.01) *A61P 35/00* (2006.01)

(21) Application number: **11009510.6**

(22) Date of filing: **17.01.2008**

(54) **INTEGRIN LIGANDS FOR USE IN TREATING COLON CANCER**

INTEGRIN-LIGANDEN ZUR VERWENDUNG IN DER BEHANDLUNG VON DARMKREBS

LIGANDS D'INTÉGRINE POUR LEUR UTILISATION DANS LE TRAITEMENT DU CANCER DU COLON

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **18.01.2007 PCT/US2007/001446**
**18.07.2007 EP 07014070**

(43) Date of publication of application:
**18.04.2012 Bulletin 2012/16**

(62) Document number(s) of the earlier application(s) in accordance with Art. 76 EPC:
**08707093.4 / 2 101 805**

(73) Proprietor: **Merck Patent GmbH**
**64293 Darmstadt (DE)**

(72) Inventors:
• **Krueger, Stefan**
  **65824 Schwalbach/Taunus (DE)**
• **Goodman, Simon**
  **64347 Griesheim (DE)**
• **Harstrick, Andreas**
  **65329 Hohenstein (DE)**
• **Picard, Martin Andreas**
  **64291 Darmstadt (DE)**
• **Nippgen, Johannes**
  **64297 Darmstadt (DE)**
• **Grimm, Ulrike**
  **83646 Wackersberg (DE)**
• **Stupp, Roger**
  **1012 Lausanne (CH)**
• **Weller, Michael**
  **8708 Maennedorf (CH)**

(56) References cited:
  WO-A-02/055106    WO-A-02/085405
  WO-A-2004/000344

• **LODE H N ET AL: "Synergy between an antiangiogenic integrin alphav antagonist and an antibody-cytokine fusion protein eradicates spontaneous tumor metastases", PROCEEDINGS OF THE NATIONAL ACADEMY OF SCIENCES OF USA, NATIONAL ACADEMY OF SCIENCE, WASHINGTON, DC, vol. 96, no. 4, 16 February 1999 (1999-02-16), pages 1591-1596, XP002134006, ISSN: 0027-8424**
• **BURKE P A ET AL: "Cilengitide Targeting of alphavbeta3 Integrin Receptor Synergizes with Radioimmunotherapy to Increase Efficacy and Apoptosis in Brest Cancer Xenografts1", CANCER RESEARCH, AMERICAN ASSOCIATION FOR CANCER RESEARCH, BALTIMORE, MD, vol. 62, no. 15, 1 August 2002 (2002-08-01), pages 4263-4272, XP002903574, ISSN: 0008-5472**

## Description

### Technical Field of the Invention:

The invention relates to a specific therapy form for the treatment of cancer, especially tumors (or tumours) and tumor metastases, comprising administration of integrin ligands together with cancer cotherapeutic agents or other cancer cotherapeutic therapy forms that have additive or synergistic efficacy when administered together with said integrin ligand, such as chemotherapeutic agents, immunotherapeutics, including antibodies, radioimmunoconjugates and immunocytokines and or radiation therapy. More specifically, the instant invention relates to the use of at least one specific integrin ligand for the manufacture of a medicament for the treatment of cancer, wherein the medicament is to be used in combination with

> a) one or more alkylating chemotherapeutic agents, and
> b) one or more further chemotherapeutic agents other than the at least one specific integrin ligand and the one or more alkylating chemotherapeutic agents. Additionally, the instant invention relates to methods of treatment, using said medicament. Preferably, the administration of the medicament can be done in a timely controlled manner. The therapy will result in a synergistic potential increase of the inhibition effect of each individual therapeutic on tumor cell and tumor endothelial cell proliferation, preferably yielding a more effective treatment than found by administering an individual component alone, together or in another therapy regime but the regime of the present invention.

### Background of the Invention:

Vascular endothelial cells are known to contain at least three RGD-dependent integrins, including the vitronectin receptors $\alpha_v\beta_3$ or $\alpha_v\beta_5$ as well as the collagen types I and IV receptors $\alpha_v\beta_1$ and $\alpha_2\beta_1$, the laminin receptors $a_{aJ}3_1$ and $\alpha_3\beta_1$, and the fibronectin receptor $\alpha_5\beta_1$ (Davis et al., 1993, J. Cell. Biochem. 51, 206). The smooth muscle cell is known to contain at least six RGD-dependent integrins, including $\alpha_v\beta_3$ and $\alpha_v\beta_5$.

Inhibition of cell adhesion in vitro using monoclonal antibodies immunospecific for various integrin $\alpha$ or $\beta$ subunits have implicated the vitronectin receptor $\alpha_v\beta_3$ in cell adhesion processes of a variety of cell types including microvascular endothelial cells (Davis et al., 1993, J. Cell. Biol. 51, 206).

Integrins are a class of cellular receptors known to bind extracellular matrix proteins, and mediate cell-extracellular matrix and cell-cell interactions, referred generally to as cell adhesion events. The integrin receptors constitute a family of proteins with shared structural characteristics of non-covalenty associated heterodimeric glycoprotein complexes formed of $\alpha$ and $\beta$ subunits. The vitronectin receptor, named for its original characteristic of preferential binding to vitronectin, is now known to refer to four different integrins, designated $\alpha_v\beta_1$, $\alpha_v\beta_3$, $\alpha_v\beta_5$ and $\alpha_v\beta_8$. $\alpha_v\beta_1$ binds fibronectin and vitronectin. $\alpha_v\beta_3$ binds a large variety of ligands, including fibrin, fibrinogen, laminin, thrombospondin, vitronectin and von Willebrand's factor. $\alpha_v\beta_5$ binds vitronectin. It is clear that there are different integrins with different biological functions as well as different integrins and subunits having shared biological specificity and function. One important recognition site in a ligand for many integrins is the Arg-Gly-Asp (RGD) tripeptide sequence. RGD is found in all of the ligands identified above for the vitronectin receptor integrins. The molecular basis of RGD recognition by $\alpha_v\beta_3$ has been identified (Xiong *et al.,* 2001) This RGD recognition site can be mimicked by linear and cyclic (poly)peptides that contain the RGD sequence. Such RGD peptides are known to be inhibitors or antagonists, respectively, of integrin function. It is important to note, however, that depending upon the sequence and structure of the RGD peptide, the specificity of the inhibition can be altered to target specific integrins. Various RGD polypeptides of varying integrin specificity have been described, for example, by Cheresh, et al., 1989, Cell 58, 945, Aumailley et al., 1991, FEBS Letts. 291, 50, and in numerous patent applications and patents (e.g. US patents 4,517,686, 4,578,079, 4,589,881, 4, 614, 517, 4, 661,111, 4, 792, 525; EP 0770 622).

The generation of new blood vessels, or angiogenesis, plays a key role in the growth of malignant disease and this has generated much interest in developing agents that inhibit angiogenesis.

Nevertheless, although various combination therapies utilizing potential angiogenesis inhibitors are under investigation, in clinical trials and on the market, the outcome of these therapies are not fruitful enough. Therefore, there still exists a need in the art to develop further combinations which can show increased efficacy and reduced side-effects.

It is known today that tumor vasculature is different from vasculature of healthy tissue. The vasculature is characteristic for the tumor and distinct from the stable, dormant vasculature of healthy tissue. It is often characterized by an increased expression and priming of specific cell adhesion molecules of the alpha-v-integrin series, especially $\alpha_v\beta_3$ and $\alpha_v\beta_5$. When activated these integrins enhance the cellular response to growth factors that drive angiogenesis, for example VEGFA and FGF2: VEGFA was originally termed vascular permeability factor, and it acts via the SRC kinase pathway to increase local vascular permeability. VEGRF2, when activated, increases the activity of $\alpha_v\beta_3$ integrin.

Further, solid tumors depend on an induced and cooped vasculature from the host to develop. This vasculature has unusual molecular properties that distinguish it from the normal host vasculature: it tends to be activated, i.e. progressing through cell cycle under the influence of tumor-derived factors like VEGFs, FGFs and others, and expresses endothelial activation markers like ICAM, VCAM and alpha-v-series Integrins, e.g. $\alpha_v\beta_3$ and $\alpha_v\beta_5$, in a ligand competent state. It has a defective extracellular matrix, and is classically described as leaky. It is notable that tumors often resist therapies systemically applied via the blood stream, due to abnormal nature of tumor vasculature.

The metastatic process is a multistep event and represents the most dreadful aspect of cancer. At the moment of diagnosis, cancers are frequently far advanced in their natural history, and the presence of metastases is a common event. In fact, approximately 30% of patients have detectable metastases at the moment of clinical diagnosis and a further 30% of patients have occult metastases. Metastases can be disseminated and they can infest different organs at the same time, or localize to a specific organ. In the case of localized disease, surgery is the treatment of choice; however recurrence and prognosis depend on many criteria such as: resectability, patient's clinical situation, and number of metastases.

After resection, recurrence is common, suggesting that micrometastatic foci are present at the moment of diagnosis. Systemic chemotherapy is an ideal setting but,only few patients are cured by it, and in the majority systemic chemotherapy fails. Many physiological barriers and pharmacokinetic parameters contribute to decrease its efficacy.

Liver, lungs and lymph nodes are filtration organs and therefore inclined to metastasization. The poor chemosensitivity of metastases, peculiarly those of colorectal origin has forced many researchers to use methods for increasing the time and the concentration of drugs. The need for decreasing or limiting the side effects for this important and delicate organ led to the development of the technique of liver isolation for perfusion of antineoplastic agents. (K. R. Aigner, Isolated liver perfusion. In: Morris DL, McArdle CS, Onik GM, eds. Hepatic Metastases. Oxford: Butterworth Heinemann, 1996. 101-107). Since 1981, modifications and technical improvements have been continuously introduced. Liver metastases may be of different origin and their chemosensitivity may vary according to the histological type and their response in presence of heat.

There still exists a growing need in the art in order to develop new therapeutic strategies for treating cancer, especially metastases systemically. The object of the present invention therefore was to develop such a new strategy. It should be applicable to systemic treatment, and it should lower the dose and/or increase the efficiency of the cancer therapeutical agents to be applied. A further object was to normalize tumor vasculature to increase delivery of systemic therapeutics of tumor, i.e. to reset the tumor vasculature to the functionality of the vasculature of non-tumor tissue.

Thus, it is a preferred objective of the instant invention to provide a more effective, better tolerated treatment for cancer patients leading to enhanced progression-free survival (PFS), QOL and increased median survival.

**Brief description of drawings:**

[For technical information only]:

Fig. 1 shows the results from the rat orthotopic glioblastoma model radiotherapy, cilengitide scheduling experiments. The results are also shown in Table 1.

[For technical information only]:

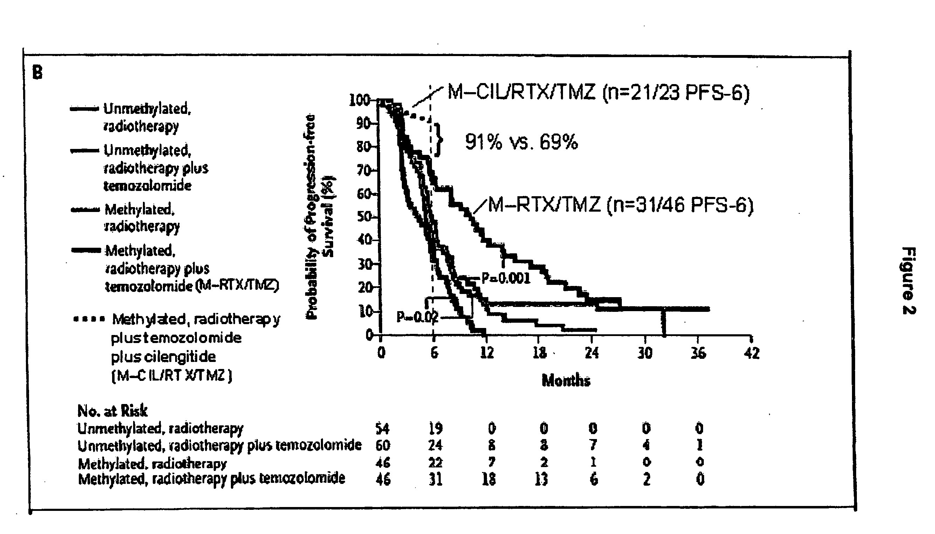

Fig. 2 shows the results of a clinical study in glioblastoma (GBM). The results are also shown in Example 3.

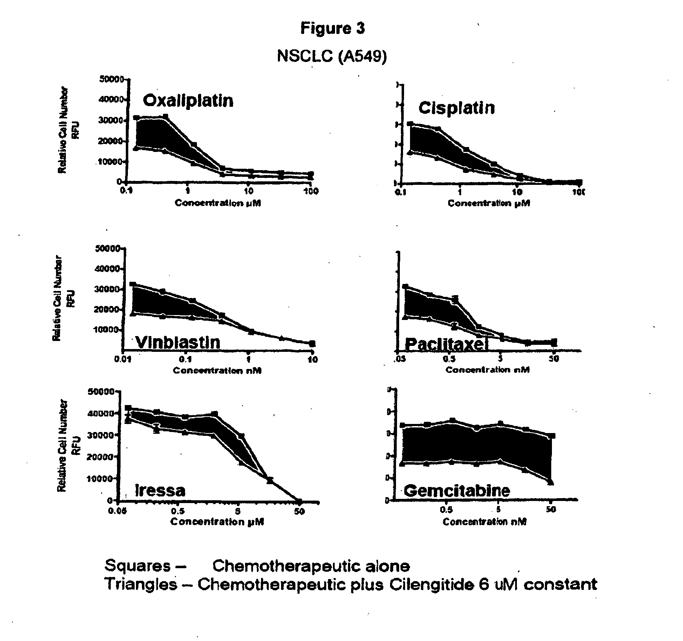

Fig. 3 and 4 shows the results of proliferation assays according to example 4.

**Summary of the Invention:**
The present invention is defined by the claims. The present inventions relates to a novel pharmaceutical treatment which is based on the new concept in tumor therapy to administer to an individual in a therapeutically effective amount a specific integrin ligand in combination with one or more specified chemotherapeutic agents and/or cancer cotherapeutic agents as described herein. Advantagously, this can be done according to the regimens as described herein.

Thus, subject of the instant invention is the use of at least one specific integrin ligand comprising cyclo-(Arg-Gly-Asp-DPhe-NMe-Val), the pharmaceutically acceptable derivatives, solvates and/or salts thereof, for use in the treatment of colon and/or rectal cancer in combination with

a) one or more alkylating chemotherapeutic agents, selected from the group consisting of the platinum containing compounds cisplatin, carboplatin and oxaliplatin, and
b) one or more further chemotherapeutic agents other than the at least one specific integrin ligand and the one or more alkylating chemotherapeutic agents, selected from the group consisting of:

i) EGFR inhibitors, selected from the group consisting of cetuximab, panitumumab, zalutumumab, nimotuzumab and matuzumab and/or the group consisting of gefitinib, erlotinib and lapatinib,

ii) cytostatic alkaloids, selected from the group consisting of etoposide, vinblastine and teniposide, the group consisting of vinorelbine, vincristine and vindesine, the group consisting of docetaxel and paclitaxel, and/or the group consisting of irinotecan and topotecan,

iii) cytostatic antibiotics, selected from the group consisting of doxorubicin, idarubicin, daunorubicin, epirubicin and valrubicin, and

iv) antimetabolites, selected from the group consisting of 5-fluorouracil, capecitabine, cytosinarabinosid and difluorodesoxycytidin and/or the group consisting of pemetrexed, methotrexat and raltitrexed,

and pharmaceutically acceptable derivatives, salts and/or solvates thereof, and methods of treating cancer using said medicament.

Surprisingly, it can be shown that the tumor vasculature can be functionally normalized by systemically applied integrin ligands as defined herein. Such inhibitors of integrin functions, also referred to as integrin ligands in the context of the present invention, increase the amount of cytotoxics and cytostatics, such as chemotherapeutic agents and/or cancer cotherapeutic agents as described herein, entering the tumor. In addition, the specific integrin ligand can be shown to enhance the numbers of leukocytes entering the tumor following systemic immunocytokines therapy, and may directly or indirectly increase the amounts of antibodies entering the tumor compartment on anti-tumor antibody therapy, or increase access to anti-tumor vaccines. Furthermore, it is believed that this functional normalization of the tumor vasculature will lead to changes in the metabolism of the tumor, e.g. a higher oxygen concentration in the tumor, and thus allows oxygen dependent therapies, like external beam radiotherapy, to become more effective.

The "functional normalizing agent" of the present invention is defined here empirically as a reagent targeting alpha-v-integrins within the tumor compartment that increases the levels of systemic tumor therapeutics or of specific bio-indicators of a systemic therapy within the tumor. The increased local therapeutic overcomes tumor resistance mechanisms, and enhances therapeutic index. For example, the systemic therapeutic might be a classical chemotherapeutic reagent, an immunocytokines, a immunotoxin, or a radioimmunotherapy etc. etc.

In one instance the present disclosure relates to a composition comprising as the cotherapeutic agent therapeutically active compounds, preferably selected from the group consisting of cytotoxic agents, chemotherapeutic agents and immunotoxic agents, and as the case may be other pharmacologically active compounds which may enhance the efficacy of said agents or reduce the side effects of said agents.

The present invention relates to pharmaceutical compositions comprising an integrin ligand, namely the cyclic peptide cyclo-(Arg-Gly-Asp-DPhe-NMe-Val), as well as the pharmaceutically acceptable dervatives, solvates and/or salts thereof.

According to the present specification therapeutically active compositions may also be provided by means of a pharmaceutical kit comprising a package comprising one or more of the said integrin ligands, and one or more cancer cotherapeutic agents, preferably as described herein, e.g. cytotoxic and/or chemotherapeutic and/or immunotoxic agents, in single packages or in separate containers. The therapy with these combinations may include optionally further treatment with radiation.

The present disclosure relates furthermore to a new therapy form comprising the administration of an integrin ligand prior to radiotherapy.

In this new therapy form comprising the administration of an integrin ligand prior to radiotherapy, it is a preferred feature that the integrin ligand is administered prior to the further cancer cotherapeutic agent. In this context, radiation, or, radiotherapy has to be understood as a cancer cotherapeutic agent.

Generally, this prior application takes place 1 to 8 hours (h), preferably 1 to 5 h, and more preferably 1 to 3 h before the application of the further cancer cotherapeutic agent. Even more preferably, this prior application takes place 2 to 8 hours (h), preferably 2 to 6 h, and more preferably 2 to 4 h before the application of the further cancer cotherapeutic agent, such as 1 to 2 h, 2 to 3 h, 3 to 6 h, 2 to 5 h or 3 to 7 h before the application of the further cancer therapeutic agent. In this context, this prior application or administration is also referred to as "timed administration" or "timed application".

As is shown by the data contained in this application, the effect is achieved in non-human animals, especially rats, if this prior application preferably takes place 1 to 8 hours (h), preferably 1 to 5 h, and more preferably 1 to 3 h before the application of the further cancer cotherapeutic agent; and even more preferably this prior application takes place 2 to 8 hours (h), preferably 2 to 6 h, and more preferably 2 to 4 h before the application of the further cancer cotherapeutic agent, such as 1 to 2 h, 2 to 3 h, 3 to 6 h, 2 to 5 h or 3 to 7 h before the application of the further cancer therapeutic agent. In this context, this prior application or administration is also referred to as "timed administration" or "timed application".

However, the data from experiments with human animals preferably shows that the time of the above/below described and discussed "prior application" can be delayed or multiplied by the factor 1 to 4 and especially 2 to 4. This difference in the response or response time between non-human animals, especially rodents, such as rats, and human animals is known and extensively discussed in the art. While the applicant wishes not to be bound by this theory, he believes that this difference is at least in part caused by the different pharmacokinetic behavior of the different species, which i. a. reflects in different halflives ($t_{1/2}$) in the different kinds of animals. For example, for compounds such as cyclopeptides, the halflives in rats usually are in the range of 10-30 minutes, whereas the halflives in human animals for the same compounds are within 2 to 6 hours and especially 3 to 4 hours.

Accordingly, a subject of this application is the claimed composition for use in the treatment and/or a method of manufacture as described above/below, wherein the prior application preferably takes place 1 to 32 hours (h), preferably 2 to 32 h, more preferably 2 to 24 h, even more preferably 4 to 24 h, even more preferably 6 to 20 h and especially 6 to 16 h, before the application of the further cancer cotherapeutic agent; or alternatively preferably this prior application takes place 6 to 32 hours (h), preferably 10 to 24 h, and more preferably 12 to 20 h before the application of the further cancer cotherapeutic agent. With respect to the present disclosure, this prior application or administration is also referred to as "timed administration" or "timed application"

A further subject of this application is the claimed composition for use in the treatment and/or a method of manufacture as described above/below, wherein the prior application preferably takes place 18 to 23 h hours (h), preferably 20 to 23 h, more preferably 20 to 22 h before the application of the further cancer cotherapeutic agent; or alternatively preferably this prior application takes place 25 to 32 h hours (h), preferably 25 to 30 h, and more preferably 26 to 30 h before the application of the further cancer cotherapeutic agent. With respect to the present disclosure, this prior application or administration is also referred to as "timed administration" or "timed application"

However, in a more preferred aspect of the present disclosure, the timed administration (regardless of whether the patient is a human or nonhuman animal) of the specific integrin ligand takes place 1 to 10 hours (h), preferably 2 to 8 h, more preferably 2 to 6 h, even more preferably 3 to 8 h, even more preferably 3 to 6 h and especially 4 to 8 h prior to the application of the one or more cancer cotherapeutic agents, e.g. 1 to 2 h, 1 to 3 h, 1 to 4 h, 2 to 3 h, 2 to 4h, 2 to 6h, 2 to 8h, 2 to 10h, 3 to 4h, 3 to 10h, 4 to 6h, 4 to 10h, 5 to 8 or 5 to 10 h. This is especially preferred if the one or more cancer cotherapeutic agents comprise external beam radiation or consist of external beam radiation. With respect to the present disclosure, this prior application or administration is also referred to as "timed administration" or "timed application".

With respect to said timed administration or timed application (of the specific integrin ligand), the hours given for said prior administration or application preferably refer to the beginning or start of the respective administration or application. Accordingly, for example, an administration of the specific integrin ligand starting three hours before the application of the respective cancer cotherapeutic agent is to be regarded as a timed administration or timed application 3 h prior to the application of the one or more cancer cotherapeutic agents according to the invention, even if the specific integrin ligand is administered by i. v. Infusion that takes an hour or two hours to be completed. This definition of prior application/prior administration is in perfect concordance with the understanding of the ones skilled in the art.

If the at least one specific integrin ligand is administered to the patient in a timed administration as described herein, it is preferably timed with respect to the one or more cancer cotherapeutic agents it is combined with. With respect to the timed administration of the specific integrin ligand in combination with two or more cancer cotherapeutic agents, it is preferably timed with respect to the two or more cancer cotherapeutic agents, more preferably timed with respect to at least one of the cancer cotherapeutic agents. If the one or more cancer cotherapeutic agents comprise radiotherapy, especially radiotherapy as described herein, the timed administration preferably refers at least to the radiotherapy.

Especially preferably, the timed administration of the specific integrin ligand refers to radiotherapy as the time-relevant cancer cotherapeutic. Accordingly, in the timed administration, the prior administration of the specific integrin ligand preferably refers to a time prior to the administration of radiotherapy. However, in many cases, it can be advantageous also to administer the one or more further cancer cotherapeutic agents other than radiotherapy within the time window given by the timed administration of the specific integrin ligand and the administration or delivery of the radiotherapy.

More preferably, the timed administration of the specific integrin ligand refers to the administration of the specific integrin ligand and the radiotherapy, and the additional cancer cotherapeutic agent is preferably administered after the administration of the specific integrin ligand, such as 1 to 2 or 1 to 3 hours after the administration of this specific integrin ligand, but preferably before the administration or delivery of the radiotherapy, preferably at least within one hour before the administration or delivery of the radiotherapy, and more preferably at least 1 hour before radiotherapy, for example 1 to 2 or 1 to 3 h prior to the administration or delivery of the radiotherapy.

If two or more specific integrin ligands are administered in a timed administration as described herein, the timed and administration preferably refers at least to one of the specific integrin ligands and more preferably to the two or more specific integrin ligands to be administered in the timed administration as described herein.

It should be understood that the administration of any combination for use according to the present invention can optionally be accompanied by radiation therapy, wherein radiation treatment can preferably be done after the administration of the integrin ligand. The administration of the different agents of the combination therapy according to the invention can optionally also be achieved substantially concurrently or sequentially.

It is known that tumors elicit alternative routes for their development and growth. If one route is blocked they often have the capability to switch to another route by expressing and using other receptors and signaling pathways. Therefore, the pharmaceutical combinations for use according to the present invention may block several of such possible development strategies of the tumor and provide consequently various therapeutic benefits. The combinations for use according to the present invention are useful in treating and preventing tumors, tumor-like and neoplasia disorders and tumor metastases, which develop and grow by activation of their relevant hormone receptors which are present on the surface of the tumor cells.

Preferably, the different combined agents of the present invention are administered at a low dose, that is, at a dose lower than has been conventionally used in clinical situations. A benefit of lowering the dose of the compounds, compositions, agents and therapies of the present invention administered to an individual includes a decrease in the incidence of adverse effects associated with higher dosages. For example, by the lowering the dosage of an agent described above and below, a reduction in the frequency and the severity of nausea and vomiting will result when compared to that observed at higher dosages. By lowering the incidence of adverse effects, an improvement in the quality of life of a cancer patient is expected. Further benefits of lowering the incidence of adverse effects include an improvement in patient compliance, a reduction in the number of hospitalizations needed for the treatment of adverse effects, and a reduction in the administration of analgesic agents needed to treat pain associated with the adverse effects. Alternatively, the combination for use according to the present invention can also maximize the therapeutic effect at higher doses.

Tumors, preferably showing an increased expression and priming of specific cell adhesion molecules of the alpha-v-integrin series, especially $\alpha_v\beta_3$ and $\alpha_v\beta_5$ in their vasculature may be successfully treated by the combinations and therapeutic regimen according to the invention. The combinations within the pharmaceutical treatment according to the invention show an astonishing synergetic effect. In administering the combination of drugs real tumor shrinking and disintegration could be observed during clinical studies while no significant adverse drug reactions were detectable.

Preferred embodiments of the present disclosure, comprising the invention, relate to:

A method for the production of a medicament for the timed and combined use as a combination therapy for the treatment of colon and/or rectal cancer, the medicament comprising, preferably in two distinct (discrete) application forms,

a composition containing at least one specific integrin ligand, comprising cyclo-(Arg-Gly-Asp-DPhe-NMe-Val), the pharmaceutically acceptable solvates and/or salts thereof, and

a composition containing one or more alkylating chemotherapeutic agents, selected from the group consisting of the platinum containing compounds cisplatin, carboplatin and oxaliplatin, and

at least one further cancer cotherapeutic agent different from the at least one specific integrin ligand of a) and from the one or more alkylating chemotherapeutic agents of b), selected from the group consisting of:

> i) EGFR inhibitors, selected from the group consisting of cetuximab, panitumumab, zalutumumab, nimotuzumab and matuzumab and/or the group consisting of gefitinib, erlotinib and lapatinib,
> ii) cytostatic alkaloids, selected from the group consisting of etoposide, vinblastine and teniposide, the group consisting of vinorelbine, vincristine and vindesine, the group consisting of docetaxel and paclitaxel, and/or the group consisting of irinotecan and topotecan,
> iii) cytostatic antibiotics, selected from the group consisting of doxorubicin, idarubicin, daunorubicin, epirubicin and valrubicin, and
> iv) antimetabolites, selected from the group consisting of 5-fluorouracil, capecitabine, cytosinarabinosid and difluorodesoxycytidin and/or the group consisting of pemetrexed, methotrexat and raltitrexed,

and pharmaceutically acceptable salts and/or solvates thereof.

In one instance of the present disclosure, a set for the treatment of colon and/or rectal cancer comprising independent dosage forms of:

> a) a therapeutically effective amount of at least one specific integrin ligand selected from the group consisting of cyclo-(Arg-Gly-Asp-DPhe-NMeVal), the pharmaceutically acceptable dervatives, solvates and/or salts thereof, and
> b) a therapeutically effective amount of one or more alkylating chemotherapeutic agents, selected from the group consisting of the platinum containing compounds cisplatin, carboplatin and oxaliplatin, and
> c) a therapeutically effective amount of at least one further cancer cotherapeutic agent different from the at least one specific integrin ligand of a) and from the one or more alkylating chemotherapeutic agents of b), selected from the group consisting of:

> > i) EGFR inhibitors, selected from the group consisting of cetuximab, panitumumab, zalutumumab, nimotuzumab and matuzumab and/or the group consisting of gefitinib, erlotinib and lapatinib,
> > ii) cytostatic alkaloids, selected from the group consisting of etoposide, vinblastine and teniposide, the group consisting of vinorelbine, vincristine and vindesine, the group consisting of docetaxel and paclitaxel, and/or the group consisting of irinotecan and topotecan,
iii) cytostatic antibiotics, selected from the group consisting of doxorubicin, idarubicin, daunorubicin, epirubicin and valrubicin, and iv) antimetabolites, selected from the group consisting of 5-fluorouracil, capecitabine, cytosinarabinosid and difluorodesoxycytidin and/or the group consisting of pemetrexed, methotrexat and raltitrexed,

and pharmaceutically acceptable dervatives, sals and/or solvates thereof, optionally wherein a) is administered 4 to 8 hours (h), preferably 4 to 7 h, and most preferably 4 to 6 h prior to the application of b).

Said set is further characterized in that it will be advantageous to give detailed instructions to and how to use the cancer cotherapeutic agent, e.g. radiotherapy, in connection with the integrin ligand in form of a specific packaging, specific package inserts and similar.

Therefore, in yet another instance, a medicament consisting of an integrin ligand as described herein as one active ingredient, designed to be applied in combination with a further cancer cotherapeutic agent as described herein, e.g. prior to the further cancer cotherapeutic agent as described herein, e.g. in the case of radiotherapy, and contained in a container or similar, the container giving in form of writing detailed instructions and/or other technical information on how to use said medicament in combination with the cancer cotherapeutic agents, e.g. with respect to the above application schedule. A further preferred embodiment of the present invention is the use of at least one specific integrin ligand, comprising cyclo-(Arg-Gly-Asp-DPhe-NMe-Val), the pharmaceutically acceptable solvates and/or salts thereof, for the manufacture of a medicament for the treatment of colon and/or rectal cancer, wherein the medicament is to be used in combination with

a) one or more alkylating chemotherapeutic agents, selected from the group consisting of the platinum containing compounds cisplatin, carboplatin and oxaliplatin, and
b) one or more further chemotherapeutic agents other than the at least one specific integrin ligand and the one or more alkylating chemotherapeutic agents, selected from the group consisting of:

i) EGFR inhibitors, selected from the group consisting of cetuximab, panitumumab, zalutumumab, nimotuzumab and matuzumab and/or the group consisting of gefitinib, erlotinib and lapatinib,
ii) cytostatic alkaloids, selected from the group consisting of etoposide, vinblastine and teniposide, the group consisting of vinorelbine, vincristine and vindesine, the group consisting of docetaxel and paclitaxel, and/or the group consisting of irinotecan and topotecan,
iii) cytostatic antibiotics, selected from the group consisting of doxorubicin, idarubicin, daunorubicin, epirubicin and valrubicin, and
iv) antimetabolites, selected from the group consisting of 5-fluorouracil, capecitabine, cytosinarabinosid and difluorodesoxycytidin and/or the group consisting of pemetrexed, methotrexat and raltitrexed,

and pharmaceutically acceptable dervatives, salts and/or solvates thereof, wherein at least one specific integrin ligand, the one or more alkylating chemotherapeutic agents (a) and/or the one or more further chemotherapeutic agents other than the at least one specific integrin ligand and the one or more alkylating chemotherapeutic agents (b) are provided and/or formulated in(to) discrete application forms.

Another further preferred embodiment of the present invention relates the use of a pharmaceutical composition or a pharmaceutical kit as defined above, below and in the claims, for the manufacture of a medicament to treat tumors and tumor metastases.

The pharmaceutical treatment using the pharmaceutical compositions according to the invention may be accompanied, concurrently or sequentially, by a radiation therapy.

The pharmaceutical combinations for use according to the present invention provide various benefits. The combinations according to the present disclosure, comprising the invention, are useful in treating and preventing tumors, tumor-like and neoplasia disorders. Preferably, the different combined agents of the present invention are administered in combination at a low dose, that is, at a dose lower than has been conventionally used in clinical situations. A benefit of lowering the dose of the compounds, compositions, agents of the present invention administered to a mammal includes a decrease in the incidence of adverse effects associated with higher dosages. For example, by the lowering the dosage of a chemotherapeutic agent such as methotrexate, doxorubicin, gemcitabine, docetaxel, paclitaxel, bleomycin, cisplatin and/or Melphalan, a reduction in the frequency and the severity of nausea and vomiting will result when compared to that observed at higher dosages. Similar benefits are contemplated for the compounds, compositions, agents and therapies in combination with the integrin antagonists of the present invention. By lowering the incidence of adverse effects, an improvement in the quality of life of a cancer patient is contemplated. Further benefits of lowering the incidence of adverse effects include an improvement in patient compliance, a reduction in the number of hospitalizations needed for the treatment of adverse effects, and a reduction in the administration of analgesic agents needed to treat pain associated with the adverse effects.

Alternatively, the combinations for use according to the present invention can also maximize the therapeutic effect at higher doses.

## Detailed Description of the Invention

The invention is defined by the claims.

If not otherwise pointed out, the terms and phrases used in this invention preferably have the meanings and definitions as given below. Moreover, these definitions and meanings describe the invention in more detail, preferred embodiments included.

If not otherwise pointed out, the reference to a compound to be used according according to the invention preferably includes the reference to the pharmaceutically acceptable dervatives, solvates and salts thereof. If not otherwise pointed out, the reference to the integrin ligands, integrin antagonists, integrin agonists, as well as the reference to the cancer-cotherapeutic agents that are compounds, preferably includes the pharmaceutically acceptable solvates and salts thereof. Even more preferably, the reference to the integrin ligand cyclo-(Arg-Gly-Asp-DPhe-NMeVal) also includes the pharmaceutically acceptable dervatives, solvates and salts thereof, more preferably the pharmaceutically solvates and salts thereof and especially preferably the pharmaceutically acceptable salts thereof, if not indicated otherwise.

By "combination therapy" is preferably meant a combination of at least two distinct therapy forms so combined as to form a single therapeutical concept in a timely controlled, consecutive manner.

In a preferred embodiment of the present invention this means the combination of an integrin ligand with a further cotherapeutic agent. It is important to note that "combination therapy" preferably does not mean a distinct and/or single pharmaceutical composition or medicament. By way of contrast, in a preferred embodiment of the present invention the integrin ligand and the further cotherapeutic agent are provided in discrete containers, packages, medicaments, formulations or equivalents. Equally, the combination of integrin ligand therapy with radiation therapy preferably lies within the meaning of "combination therapy" of the present invention. "Therapy forms" preferably are any means, uses and/or formulations for treating cancer known in the art. By the term "distinct therapy forms" therefore it is meant that two different means, uses and/or formulations for treating cancer are combined. In the context of the present invention it is preferred that the first to be applied therapy form has anti-integrin activity (synonym: integrin ligand), and is applied prior to the second therapy form, preferably following the schedule as detailed above.

The term "composition comprising radiotherapy" preferably simply means that subsequent to the integrin ligand radiotherapy is applied. Therefore, the term "composition comprising radiotherapy" in the context of the present invention does not apply to a pharmaceutical composition as such, but to a pharmaceutical composition to be used in combination with radiotherapy.

With "cancer-cotherapeutic agent" or "cotherapeutic agent" preferably a cytotoxic, chemotherapeutical or immunotoxic agent is meant. Equally preferred is radiotherapy.

A "receptor" or "receptor molecule" is preferably a soluble or membrane bound or membrane associated protein or glycoprotein comprising one or more domains to which a ligand binds to form a receptor-ligand complex. By binding the ligand, which may be an agonist or an antagonist the receptor is activated or inactivated and may initiate or block pathway signaling.

By "ligand" or "receptor ligand" is preferably meant a natural or synthetic compound which binds a receptor molecule to form a receptor-ligand complex. The term ligand includes agonist, antagonists, and compounds with partial agonist/antagonist activity.

An "agonist" or "receptor agonist" is preferably a natural or synthetic compound which binds the receptor to form a receptor-agonist complex by activating said receptor and receptor-agonist complex, respectively, initiating a pathway signaling and further biological processes.

By "antagonist" or "receptor antagonist" is preferably meant a natural or synthetic compound that has a biological effect opposite to that of an agonist. An antagonist binds the receptor and blocks the action of a receptor agonist by competing with the agonist for receptor. An antagonist is defined by its ability to block the actions of an agonist. A receptor antagonist may be also an antibody or an immunotherapeutically effective fragment thereof. Preferred antagonists are cited and discussed below.

The term "integrin antagonists / inhibitors" or "integrin receptor antagonists / inhibitors" preferably refers to a natural or synthetic molecule, preferably a synthetic molecule, that blocks and inhibit an integrin receptor. In some cases, the term includes antagonists directed to the ligands of said integrin receptors (such as for $\alpha_v\beta_3$: vitronectin, fibrin, fibrinogen, von Willebrand's factor, thrombospondin, laminin; for $\alpha_v\beta_3$: vitronectin; for $\alpha_v\beta_1$: fibronectin and vitronectin; for $\alpha_v\beta_6$: fibronectin). Antagonists directed to the integrin receptors are preferred. Integrin (receptor) antagonists may be natural or synthetic peptides, non-peptides, peptidomimetica, immunoglobulins, such as antibodies or functional fragments thereof, or immunoconjugates (fusion proteins). Preferred integrin inhibitors are directed to receptor of $\alpha_v$ integrins (e.g. $\alpha_v\beta_3$, $\alpha_v\beta_5$, $\alpha_v\beta_6$ and sub-classes). Preferred integrin inhibitors are $\alpha_v$ antagonists, and in particular $\alpha_v\beta_3$ antagonists. Preferred $\alpha_v$ antagonists are RGD peptides, peptidomimetic (non-peptide) antagonists and anti-integrin receptor antibodies such as antibodies blocking $\alpha_v$ receptors.

Exemplary, non-immunological $\alpha_v\beta_3$ antagonists are described in the teachings of US 5,753,230 and US 5,766,591. Preferred antagonists are linear and cyclic RGD-containing peptides. Cyclic peptides are, as a rule, more stable and elicit an enhanced serum half-life. The most preferred integrin antagonist of the invention is, however, cyclo-(Arg-Gly-Asp-DPhe-NMeVal) (EMD 121974, Cilengitide®, Merck KGaA, Germany; EP 0770 622) which is efficacious in blocking the integrin receptors $\alpha_v\beta_3$, $\alpha_v\beta_1$, $\alpha_v\beta_6$, $\alpha_v\beta_8$, $\alpha_{IIb}\beta_3$, and preferably especially efficacious with respect to integrin receptors $\alpha_v\beta_3$ and/or $\alpha_v\beta_5$. As is clear to the ones skilled in the art, the cyclo-(Arg-Gly-Asp-DPhe-NMeVal) can be also applied in the context of the instant invention in the form of a solvate and/or a salt thereof. The same preferably also applies to all other compounds or active ingredients to be used in the context of the present invention.

Suitable peptidyl as well as peptidomimetic (non-peptide) antagonists of the $\alpha_v\beta_3$ / $\alpha_v\beta_5$ / $\alpha_v\beta_6$ integrin receptor have been described both in the scientific and patent literature. For example, reference is made to Hoekstra and Poulter, 1998, Curr. Med. Chem. 5, 195; WO 95/32710; WO 95/37655; WO 97/01540; WO 97/37655; WO 97/45137; WO 97/41844; WO 98/08840; WO 98/18460; WO 98/18461; WO 98/25892; WO 98/31359; WO 98/30542; WO 99/15506; WO 99/15507; WO 99/31061; WO 00/06169; EP 0853 084; EP 0854 140; EP 0854 145; US 5,780,426; and US 6,048,861. Patents that disclose benzazepine, as well as related benzodiazepine and benzocycloheptene $\alpha_v\beta_3$ integrin receptor antagonists, which are also suitable for the use in this invention, include WO 96/00574, WO 96/00730, WO 96/06087, WO 96/26190, WO 97/24119, WO 97/24122, WO 97/24124, WO 98/15278, WO 99/05107, WO 99/06049, WO 99/15170, WO 99/15178, WO 97/34865, WO 97/01540, WO 98/30542, WO 99/11626, and WO 99/15508. Other integrin receptor antagonists featuring backbone conformational ring constraints have been described in WO 98/08840; WO 99/30709; WO 99/30713; WO 99/31099; WO 00/09503: US 5,919,792; US 5,925,655; US 5,981,546; and US 6,017,926. In US 6,048,861 and WO 00/72801 a series of nonanoic acid derivatives which are potent $\alpha_v\beta_3$ integrin receptor antagonists were disclosed. Other chemical small molecule integrin antagonists (mostly vitronectin antagonists) are described in WO 00/38665. Other $\alpha_v\beta_3$ receptor antagonists have been shown to be effective in inhibiting angiogenesis. For example, synthetic receptor antagonists such as (S)-10,11-Dihydro-3-[3-(pyridin-2-ylamino)-1-propyloxy]-5H-dibenzo[ a,d]cycloheptene-10-acetic acid (known as SB-265123) have been tested in a variety of mammalian model systems. (Keenan et al., 1998, Bioorg. Med. Chem. Lett. 8(22), 3171; Ward et al., 1999, Drug Metab. Dispos. 27(11), 1232). Assays for the identification of integrin antagonists suitable for use as an antagonist are described, e.g. by Smith et al., 1990, J. Biol. Chem. 265, 12267, and in the referenced patent literature. Anti-integrin receptor antibodies are also well known. Suitable anti-integrin (e.g. $\alpha_v\beta_3$, $\alpha_v\beta_5$, $\alpha_v\beta_6$) monoclonal antibodies can be modified to encompass antigen binding fragments thereof, including F(ab)$_2$, Fab, and engineered Fv or single-chain antibody. One suitable and preferably used monoclonal antibody directed against integrin receptor $\alpha_v\beta_3$ is identified as LM609 (Brooks et al., 1994, Cell 79, 1157; ATCC HB 9537). A potent specific anti-$\alpha_v\beta_5$ antibody, P1F6, is disclosed in WO 97/45447, which is also preferred according to this invention. A further suitable $\alpha_v\beta_6$ selective antibody is MAb 14D9.F8 (WO 99/37683, DSM ACC2331, Merck KGaA, Germany) which is selectively directed to the $\alpha_v$- chain of integrin receptors. Another suitable anti-integrin antibody is the commercialized Vitraxin ®.

The term "antibody" or "immunoglobulin" herein is preferably used in the broadest sense and specifically covers intact monoclonal antibodies, polyclonal antibodies, multispecific antibodies (e.g. bispecific antibodies) formed from at least two intact antibodies, and antibody fragments, so long as they exhibit the desired biological activity. The term generally includes heteroantibodies which are composed of two or more antibodies or fragments thereof of different binding specificity which are linked together.

Depending on the amino acid sequence of their constant regions, intact antibodies can be assigned to different "antibody (immunoglobulin) classes". There are five major classes of intact antibodies: IgA, IgD, IgE, IgG, and IgM, and several of these may be further divided into "subclasses" (isotypes), e.g., IgG1, IgG2. IgG3, IgG4, IgA, and IgA2. The heavy-chain constant domains that correspond to the different classes of antibodies are called $\alpha$, $\delta$, $\epsilon$, $\gamma$ and $\mu$ respectively. Preferred major class for antibodies according to the invention is IgG, in more detail IgG1 and IgG2.

Antibodies are usually glycoproteins having a molecular weight of about 150,000, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intra-chain disulfide bridges. Each heavy chain has at one end a variable domain (VH) followed by a number of constant domains. The variable regions comprise hypervariable regions or "CDR" regions, which contain the antigen binding site and are responsible for the specificity of the antibody, and the "FR" regions, which are important with respect to the affinity / avidity of the antibody. The hypervariable region generally comprises amino acid residues from a "complementarity determining region" or "CDR" (e.g. residues 24-34 (L1), 50-56 (L2) and 89-97 (L3) in the light chain variable domain and 31-35 (H1), 50-65 (H2) and 95-102 (H3) in the heavy chain variable domain; and/or those residues from a "hypervariable loop" (e.g. residues 26-32 (L1), 50-52 (L2) and 91-96 (L3) in the light chain variable domain and 26-32 (H1), 53-55 (H2) and 96-101 (H3) in the heavy chain variable domain; Chothia and Lesk J. Mol. Biol. 196:901-917 (1987)).The "FR" residues (frame work region) are those variable domain residues other than the hypervariable region residues as herein defined. Each light chain has a variable domain at one end (VL) and a constant domain at its other end. The constant domain of the light chain is aligned with the first constant domain of the heavy chain, and the light-chain variable domain is aligned with the variable domain of the heavy chain. Particular amino acid residues are believed to form an interface between the light chain and heavy chain variable domains. The "light chains" of antibodies from any vertebrate species can be assigned to one of two clearly distinct types, called kappa ($\kappa$) and lambda ($\lambda$), based on the amino acid sequences of their constant domains.

The term "monoclonal antibody" as used herein preferably refers to an antibody obtained from a population of substantially homogeneous antibodies, *i.e.*, the individual antibodies comprising the population are identical except for possible naturally occurring mutations that may be present in minor amounts. Monoclonal antibodies are highly specific, being directed against a single antigenic site. Furthermore, in contrast to polyclonal antibody preparations which include different antibodies directed against different determinants (epitopes), each monoclonal antibody is directed against a single determinant on the antigen. In addition to their specificity, the monoclonal antibodies are advantageous in that they may be synthesized uncontaminated by other antibodies. Methods for making monoclonal antibodies include the hybridoma method described by Kohler and Milstein (1975, Nature 256, 495) and in "Monoclonal Antibody Technology, The Production and Characterization of Rodent and Human Hybridomas" (1985, Burdon et al., Eds, Laboratory Techniques in Biochemistry and Molecular Biology, Volume 13, Elsevier Science Publishers, Amsterdam), or may be made by well known recombinant DNA methods (see, e.g., US 4,816,567). Monoclonal antibodies may also be isolated from phage antibody libraries using the techniques described in Clackson et al., Nature, 352:624-628 (1991) and Marks et al., J. Mol. Biol., 222:58, 1-597(1991), for example. The term "chimeric antibody" preferably means antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (e.g.: US 4,816,567; Morrison et al., Proc. Nat. Acad. Sci., USA, 81:6851-6855 (1984)). Methods for making chimeric and humanized antibodies are also known in the art. For example, methods for making chimeric antibodies include those described in patents by Boss (Celltech) and by Cabilly (Genentech) (US 4,816,397; US 4,816,567).

"Humanized antibodies" preferably are forms of non-human (e.g., rodent) chimeric antibodies that contain minimal sequence derived from non-human immunoglobulin. For the most part, humanized antibodies are human immunoglobulins (recipient antibody) in which residues from a hypervariable region (CORs) of the recipient are replaced by residues from a hypervariable region of a non-human species (donor antibody) such as mouse, rat, rabbit or nonhuman primate having the desired specificity, affinity and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. Methods for making humanized antibodies are described, for example, by Winter (US 5,225,539) and Boss (Celltech, US 4,816,397). "Antibody fragments" preferably comprise a portion of an intact antibody, preferably comprising the antigen-binding or variable region thereof. Examples of antibody fragments include Fab, Fab', F(ab')$_2$, Fv and Fc fragments, diabodies, linear antibodies, single-chain antibody molecules; and multispecific antibodies formed from antibody fragment(s). An "intact" antibody is one which comprises an antigen-binding variable region as well as a light chain constant domain (CL) and heavy chain constant domains, CH1, CH2 and CH3. Preferably, the intact antibody has one or more effector functions. Papain digestion of antibodies produces two identical antigen-binding fragments, called "Fab" fragments, each comprising a single antigen-binding site and a CL and a CH1 region, and a residual "Fc" fragment, whose name reflects its ability to crystallize readily. The "Fc" region of the antibodies comprises, as a rule, a CH2, CH3 and the hinge region of an IgG1 or IgG2 antibody major class. The hinge region is a group of about 15 amino acid residues which combine the CH1 region with the CH2-CH3 region. Pepsin treatment yields an "F(ab)2" fragment that has two antigen-binding sites and is still capable of cross-linking antigen. "Fv" is the minimum antibody fragment which contains a complete antigen-recognition and antigen-binding site. This region consists of a dimer of one heavy chain and one light chain variable domain in tight, non-covalent association. It is in this configuration that the three hypervariable regions (CDRs) of each variable domain interact to define an antigen-binding site on the surface of the VH - VL dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site. The Fab fragment also contains the constant domain of the light chain and the first constant domain (CH 1) of the heavy chain. "Fab" fragments differ from Fab fragments by the addition of a few residues at the carboxy terminus of the heavy chain CH1 domain including one or more cysteines from the antibody hinge region. F(ab')2 antibody fragments originally were produced as pairs of Fab' fragments which have hinge cysteines between them. Other chemical couplings of antibody fragments are also known (see e.g. Hermanson, Bioconjugate Techniques, Academic Press, 1996; US 4,342,566). "Single-chain Fv" or "scFv" antibody fragments preferably comprise the V, and V, domains of antibody, wherein these domains are present in a Single polypeptide chain. Preferably, the Fv polypeptide further comprises a polypeptide linker between the VH and VL domains which enables the scFv to form the desired structure for antigen binding. Single-chain FV antibodies are known, for example, from Plückthun (The Pharmacology of Monoclonal Antibodies, Vol. 113, Rosenburg and Moore eds., Springer-Verlag, New York, pp. 269-315 (1994)), WO93/16185; US 5,571,894; US 5,587,458; Huston et al. (1988, Proc. Natl. Acad. Sci. 85, 5879) or Skerra and Plueckthun (1988, Science 240, 1038). "Bispecific antibodies" preferably are single, divalent antibodies (or immunotherapeutically effective fragments thereof) which have two differently specific antigen binding sites. For example the first antigen binding site is directed to an angiogenesis receptor (e.g. integrin or VEGF receptor), whereas the second antigen binding site is directed to an ErbB receptor (e.g. EGFR or Her 2). Bispecific antibodies can be produced by chemical techniques (see e.g., Kranz et al. (1981) Proc. Natl. Acad. Sci. USA 78, 5807), by "polydoma" techniques (See US 4,474,893) or by recombinant DNA techniques, which all are known per se. Further methods are described in WO 91/00360, WO 92/05793 and WO 96/04305. Bispecific antibodies can also be prepared from single chain antibodies (see e.g., Huston et al. (1988) Proc. Natl. Acad. Sci. 85, 5879; Skerra and Plueckthun (1988) Science 240, 1038). These are analogues of antibody variable regions produced as a single polypeptide chain. To form the bispecific binding agent, the single chain antibodies may be coupled together chemically or by genetic engineering methods known in the art. It is also possible to produce bispecific antibodies by using leucine zipper sequences. The sequences employed are derived from the leucine zipper regions of the transcription factors Fos and Jun (Landschulz et al., 1988, Science 240,1759; for review, see Maniatis and Abel, 1989, Nature 341, 24). Leucine zippers are specific amino acid sequences about 20-40 residues long with leucine typically occurring at every seventh residue. Such zipper sequences form amphiphatic $\alpha$-helices, with the leucine residues lined up on the hydrophobic side for dimer formation. Peptides corresponding to the leucine zippers of the Fos and Jun proteins form heterodimers preferentially (O'Shea et al., 1989, Science 245, 646). Zipper containing bispecific antibodies and methods for making them are also disclosed in WO 92/10209 and WO 93/11162. A bispecific antibody may be an antibody, directed to VEGF receptor and $\alpha v\beta 3$ receptor as discussed above with respect to the antibodies having single specificity.

"Heteroantibodies" preferably are two or more antibodies or antibody-binding fragments which are linked together, each of them having a different binding specificity. Heteroantibodies can be prepared by conjugating together two or more antibodies or antibody fragments. Preferred heteroantibodies are comprised of cross-linked Fab/Fab' fragments. A variety of coupling or crosslinking agents can be used to conjugate the antibodies. Examples are protein A, carboimide, N-succinimidyl-S-acetyl-thioacetate (SATA) and N-succinimidyl-3-(2-pyridyldithio) propionate (SPDP) (see e.g., Karpovsky et al. (1984) J. EXP. Med. 160,1686; Liu et a. (1985) Proc. Natl. Acad. Sci. USA 82, 8648). Other methods include those described by Paulus, Behring Inst. Mitt., No. 78, 118 (1985); Brennan et a. (1985) Science 30 Method:81 or Glennie et al. (1987) J. Immunol. 139, 2367. Another method uses o-phenylenedimaleimide (oPDM) for coupling three Fab' fragments (WO 91/03493). Multispecific antibodies are in context of the present disclosure also suitable and can be prepared, for example according to the teaching of WO 94/13804 and WO 98/50431.

The term "fusion protein" preferably refers to a natural or synthetic molecule consisting of one ore more proteins or peptides or fragments thereof having different spificity which are fused together optionally by a linker molecule. Preferably the term includes fusion constructs, wherein at least one protein or peptide is a immunoglobulin or antibody, respectively or parts thereof ("immunoconjugates").

The term "immunoconjugate" preferably refers to an antibody or immunoglobulin respectively, or a immunologically effective fragment thereof, which is fused by covalent linkage to a non-immunologically effective molecule. Preferably this fusion partner is a peptide or a protein, which may be glycosylated. Said non-antibody molecule can be linked to the C-terminal of the constant heavy chains of the antibody or to the N-terminals of the variable light and/or heavy chains. The fusion partners can be linked via a linker molecule, which is, as a rule, a 3 - 15 amino acid residues containing peptide. Immunoconjugates according to the invention consist of an immunoglobulin or immunotherapeutically effective fragment thereof, directed to a receptor tyrosine kinase, preferably an ErbB (ErbB1/ErbB2) receptor and an integrin antagonistic peptide, or an angiogenic receptor, preferably an integrin or VEGF receptor and TNF$\alpha$ or a fusion protein consisting essentially of TNF$\alpha$ and IFN$\gamma$ or another suitable cytokine, which is linked with its N-terminal to the C-terminal of said immunoglobulin, preferably the Fc portion thereof. The term includes also corresponding fusion constructs comprising bi- or multi-specific immunoglobulins (antibodies) or fragments thereof. The term "functionally intact derivative" means according to the understanding of this invention preferably a fragment or portion, modification, variant, homologue or a de-immunized form (a modification, wherein epitopes, which are responsible for immune responses, are removed) of a compound, peptide, protein, antibody (immunoglobulin), immunconjugate, etc., that has principally the same biological and / or therapeutic function as compared with the original compound, peptide, protein, antibody (immunoglobulin), immunconjugate, etc. However, the term includes also such derivatives, which elicit a reduced or enhanced efficacy.

The term "cytokine" is preferably a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; mouse gonadotropin-associated peptide; inhibin; activin; vascular endothelial growth factor (VEGF); integrin; thrombopoietin (TPO); nerve growth factors such as NGFβ; platelet-growth factor; transforming growth factors (TGFs) such as TGFα and TGFβ; erythropoietin (EPO); interferons such as IFNα, IFNβ, and IFNγ; colony stimulating factors such as M-CSF, GM-CSF and G-CSF; interleukins such as IL-1, IL-1a, IL-2, IL-3, IL-4, IL-5, IL-6, IL-7, IL-8, IL-9. IL-10, IL-11, IL-12; and TNFα or TNFβ. Preferred cytokines are interferons and TNFα.

The term "cytotoxic agent" as used herein preferably refers to a substance that inhibits or prevents the function of cells and/or causes destruction of cells. The term is preferably intended to include radioactive isotopes, chemotherapeutic agents, and toxins such as enzymatically active toxins of bacterial, fungal, plant or animal origin, or fragments thereof. The term may include also members of the cytokine family, preferably IFNγ as well as anti-neoplastic agents having also cytotoxic activity.

The term "chemotherapeutic agent", "chemotherapeutical agent" or "anti-neoplastic agent" is regarded according to the understanding of this invention preferably as a member of the class of "cytotoxic agents", as specified above, and includes chemical agents that exert anti-neoplastic effects, i.e., prevent the development, maturation, or spread of neoplastic cells, directly on the tumor cell, e.g., by cytostatic or cytotoxic effects, and not indirectly through mechanisms such as biological response modification. Suitable chemotherapeutic agents are preferably natural or synthetic chemical compounds, but biological molecules, such as proteins, polypeptides etc. are not expressively excluded. There are large numbers of anti-neoplastic agents available in commercial use, in clinical evaluation and in pre-clinical development, which could be included in the present specification for treatment of tumors / neoplasia by combination therapy with TNFα and the anti-angiogenic agents as cited above, optionally with other agents such as EGF receptor antagonists. It should be pointed out that the chemotherapeutic agents can be administered optionally together with above-said drug combination. Examples of chemotherapeutic or agents include alkylating agents, for example, nitrogen mustards, ethyleneimine compounds, alkyl sulphonates and other compounds with an alkylating action such as nitrosoureas, cisplatin and dacarbazine; antimetabolites, for example, folic acid, purine or pyrimidine antagonists; mitotic inhibitors, for example, vinca alkaloids and derivatives of podophyllotoxin; cytotoxic antibiotics and camptothecin derivatives. Preferred chemotherapeutic agents or chemotherapy include amifostine (ethyol), cisplatin, dacarbazine (DTIC), dactinomycin, mechlorethamine (nitrogen mustard), streptozocin, cyclophosphamide, carmustine (BCNU), lomustine (CCNU), doxorubicin (adriamycin), doxorubicin lipo (doxil), gemcitabine (gemzar), daunorubicin, daunorubicin lipo (daunoxome), procarbazine, mitomycin, cytarabine, etoposide, methotrexate, 5-fluorouracil (5-FU), vinblastine, vincristine, bleomycin, paclitaxel (taxol), docetaxel (taxotere), aldesleukin, asparaginase, busulfan, carboplatin, cladribine, camptothecin, CPT-11, 10-hydroxy-7-ethyl-camptothecin (SN38), dacarbazine, floxuridine, fludarabine, hydroxyurea, ifosfamide, idarubicin, mesna, interferon alpha, interferon beta, irinotecan, mitoxantrone, topotecan, leuprolide, megestrol, melphalan, mercaptopurine, plicamycin, mitotane, pegaspargase, pentostatin, pipobroman, plicamycin, streptozocin, tamoxifen, teniposide, testolactone, thioguanine, thiotepa, uracil mustard, vinorelbine, chlorambucil and combinations thereof.

Preferred chemotherapeutic agents according to the invention include cisplatin, gemcitabine, temozolomide, doxorubicin, paclitaxel (taxol) and bleomycin.

The term "immunotoxic" preferably refers to an agent which combines the specifity of a immunomolecule .e.g. an antibody or a functional equivalent thereof with a toxic moiety, e.g. a cytotoxic function as defined above.

Further examples of cancer cotherapeutic agents and preferably of chemotherapeutical agents, cytotoxic agents, immunomodulating agents and/or immunotoxic agents preferably include antibodies against one or more target, preferably selected from the group consisting of HER, HER2, PDGF, PDGFR, EGF, EGFR, VEGF, VEGFR and/or VEGFR2, wherein said antibodies are preferably selected from Herceptin, Bevacizumab (rhuMAb-VEGF, Avastin®), Cetuximab (Erbitux®) and Nimotuzumab, and preferably small molecules or NCEs against one or more of said targets, preferably selected from the group consisting of Sorafenib (Nexavar®), Sunitinib (Sutent®) and ZD6474 (ZACTIMA™).

In a preferred instance of the present disclosure, the chemotherapeutical agents, cytotoxic agents, immunomodulating agents and/or immunotoxic agents are selected from one or more of the following groups:

    a) alkylating agents,
    b) antibiotics,
    c) antimetabolites,
    d) biologicals and immunomodulators,

e) hormones and antagonists thereof,
f) mustard gas derivatives,
g) alkaloids,
h) protein kinase inhibitors.

In a more preferred aspect of the present disclosure, the chemotherapeutical agents, cytotoxic agents, immunomodulating agents and/or immunotoxic agents are selected from one or more of the following groups:

a) alkylating agents, selected from busulfan, melphalan, carboplatin, cisplatin, cyclophosphamide, dacarbazine, carmustine (BCNU), nimustin (ACNU), lomustine (CCNU), ifosfamide, temozolomide and altretamine,
b) antibiotics, selected from leomycin, doxorubicin, adriamycin, idarubicin, epirubicin and plicamycin,
c) antimetabolites, selected from sulfonamides, folic acid antagonists, gemcitabine, 5-fluorouracil (5-FU), leucovorine, leucovorine with 5-FU, 5-FU with calcium folinate, and leucovorin, capecitabine, mercaptopurine, cladribine, pentostatine, methotrexate, raltitrexed, pemetrexed, thioguanine, camptothecin derivates (topotecan, irinotecan)
d) biologicals and immunomodulators, selected from interferon a2A, interleukin 2 and levamisole,
e) hormones and antagonists thereof, selected from flutamide, goserelin, mitotane and tamoxifen,
f) mustard gas derivatives, selected from melphalan, carmustine and nitrogen mustard,
g) alkaloids, selected from taxanes, docetaxel, paclitaxel, etoposide, vincristine, vinblastine and vinorelbine.

With respect to the instant invention, the term "further chemotherapeutic agent" refers to a chemotherapeutic agent that is different from the at least one specific integrin ligand as defined herein and different from the one or more alkylating chemotherapeutic agents as defined herein. With respect to the instant invention, the "further chemotherapeutic agent" as defined herein is also referred to as "further chemotherapeutic agent (b)" or as "further chemotherapeutic agent other than the at least one specific integrin ligand and the one or more alkylating chemotherapeutic agents (b)".

With respect to the instant invention, the term "one or more further chemotherapeutic agents" refers to one or more chemotherapeutic agents that are different from the at least one specific integrin ligand as defined herein and different from the one or more alkylating chemotherapeutic agents as defined herein. With respect to the instant invention, the "one or more further chemotherapeutic agents" as defined herein are also referred to as "one or more further chemotherapeutic agents (b)" or as "one or more further chemotherapeutic agents other than the at least one specific integrin ligand and the one or more alkylating chemotherapeutic agents (b)".

The term "cancer cotherapeutic agent" or preferably "further cancer cotherapeutic agent" is preferably as defined herein. More preferably, it is selected from the group consisting of

i) a further chemotherapeutic agent as defined herein that is different from the at least one specific integrin ligand as defined herein and different from the alkylating chemotherapeutic agent, as defined herein, and
ii) radiotherapy, preferably radiotherapy as defined herein.

The term "one or more further cancer cotherapeutic agent" is preferably as defined herein. More preferably, it is selected from the group consisting of

i) one or more further chemotherapeutic agents other than the at least one specific integrin ligand as defined herein and the one or more alkylating chemotherapeutic agents as defined herein, and
ii) radiotherapy, preferably radiotherapy as defined herein.

Even more preferably, the term "one or more further cancer cotherapeutic agent" is selected from the group consisting of one or more further chemotherapeutic agents other than the at least one specific integrin ligand as defined herein and the one or more alkylating chemotherapeutic agents as defined herein.

Dosings and preferably standard administration schedules for the above and/or below given cancer cotherapeutic agents are known in the art.

The terms "cancer" and "tumor" preferably refer to or describe the physiological condition in mammals that is typically characterized by unregulated cell growth. By means of the pharmaceutical compositions according of the present disclosure tumors can be treated such as tumors of the breast, heart, lung, small intestine, colon, spleen, kidney, bladder, head and neck, ovary, prostate, brain, pancreas, skin, bone, bone marrow, blood, thymus, uterus, testicles, cervix, and liver. More specifically the tumor is selected from the group consisting of adenoma, angio-sarcoma, astrocytoma, epithelial carcinoma, germinoma, glioblastoma, glioma, hamartoma, hemangioendothelioma, hemangiosarcorna, hematoma, hepatoblastoma, leukemia, lymphoma, medulloblastoma, melanoma, neuroblastoma, osteosarcoma, retinoblastoma, rhabdomyosarcoma, sarcoma and teratoma.

In detail, the tumor/cancer is selected from the group consisting of acral lentiginous melanoma, actinic keratoses, adenocarcinoma, adenoid cycstic carcinoma, adenomas, adenosarcoma, adenosquamous carcinoma, astrocytic tumors, bartholin gland carcinoma, basal cell carcinoma, bronchial gland carcinomas, capillary, carcinoids, carcinoma, carcinosarcoma, cavernous, cholangio-carcinoma, chondosarcoma, choriod plexus papilloma/carcinoma, clear cell carcinoma, cystadenoma, endodermal sinus tumor, endometrial hyperplasia, endometrial stromal sarcoma, endometrioid adenocarcinoma, ependymal, epitheloid, Ewing's sarcoma, fibrolamellar, focal nodular hyperplasia, gastrinoma, germ cell tumors, glioblastoma, glucagonoma, hemangiblastomas, hemangioendothelioma, hemangiomas, hepatic adenoma, hepatic adenomatosis, hepatocellular carcinoma, insulinoma, intaepithelial neoplasia, interepithelial squamous cell neoplasia, invasive squamous cell carcinoma, large cell carcinoma, leiomyosarcoma, lentigo maligna melanomas, malignant melanoma, malignant mesothelial tumors, medulloblastoma, medulloepithelioma, melanoma, meningeal, mesothelial, metastatic carcinoma, mucoepidermoid carcinoma, neuroblastoma, neuroepithelial adenocarcinoma nodular melanoma, oat cell carcinoma, oligodendroglial, osteosarcoma, pancreatic polypeptide, papillary serous adeno-carcinoma, pineal cell, pituitary tumors, plasmacytoma, pseudo-sarcoma, pulmonary blastoma, renal cell carcinoma, retinoblasioma, rhabdomyo-sarcoma, sarcoma, serous carcinoma, small cell carcinoma, soft tissue carcinomas, somatostatin-secreting tumor, squamous carcinoma, squamous cell carcinoma, submesothelial, superficial spreading melanoma, undifferentiated carcinoma, uveal melanoma, verrucous carcinoma, vipoma, well differentiated carcinoma, and Wilm's tumor. More preferably, the tumor/cancer is selected from the group consisting of intracerebral cancer, head-and-neck cancer, rectal cancer, astrocytoma, preferably astrocytoma grade II, III or IV, glioblastoma, preferably glioblastoma multiforme (GBM), small cell lung cancer (SCLC) and non-small cell lung cancer (NSCLC), preferably non-small cell lung cancer (NSCLC), metastatic melanoma, metastatic androgen independent prostate cancer (AIPCa), metastatic androgen dependent prostate cancer (ADPCa) and breast cancer. Even more preferably, the tumor/cancer is selected from the group consisting of astrocytoma, preferably astrocytoma grade II, III or IV, glioblastoma, preferably glioblastoma multiforme, small cell lung cancer (SCLC) and non-small cell lung cancer (NSCLC), preferably non-small cell lung cancer (NSCLC), metastatic melanoma, metastatic androgen independent prostate cancer (AIPCa), metastatic androgen dependent prostate cancer (ADPCa). Also more preferably, the tumor/cancer is selected from metastases, preferably brain metastases, of small cell lung cancer (SCLC) and non-small cell lung cancer (NSCLC), preferably non-small cell lung cancer (NSCLC), metastatic melanoma, metastatic androgen independent prostate cancer (AIPCa), metastatic androgen dependent prostate cancer (ADPCa) and breast cancer.

In one instance of the present disclosure comprising the invention, the "pharmaceutical compositions" of the invention may comprise agents that reduce or avoid side effects associated with the combination therapy of the present invention ("adjunctive therapy"), including, but not limited to, those agents, for example, that reduce the toxic effect of anticancer drugs, e.g., bone resorption inhibitors, cardioprotective agents. Said adjunctive agents prevent or reduce the incidence of nausea and vomiting associated with chemotherapy, radiotherapy or operation, or reduce the incidence of infection associated with the administration of myelosuppressive anticancer drugs. Adjunctive agents are well known in the art. The immunotherapeutic agents for use according to the invention can additionally administered with adjuvants like BCG and immune system stimulators. Furthermore, the compositions may include immunotherapeutic agents or chemotherapeutic agents which contain cytotoxic effective radio labeled isotopes, or other cytotoxic agents, such as a cytotoxic peptides (e.g. cytokines) or cytotoxic drugs and the like. The term "pharmaceutical kit" for treating tumors or tumor metastases refers to a package and, as a rule, instructions for using the reagents in methods to treat tumors and tumor metastases. A reagent in a kit of this disclosure is typically formulated as a therapeutic composition as described herein, and therefore can be in any of a variety of forms suitable for distribution in a kit. Such forms can include a liquid, powder, tablet, suspension and the like formulation for providing the antagonist and/or the fusion protein of the present invention. The reagents may be provided in separate containers suitable for administration separately according to the present methods, or alternatively may be provided combined in a composition in a single container in the package. The package may contain an amount sufficient for one or more dosages of reagents according to the treatment methods described herein. A kit of this invention also contains "instruction for use" of the materials contained in the package.

As used herein. the terms "pharmaceutically acceptable" and grammatical variations thereof, as they refer to compositions, carriers, diluents and reagents, are used interchangeably and represent that the materials are capable of administration to or upon a mammal without the production of undesirable physiological effects such as nausea, dizziness, gastric upset and the like. The preparation of a pharmacological composition that contains active ingredients dissolved or dispersed therein is well understood in the art and need not be limited based on formulation. Typically, such compositions are prepared as injectables either as liquid solutions or suspensions, however, solid forms suitable for solution, or suspensions, in liquid prior to use can also be prepared. The preparation can also be emulsified. The active ingredient can be mixed with excipients which are pharmaceutically acceptable and compatible with the active ingredient and in amounts suitable for use in the therapeutic methods described herein. Suitable excipients are, for example, water, saline, dextrose, glycerol, ethanol or the like and combinations thereof. In addition, if desired, the composition can contain minor amounts of auxiliary substances such as wetting or emulsifying agents., pH buffering agents and the like which enhance the effectiveness of the active ingredient. The therapeutic composition of the present invention can include pharmaceutically acceptable salts of the components therein. Pharmaceutically acceptable salts include the acid addition salts (formed with the free amino groups of the polypeptide) that are formed with inorganic acids such as. for example, hydrochloric or phosphoric acids, or such organic acids as acetic, tartaric, mandelic and the like. Salts formed with the free carboxyl groups can also be derived from inorganic bases such as, for example, sodium, potassium, ammonium, calcium or ferric hydroxides, and such organic bases as isopropylamine, trimethylamine, 2-ethylamino ethanol, histidine, procaine and the like. Particularly preferred is the HCl salt when used in the preparation of cyclic polypeptide $\alpha v$ antagonists. Physiologically tolerable carriers are well known in the art. Exemplary of liquid carriers are sterile aqueous solutions that contain no materials in addition to the active ingredients and water, or contain a buffer such as sodium phosphate at physiological pH value, physiological saline or both, such as phosphate-buffered saline. Still further, aqueous carriers can contain more than one buffer salt, as well as salts such as sodium and potassium chlorides, dextrose, polyethylene glycol and other solutes. Liquid compositions can also contain liquid phases in addition to and to the exclusion of water. Exemplary of such additional liquid phases are glycerin. vegetable oils such as cottonseed oil, and water-oil emulsions. Typically, a therapeutically effective amount of an immunotherapeutic agent in the form of a, for example, antibody or antibody fragment or antibody conjugate is an amount such that when administered in physiologically tolerable composition is sufficient to achieve a plasma concentration of from about 0.01 microgram ($\mu$g) per milliliter (ml) to about 100 $\mu$g/ml, preferably from about 1 $\mu$g/ml to about 5 $\mu$g/ml and usually about 5 $\mu$g/ml. Stated differently the dosage can vary from about 0.1 mg/kg to about 300 mg/kg, preferably from about 0.2 mg/kg to about 200 mg/kg, most preferably from about 0.5 mg/kg to about 20 mg/kg, in one or more dose administrations daily for one or several days. Where the immunotherapeutic agent is in the form of a fragment of a monoclonal antibody or a conjugate, the amount can readily be adjusted based on the mass of the fragment / conjugate relative to the mass of the whole antibody. A preferred plasma concentration in molarity is from about 2 micromolar ($\mu$M) to about 5 millimolar (mM) and preferably, about 100 $\mu$M to 1 mM antibody antagonist. A therapeutically effective amount of an agent according of this invention which is a non-immunotherapeutic peptide or a protein polypeptide (e.g. IFN-alpha), or other similarly-sized small molecule, is typically an amount of polypeptide such that when administered in a physiologically tolerable composition is sufficient to achieve a plasma concentration of from about 0.1 microgram ($\mu$g) per milliliter (ml) to about 200 $\mu$g/ml, preferably from about 1 $\mu$g/ml to about 150 $\mu$g/ml. Based on a polypeptide having a mass of about 500 grams per mole, the preferred plasma concentration in molarity is from about 2 micromolar ($\mu$M) to about 5 millimolar (mM) and preferably about 100 $\mu$M to 1 mM polypeptide antagonist. The typical dosage of an active agent, which is a preferably a chemical antagonist or a (chemical) chemotherapeutic agent according to the invention (neither an immunotherapeutic agent nor a non-immunotherapeutic peptide/protein) is 10 mg to 1000 mg, preferably about 20 to 200 mg, and more preferably 50 to 100 mg per kilogram body weight per day. The preferred dosage of an active agent, which is a preferably a chemical antagonist or a (chemical) chemotherapeutic agent according to the invention (neither an immunotherapeutic agent nor a non-immunotherapeutic peptide/protein) is 0.5 mg to 3000 mg per patient and day, more preferably 10 to 2500 mg per patient and per day, and especially 50 to 1000 mg per patient and per day, or, per kilogram body weight, preferably about 0.1 to 100 mg/kg, and more preferably 1 mg to 50 mg/kg, preferably per dosage unit and more preferably per day, or, per square meter of the bodysurface, preferably 0.5 mg to 2000 mg/m$^2$, more preferably 5 to 1500 mg/m$^2$, and especially 50 to 1000 mg/m$^2$, preferably per dosage unit and more preferably per day.

The term "therapeutically effective" or "therapeutically effective amount" refers to an amount of a drug effective to treat a disease or disorder in a mammal. In the case of cancer, the therapeutically effective amount of the drug may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the cancer. To the extent the drug may prevent growth and/or kill existing cancer cells, it may be cytostatic and/or cytotoxic. For cancer therapy, efficacy can, for example, be measured by assessing the time to disease progression (TTP) and/or determining the response rate (RR).

As used herein, the term "physiologically functional derivative" preferably refers to any pharmaceutically acceptable derivative of a compound to be used according to the present invention, for example, an ester or an amide, which upon administration to a mammal is capable of providing (directly or indirectly) a compound of the present invention or an active metabolite thereof. Such derivatives are clear to those skilled in the art, without undue experimentation, and with reference to the teaching of Burger's Medicinal Chemistry And Drug Discovery, 5th Edition, Vol 1: Principles and Practice, which is incorporated herein by reference to the extent that it teaches physiologically functional derivatives.

As used herein, the term "solvate" preferably refers to a complex of variable stoichiometry formed by a solute (in this invention, a specific integrin ligand and/or a further cancer cotherapeutic agent (or a salt or physiologically functional derivative thereof)) and a solvent. Such solvents for the purpose of the invention may not interfere with the biological activity of the solute. Examples of suitable solvents include, but are not limited to, water, methanol, ethanol and acetic acid. Preferably the solvent used is a pharmaceutically acceptable solvent. Examples of suitable pharmaceutically acceptable solvents include, without limitation, water, ethanol and acetic acid. Most preferably the solvent used is water. Pharmaceutically acceptable salts of compounds to be used according to the invention and their preparation is known in the art. If the compound itself is not a salt, it can be easily transferred into a salt by addition of a pharmaceutically acceptable acid or of a pharmaceutically acceptable base. Pharmaceutically acceptable acids and bases are known in the art, for example from the literature cited herein.

The compounds to be used according to the invention, preferably the specific integrin ligand and/or at least one further cancer cotherapeutic agent different from the at least one specific integrin ligand, can generally be administered to the patient in a form and in a way or manner that is known in the art for the respective compounds or class of compounds, for example as describe herein or as described in the literature cited herein.

The specific integrin ligand cyclo-(Arg-Gly-Asp-DPhe-NMeVal) is preferably applied as a pharmaceutically acceptable salt, more preferably the pharmacologically acceptable hydrochloride salt, and especially preferably applied as the inner (or internal) salt, which is the compound cyclo-(Arg-Gly-Asp-DPhe-NMeVal) as such.

With regard to the specific integrin ligand cyclo-(Arg-Gly-Asp-DPhe-NMeVal), the following kinds of writing the name are preferably to be regarded as equivalent:

cyclo-(Arg-Gly-Asp-DPhe-[NMe]Val) = cyclo-(Arg-Gly-Asp-DPhe-[NMe]-Val) = cyclo-(Arg-Gly-Asp-DPhe-NMeVal) = cyclo-(Arg-Gly-Asp-DPhe-NMe-Val) = cyclo(Arg-Gly-Asp-DPhe-NMeVal) = cyclo(Arg-Gly-Asp-DPhe-NMe-Val) = cRGDfNMeV = c(RGDfNMeV).

The specific integrin ligand cyclo-(Arg-Gly-Asp-DPhe-NMeVal) is also referred to as Cilengitide, which is the INN (International Non-propriety Name) of said compound.

The specific integrin ligand cyclo-(Arg-Gly-Asp-DPhe-NMeVal) is also described in EP 0 770 622 A, US 6,001,961, WO 00/15244 and PCT/US07/01446 of the same applicant.

Recent results show that inhibiting integrins, especially $\alpha v \beta 3$ and/or $\alpha v \beta 5$, commonly expressed in various cancerous cells, can significantly decrease the resistance to chemotherapeutic agents and/or ionising radiation of otherwise chemo- or radioresistant cancerous cells and/or can induce an increased sensitivity of cancerous cells towards chemotherapeutic agents and/or ionising radiation.

Accordingly, specific integrin ligands, especially integrin ligands specific to $\alpha_v\beta_3$ and/or $\alpha_v\beta_5$ integrins according to the invention can be successfully applied to improve the efficacy of various cancer cotherapeutic agents.

For example, a phase I clinical study used cilengitide treatment in a dose escalation study on various brain tumors (NABT 9911). In some of the GBM patients in this study, an indication of response was seen. Cilengitide (= cyclo-(Arg-Gly-Asp-DPhe-NMe-Val), in very marked contrast to most cancer therapeutics currently in use has a very innocuous side effect profile, with no known MTD in humans - and is very well tolerated.

In addition to the essentially 100% mortality in GBM patients (2-year survival rate about 25%), the morbidity from neurological complications also rapidly degrades the quality of life (QOL).

For example, the standard of treatment of glioblastoma multiforme, associating radiotherapy and temozolomide; has only increased the median survival of resected patients by 2.5 months (12.1 $\rightarrow$ 14.6 months) compared to radiotherapy alone (Stupp et al., 2005). However, in combination with at least one specific integrin ligand according to the invention, preferably selected from Vitaxin, Abegrin, CNTO95 and cyclo-(Arg-Gly-Asp-DPhe-NMe-Val), more preferably selected from Vitaxin, Abegrin and cyclo-(Arg-Gly-Asp-DPhe-NMe-Val) and especially preferably cyclo-(Arg-Gly-Asp-DPhe-NMe-Val), this standard treatment shows significantly improved efficacy with respect to an increased median survival and quality of life. The literature cited in this paragraph is explicitly incorporated into the disclosure of the instant application by reference.

[For technical information only] SCCHN: Squamous Cell Cancer of the Head and Neck (also referred to as Squamous Cell Carcinoma of the Head and Neck):

The annual worldwide incidence of squamous cell cancer of the head and neck is estimated at 500,000 patients; in the United States and Europe, 118.000 new patients are diagnosed annually. SCCHN is more predominant in males with a male:female ratio of 2:1-4:1. There is a positive relationship between smoking habits, alcohol consumption, and head and neck cancer.

Approximately 90% of all head and neck malignancies are of squamous cell histology (SCCHN). Most patients are diagnosed with SCCHN at an age of 50-70 years.

A majority of patients (75%) have locally advanced disease at diagnosis. Those patients are mainly treated with radiotherapy and in some cases surgery. Newer strategies such as induction chemotherapy or chemoradiotherapy could provide better survival; however, the 5-year survival rate remains around 30%, and 60% of subjects will experience a loco-regional or distant relapse within 2 years of initial treatment.

The group of subjects with recurrent disease and/or with newly diagnosed distant metastases has very heterogeneous disease characteristics. Their median survival time, however, remains around 6-8 months with a poor quality of life. This dismal prognosis has not changed in the past 30 years. The standard chemotherapeutic treatments for recurrent and/or metastatic SCCHN include drugs such as methotrexate, bleomycin, 5-fluorouracil (5-FU), and platinum compounds. Promising phase II results with new agents such as taxanes could not be confirmed in phase III studies. Cisplatin is the most widely used drug for the treatment of recurrent and/or metastatic SCCHN and, as such, is considered the standard treatment in this indication. Overall, all published randomized trials suggest that cisplatin and 5-FU in combination produced higher response rates compared to single agents and most of the other combinations. In general, combination therapy was associated with higher hematological and non-hematological toxicity. The combination of cisplatin plus 5-FU produced a small but questionable improvement over monotherapy with a median survival of 6 to 8 months. Carboplatin + 5-FU containing regimens are also frequently used because of their better safety profile (lower renal, otologic, neurologic, and gastrointestinal toxicity than cisplatin). Response rates and survival are not statistically different from cisplatin-based regimens. Carboplatin is therefore approved for the treatment of SCCHN in several European countries.

The epidermal growth factor receptor (EGFR) is expressed in nearly all SCCHN. EGFR expression carries a strong prognostic significance, providing the rationale for using EGFR-targeted agents, such as cetuximab (Erbitux®), in this indication (Burtness, JCO 2005; Bourhis, JCO 2006). Erbitux is approved in the U.S. for monotherapy of metastatic disease, and in combination with radiotherapy for unresectable SCCHN, where it has demonstrated a prolongation of survival by 20 months.

A phase III trial with the combination of Cis- or Carboplatinum, 5-FU and Erbitux has been demonstrated to significantly prolong the median survival time in patients with locally recurrent/metastatic SCCHN. The observed median survival time of 10.1 months is among the longest ever reported in a phase III trial for these patients. The literature cited in this paragraph is explicitly incorporated into the disclosure of the instant application by reference.

[For technical information only] NSCLC: Non-Small Cell Lung Cancer Lung cancer is the leading cause of cancer deaths worldwide. About 170,000 new cases of lung cancer and 160,000 deaths due to this disease per year occur in the United States alone. NSCLC accounts for approximately 80% of all lung cancers.

At the time of diagnosis, approximately 30% of NSCLC patients present with locally advanced, and 40% with metastatic disease. Surgical results in earlier stages are poor compared to other tumor types (about 40% of recurrence in stages I-II). In metastatic disease, chemotherapy is the treatment of choice, but survival benefits have been modest, resulting in one-year survival of 40%, and five-year survival of less than 15%.

It is commonly accepted that the standard treatment for advanced disease (stage IV and IIIb with malignant pleural effusion) consists of platin-based (cisplatin or carboplatin) chemotherapy. However, there are many open questions in the management of these patients, such as the role of combination therapy regimen including more than two drugs, non-platinum-based therapies, and new targeted therapeutical approaches.

Currently, response rates of about 20%-30% and median survival times of 6 to 11 months have been observed in the treatment of metastatic NSCLC. Several chemotherapy combinations are used with comparable efficacy. The combinations of cis-/carboplatin plus vinorelbine, gemcitabine, paclitaxel, or docetaxel are among the regimens most commonly used for first-line therapy of metastatic NSCLC.

A phase III trial has been initiated based on the results of a randomized phase II trial in 86 patients treated with cisplatin/vinorelbine plus cetuximab versus cisplatin/ vinorelbine alone. The phase II trial revealed an advantage of the cetuximab combination with regard to overall response rate (53% in the experimental arm and 32% in the control arm [Gatzerneier, ASCO 2003, abstract # 2582]). The phase III trial planned to include 1100 patients (550 per arm), and was powered to demonstrate an increase in median overall survival from 7 months (standard arm) to 10 months (combination with Erbitux). This study has already finished enrollment, and first results are expected soon. The literature cited in this paragraph is explicitly incorporated into the disclosure of the instant application by reference.

[For technical information only] SCLC: Small Cell Lung Cancer Small cell lung cancer (SCLC) accounts for 15-20% of all lung cancer cases in the world, equating to approximately 80,000 new patients every year. A recent analysis of the Surveillance, Epidemiology and End Results database confirmed that in the United States, the proportion of small cell lung cancer patients has decreased from about 20% to 13.8% in 1998, likely due to the implementation of smoking cessation programs. This success, however, is to some extent outweighed by the high and rising prevalence of tobacco smoking in other parts of the world.

SCLC is typically disseminated at the time of presentation, with approximately 60% to 70% of patients having disseminated (extensive-stage) disease at presentation. Thus, surgery is rarely an option, and applies only to patients with localized (limited) disease. Relapse and death from SCLC is imminent even in patients who are treated with surgical resection. Without other therapy than surgery, survival was 2 months for patients with extensive-stage SCLC and 3 months for patients with limited-stage SCLC (Green, Am J Med 1969).

Systemic combination chemotherapy remains the mainstay of SCLC treatment, both in limited and extensive stage of their disease. For more than 20 years, etoposide and cis-/carboplatin are considered the current standard agents used in combination for the first-line treatment of patients with SCLC in the Western world. Combination therapy with more than two drugs in clinical trials has resulted in higher response rates, but also higher toxicity, and did not result in a clinically relevant overall survival benefit. A combination regimen consisting of cyclophosphamide, doxorubicin, and vincristine was shwn to be equally effective as the platinum/etoposide combination, but has a more unfavourable toxicity profile due to the inclusion of an anthracycline. In Japan, cisplatin plus irinotecan is used more frequently for the first-line treatment of SCLC after a Japanese trial has yielded favourable overall survival. Studies in the Western hemisphere, however, were not able to confirm those results, thus, this regimen is not used as widely in that part of the world.

In extensive stage SCLC, overall response rates to chemotherapy range from 40% to 70%. Time to progression is short, with the majority of patients progressing within 3 months of completing chemotherapy. The median survival is 7 to 11 months. Less than 5% of patients survive longer than 2 years. The literature cited in this paragraph is explicitly incorporated into the disclosure of the instant application by reference.

Thus, even in view of the results achieved within the last years, the prognosis of the patients regarding most cancerous diseases is still very grim. Thus, there is a need for improved medicaments, therapy methods and treatment regimen.

It is an objective of the instant invention to provide such improved medicaments for use in treatment of colon and/or rectal cancer and treatment regimens.

Thus, subject of the instant invention is:

[1] The use of at least one specific integrin ligand, comprising cyclo-(Arg-Gly-Asp-DPhe-NMe-Val), the pharmaceutically acceptable, solvates and/or salts thereof, for the manufacture of a medicament for the treatment of colon and/or rectal cancer, wherein the medicament is to be used in combination with

a) one or more alkylating chemotherapeutic agents, selected from the group consisting of the platinum containing compounds cisplatin, carboplatin and oxaliplatin, and
b) one or more further chemotherapeutic agents other than the at least one specific integrin ligand and the one or more alkylating chemotherapeutic agents, selected from the group consisting of:

i) EGFR inhibitors, selected from the group consisting of cetuximab, panitumumab, zalutumumab, nimotuzumab and matuzumab and/or the group consisting of gefitinib, erlotinib and lapatinib,
ii) cytostatic alkaloids, selected from the group consisting of etoposide, vinblastine and teniposide, the group consisting of vinorelbine, vincristine and vindesine, the group consisting of docetaxel and paclitaxel, and/or the group consisting of irinotecan and topotecan,
iii) cytostatic antibiotics, selected from the group consisting of doxorubicin, idarubicin, daunorubicin, epirubicin and valrubicin, and
iv) antimetabolites, selected from the group consisting of 5-fluorouracil, capecitabine, cytosinarabinosid and difluorodesoxycytidin and/or the group consisting of pemetrexed, methotrexat and raltitrexed,

and pharmaceutically acceptable derivatives, salts and/or solvates thereof.

The specific integrin ligand, comprising cyclo-(Arg-Gly-Asp-DPhe-NMe-Val), the pharmaceutically acceptable solvates and/or salts thereof, for use in the treatment of colon and/or rectal cancer in combination with

a) one or more alkylating chemotherapeutic agents, selected from the group consisting of the platinum containing compounds cisplatin, carboplatin and oxaliplatin, and
b) one or more further chemotherapeutic agents other than the at least one specific integrin ligand and the one or more alkylating chemotherapeutic agents, selected from the group consisting of:

i) EGFR inhibitors, selected from the group consisting of cetuximab, panitumumab, zalutumumab, nimotuzumab and matuzumab and/or the group consisting of gefitinib, erlotinib and lapatinib,
ii) cytostatic alkaloids, selected from the group consisting of etoposide, vinblastine and teniposide, the group consisting of vinorelbine, vincristine and vindesine, the group consisting of docetaxel and paclitaxel, and/or the group consisting of irinotecan and topotecan,
iii) cytostatic antibiotics, selected from the group consisting of doxorubicin, idarubicin, daunorubicin, epirubicin and valrubicin, and
iv) antimetabolites, selected from the group consisting of 5-fluorouracil, capecitabine, cytosinarabinosid and difluorodesoxycytidin and/or the group consisting of pemetrexed, methotrexat and raltitrexed,

and pharmaceutically acceptable derivatives, salts and/or solvates thereof.

The specific integrin ligand, comprising cyclo-(Arg-Gly-Asp-DPhe-NMe-Val), the pharmaceutically acceptable solvates and/or salts thereof, for use as described herein, wherein the at least one specific integrin ligand selected from the group consisting of cyclo-(Arg-Gly-Asp-DPhe-NMe-Val), the pharmaceutically acceptable solvates and/or salts thereof is administered to a patient in an amount of 250 mg to 12500 mg per week.

The further chemotherapeutic agents other than the at least one specific integrin ligand and the one or more alkylating chemotherapeutic agents in this respect are selected from cytostatic antibiotics, antimetabolites, cytostatic alkaloids and EGFR inhibitors as defined below.

Cytostatic antibiotics in this respect are selected from:

the Anthracyclines Daunorubicine, Doxorubicine, Epirubicine and Idarubicine; and pharmaceutically acceptable dervatives, salts and/or solvates thereof.

Antimetabolites in this respect are selected from:

the antifolates Methotrexate, Raltitrexed, and Pemetrexed; and
the pyrimidine antagonists 5-Fluorouracil, Capecitabine, Cytosinarabinoside and Difluorodesoxycytidine; and pharmaceutically acceptable dervatives, salts and/or solvates thereof.

Cytostatic alkaloids in this respect are selected from:

the podophyllotoxinderivatives Etoposide and Teniposide;
the vinca alkaloids Vinblastine, Vincristine, Vindesine and Vinorelbine;
the taxanes Docetaxel and Paclitaxel; and
Camptothecin derivatives, more preferably from the Camptothecin derivatives Irinotecane and Topotecane;

and pharmaceutically acceptable dervatives, salts and/or solvates thereof.

[For technical information only] A preferred cytostatic enzyme in this respect is L-asparaginase; and pharmaceutically acceptable dervatives, salts and/or solvates thereof.

EGFR inhibitors in this respect are selected from the group consisting of:

the anti-EGFR biologicals cetuximab, panitumumab, zalutumumab, nimotuzumab and matuzumab; and
the anti-EGFR chemically derived compounds gefitinib, erlotinib and lapatinib;

and pharmaceutically acceptable dervatives, salts and/or solvates thereof.

Generally, the at least one specific integrin ligand, the one or more alkylating chemotherapeutic agents (a), and/or the one or more further chemotherapeutic agents other than the at least one specific integrin ligand and the one or more alkylating chemotherapeutic agents (b) can be administered in an amount and/or a regimen as it is known in the art for the respective compound.

Preferably, the at least one specific integrin ligand, the one or more alkylating chemotherapeutic agents (a), and/or the one or more further chemotherapeutic agents other than the at least one specific integrin ligand and the one or more alkylating chemotherapeutic agents (b) are administered in an amount and/or a regimen as it is described above and/or below for the respective compound.

Preferably, the at least one specific integrin ligand, the one or more alkylating chemotherapeutic agents, and/or the one or more further chemotherapeutic agents other than the at least one specific integrin ligand and the one or more alkylating chemotherapeutic agents are administered in an amount and/or a regimen as it is described below for the respective compound and for the respective cancer.

More preferably, the at least one specific integrin ligand, the one or more alkylating chemotherapeutic agents (a), and/or the one or more further chemotherapeutic agents other than the at least one specific integrin ligand and the one or more alkylating chemotherapeutic agents (b) are administered in an amount and/or a regimen as it is described below for the respective compound and for the respective.

Preferably, the cancer types given herein also include metastases of the respective cancer in other organs or parts of the body of the subject. Examples of other organs or parts of the body of a subject that are prone to developing metastases include, but are not limited to lung, bone, liver, brain, kidney, adrenal gland, lymph nodes (including lymphangiosis carcinomatosa), heart and skin.

The use as described above and/or below, wherein the one or more further chemotherapeutic agents other than the at least one specific integrin ligand and the one or more alkylating chemotherapeutic agents (b) is selected from the group consisting of:

i) EGFR inhibitors,
ii) cytostatic alkaloids,
iii) cytostatic antibiotics, and
iv) antimetabolites,

and pharmaceutically acceptable dervatives, salts and/or solvates thereof.

The use as described above and/or below, wherein the one or more further chemotherapeutic agents other than the at least one specific integrin ligand and the one or more alkylating chemotherapeutic agents (b) are selected from the group consisting of:

i) EGFR inhibitors, selected from anti-EGFR biologicals and chemically derived compounds,
ii) cytostatic alkaloids, selected from podophylotoxines, vinca alkaloids, taxanes and campthothecines,
iii) cytostatic antibiotics, selected from anthracyclines, and
iv) antimetabolites, selected from pyrimidin antagonists and antifolates, and pharmaceutically acceptable dervatives, salts and/or solvates thereof.

Anti-EGFR biologicals in this respect are selected from cetuximab, panitumumab, zalutumumab, nimotuzumab and matuzumab;
Anti-EGFR chemically derived compounds in this respect are selected from gefitinib, erlotinib and lapatinib;
Podophyllotoxinderivatives in this respect are selected from Etoposide and Teniposide;
Vinca alkaloids in this respect are selected from Vinblastine, Vincristine, Vindesine and Vinorelbine;
Taxanes in this respect are selected from Docetaxel and Paclitaxel; Camptothecin derivatives in this respect are preferably selected from Irinotecane and Topotecane;
Anthracyclines in this respect are selected from Daunorubicine,
Doxorubicine, Epirubicine and Idarubicine;
Antifolates in this respect are selected from Methotrexate, Raltitrexed, and Pemetrexed;
Pyrimidine antagonists in this respect are selected from 5-Fluorouracil, Capecitabine, Cytosinarabinoside and Difluorodesoxycytidine;
and pharmaceutically acceptable dervatives, salts and/or solvates thereof.
The use as described above and/or below, wherein the one or more further chemotherapeutic agents other than the at least one specific integrin ligand.and the one or more alkylating chemotherapeutic agents (b) are selected from the group consisting of:

i) EGFR inhibitors, selected from the group consisting of cetuximab, panitumumab, zalutumumab, nimotuzumab and matuzumab and/or the group consisting of gefitinib, erlotinib and lapatinib,
ii) cytostatic alkaloids, selected from the group consisting of etoposide, vinblastine and teniposide, the group consisting of vinorelbine, vincristine and vindesine, the group consisting of docetaxel and paclitaxel, and/or the group consisting of irinotecan and topotecan,
iii) cytostatic antibiotics, selected from the group consisting of doxorubicin, idarubicin, daunorubicin, epirubicin and valrubicin, and
iv) antimetabolites, selected from the group consisting of 5-fluorouracil, capecitabine, cytosinarabinosid and difluorodesoxycytidin and/or the group consisting of pemetrexed, methotrexat and raltitrexed,

and pharmaceutically acceptable dervatives, salts and/or solvates thereof.
The use as described above and/or below, wherein the at least one specific integrin ligand selected from the group consisting of cyclo-(Arg-Gly-Asp-DPhe-NMe-Val), the pharmaceutically acceptable solvates and/or salts thereof is administered to a patient in an amount of 250 mg to 12500 mg per week.
The use as described above and/or below and especially as described in one or more of the paragraphs numbered [1] to [13], wherein the platinum containing chemotherapeutic agents cisplatin, carboplatin and oxaliplatin are administered to the patient in an amount of 100 to 1000 mg in one or more portions within a time period of 2 to 4 weeks.
The treatment of cancer, at least the treatment of cancer with chemotherapeutic agents in the broadest sense, is a protracted issue. Thus, the treatment of cancer with chemotherapeutic agents generally includes a prolonged exposure to the one or more respective chemotherapeutic agents. Taking into account that most of the chemotherapeutic agents, when applied in an efficient dose, are toxic for the body of the patient, the chemotherapeutic agents (unless they show any or hardly any acute toxicity) are generally applied over a certain, limited time, followed by a time period without the administration of the respective chemotherapeutic agent, during which time the patient's body is allowed to recover from the toxicity of said chemotherapeutic agent. generally, this treatment regimen comprising the application time period of the respective chemotherapeutic agent and the recovery time period after the application of the respective chemotherapeutic agent is repeated one or more times, preferably several times. This kind of regimen is usually referred to by the skilled artisan as "cycles", each cycle comprising the application time period of the respective chemotherapeutic agent and the recovery time period after the application of the respective chemotherapeutic agent. The duration of the application time period and/or the recovery time period after the application of the chemotherapeutic are usually depending on the properties of the respective chemotherapeutic agent. Accordingly, different chemotherapeutic agents can have different durations of the application time period and/or the recovery time period after the of the chemotherapeutic. Thus, the length or duration of a cycle can be different for different chemotherapeutic agents. Generally, the length of a cycle is in between one week and 12 weeks, more preferably one week to six weeks and especially 2 to 4 weeks. Preferably, the dosing of the respective chemotherapeutic agent is given in an amount per cycle, allowing the the physicist to adapt the actual administration to the status of the patient, i.e. whether the amount per cycle is given in one single administration or divided into two or more portions administered at different times within the cycle. In the setting of a combination treatment comprising two or more chemotherapeutic agents, generally two or more cycles (having the same or a different length) run in parallel. If the chemotherapeutic agent is administered to the patient in two or more portions within one cycle, each portion is preferably given on a different day within said cycle. With respect to each of the chemotherapeutics administered, generally more than one cycle, preferably two or more cycles, even more preferably three or more cycles are applied to the patient, preferably substantially with out a pause. Generally, not more than 24 cycles are applied to the patient substantially without a pause. The application of about six cycles substantially without a pause to the patient for each of the chemotherapeutics administered is generally a standard for of many of the chemotherapeutics described herein.

Accordingly, the time period of 2 to 4 weeks referred to in the paragraph numbered [14], wherein the platinum containing chemotherapeutic agents cisplatin, carboplatin and oxaliplatin are administered to the patient in an amount of 100 to 1000 mg in one or more portions (within said time period of 2 to 4 weeks) is preferably to be regarded as one cycle. More preferably, the time period or cycle, wherein the platinum containing therapeutic agent is administered is about three weeks (about 21 days). With respect to oxaliplatin, following administration is also preferred: oxaliplatin is preferably administered to the patient in an amount of 50 to 500 mg in one or more portions, preferably one portion, within a time period of about two weeks. Accordingly, the duration of a cycle with respect to oxaliplatin is preferably about two weeks.

Generally, the cisplatin can be administered to the patient as is known in the art.

Preferably, cisplatin is administered to the patient in an amount of 50 mg to 500 mg within one cycle, more preferably 80 mg to 300 mg within one cycle. Preferably, the amount of cisplatin is administered to the patient is given in mg per square metre of the by the surface of the patient, i.e. in mg/m$^2$. Accordingly, cisplatin is preferably administered to the patient in an amount of 50 to 150 mg/m$^2$, more preferably 80 to 120 mg/m$^2$ and especially about 100 mg/m$^2$ within one cycle.

The amount cisplatin can be administered in one or more portions, more preferably 1 to 5 portions, even more preferred 1 to 3 and especially preferably in one portion on one day. Generally, cisplatin is administered as an i. V. infusion.

Generally, the carboplatin can be administered to the patient as is known in the art.

Preferably, carboplatin is administered to the patient in an amount of 200 mg to 1000 mg within one cycle, more preferably 300 mg to 800 mg within one cycle and especially 400 to 700 mg within one cycle. Even more preferably, the carboplatin is administered to the patient in an AUC (Area Under the Curve) regimen, more specifically an AUC 4-8 regimen (4-8 mg/ml/min), preferably an AUC 5-7 regimen (5-7 mg/ml/min). The principles of the AUC regimen or dosing are known in the art. Preferably, the amounts to be administered to the patient in the AUC regimen according to the invention are calculated using the Calvert formula and/or the Chatelut formula, preferably the Calvert formula.

Calvert Formula:

$$\text{Carboplatin dose (mg)} = \text{AUC} \times (\text{CrCl (ml/min)} + 25);$$

wherein:

$$\text{AUC} = \text{Area Under the Curve (mg/ml} \times \text{min)}$$

$$\text{x} = \text{multiplied}$$

CrCl = Creatinin Clearence (of the respective patient)

Chatelut formula:

$$\text{Carboplatin dosage (mg)} = \text{AUC (mg/ml} \times \text{min)} \times \text{carboplatin clearance (ml/min);}$$

wherein:

## AUC = Area Under the Curve

Formula suitable for estimation of the carboplatin clearance of a patient for use in the Chatelut formula:

$$\text{for Males} = (0.134 \times \text{weight}) + (218 \times \text{weight} \times (1 - 0.00457 \times \text{age})/\text{serum creat.})$$

$$\text{for Females} = (0.134 \times \text{weight}) + 0.686 \times (218 \times \text{weight} \times (1 - 0.00457 \times \text{age})/\text{serum creat.})$$

Age = age in years
x = multiplied
weight = weight in kg serum creat. = the serum concentration of creatinine

The amount carboplatin can be administered in one or more portions, more preferably 1 to 5 portions, even more preferred 1 to 3 and especially preferably in one portion on one day. Generally, carboplatin is administered as an i. V. infusion.

Generally, the oxaliplatin can be administered to the patient as is known in the art.

Preferably, oxaliplatin is administered to the patient in an amount of 50 mg to 500 mg within one cycle, more preferably 80 mg to 300 mg within one cycle. If the duration of the cycle is about three or about five weeks, the oxaliplatin is preferably administered to the patient in an amount of 100 to 500 mg. If the duration of the cycle is about two weeks, the oxaliplatin is preferably administered to the patient in an amount of 50 to 250 mg. Preferably, the amount of oxaliplatin is administered to the patient is given in mg per square metre of the by the surface of the patient, i.e. in $mg/m^2$. Accordingly, oxaliplatin is preferably administered to the patient in an amount of 80 to 150 $mg/m^2$ within one cycle, for example about 130 $mg/m^2$ within one cycle, especially if the duration of the cycle is about three or about four weeks. Alternatively, the oxaliplatin is preferably administered to the patient in an amount of 50 to 100 $mg/m^2$ within one cycle, for example about 85 $mg/m^2$ within one cycle, especially if the duration of the cycle is about two weeks.

The amount oxaliplatin can be administered in one or more portions, more preferably 1 to 5 portions, even more preferred 1 to 3 and especially preferably in one portion on one day. Generally, oxaliplatin is administered as an i. V. infusion.

[I] Generally, the cyclo-(Arg-Gly-Asp-DPhe-NMe-Val), the pharmaceutically acceptable solvates and/or salts thereof, preferably cyclo-(Arg-Gly-Asp-DPhe-NMe-Val), can be administered to the patient as it is known in the art.

[II] Preferably, cyclo-(Arg-Gly-Asp-DPhe-NMe-Val), the pharmaceutically acceptable solvates and/or salts thereof and preferably cyclo-(Arg-Gly-Asp-DPhe-NMe-Val), is administered to the patient in an amount of 250 mg to 12500 mg, more preferably 450 to 10500 mg, within a time period of one week. This is also referred to as the weekly administration with respect to cyclo-(Arg-Gly-Asp-DPhe-NMe-Val). Preferably, a weekly administration of the given amounts takes place two or more times, preferably two or three times, within a time period of about three weeks. Preferably, a weekly administration of the given amounts takes place two or more times, preferably two, three or four times , within a time period of about four weeks. Preferably, the weekly administration with respect to cyclo-(Arg-Gly-Asp-DPhe-NMe-Val) takes place during two or more weeks within the cycle or the cycles with respect to the

    a) one or more alkylating chemotherapeutic agents, and/or
    b) one or more further chemotherapeutic agents other than the at least one specific integrin ligand and the one or more alkylating chemotherapeutic agents.

[III] Even more preferably, the weekly administration with respect to cyclo-(Arg-Gly-Asp-DPhe-NMe-Val) takes place during every week within the cycle or the cycles with respect to the

    a) one or more alkylating chemotherapeutic agents, and/or
    b) one or more further chemotherapeutic agents other than the at least one specific integrin ligand and the one or more alkylating chemotherapeutic agents.

[IV] The amount of cyclo-(Arg-Gly-Asp-DPhe-NMe-Val), the pharmaceutically acceptable solvates and/or salts thereof and preferably cyclo-(Arg-Gly-Asp-DPhe-NMe-Val) to be administered in the weekly administration with respect to cyclo-(Arg-Gly-Asp-DPhe-NMe-Val) can be a the same or different in each week.

[V] The following dosings or regimen are preferred in this respect:

(A) The cyclo-(Arg-Gly-Asp-DPhe-NMe-Val), the pharmaceutically acceptable solvates and/or salts thereof and preferably cyclo-(Arg-Gly-Asp-DPhe-NMe-Val) is administered to the patient in an amount of about 500 mg or about 2000 mg once a week each week during the cycle or the cycles with respect to the

a) one or more alkylating chemotherapeutic agents, and/or
b) one or more further chemotherapeutic agents other than the at least one specific integrin ligand and the one or more alkylating chemotherapeutic agents.

(B) The cyclo-(Arg-Gly-Asp-DPhe-NMe-Val), the pharmaceutically acceptable solvates and/or salts thereof and preferably cyclo-(Arg-Gly-Asp-DPhe-NMe-Val) is administered to the patient in an amount of about 500 mg or about 2000 mg twice a week each week during the cycle or the cycles with respect to the

a) one or more alkylating chemotherapeutic agents, and/or
b) one or more further chemotherapeutic agents other than the at least one specific integrin ligand and the one or more alkylating chemotherapeutic agents.

(C) The cyclo-(Arg-Gly-Asp-DPhe-NMe=Val), the pharmaceutically acceptable solvates and/or salts thereof and preferably cyclo-(Arg-Gly-Asp-DPhe-NMe-Val) is administered to the patient in an amount of about 500 mg each day on five consecutive days within one first week and in an amount of about 500 mg on one day within each further week during the cycle or the cycles with respect to the

a) one or more alkylating chemotherapeutic agents, and/or
b) one or more further chemotherapeutic agents other than the at least one specific integrin ligand and the one or more alkylating chemotherapeutic agents.

(D) Alternatively preferably, the cyclo-(Arg-Gly-Asp-DPhe-NMe-Val), the pharmaceutically acceptable dervatives, solvates and/or salts thereof and preferably cyclo-(Arg-Gly-Asp-DPhe-NMe-Val) is administered to the patient in an amount of about 2000 mg each day on three consecutive days within one first week and in an amount of about 2000 mg on one day within each further week during the cycle or the cycles with respect to the

a) one or more alkylating chemotherapeutic agents, and/or
b) one or more further chemotherapeutic agents other than the at least one specific integrin ligand and the one or more alkylating chemotherapeutic agents.

(E) Preferably, the cyclo-(Arg-Gly-Asp-DPhe-NMe-Val), the pharmaceutically acceptable solvates and/or salts thereof and preferably cyclo-(Arg-Gly-Asp-DPhe-NMe-Val) is administered to the patient in an amount of about 2000 mg once a week each week during the cycle or the cycles with respect to the

a) one or more alkylating chemotherapeutic agents, and/or
b) one or more further chemotherapeutic agents other than the at least one specific integrin ligand and the one or more alkylating chemotherapeutic agents.

[VI] Preferably, more than one cycle with respect to the

a) one or more alkylating chemotherapeutic agents, and/or
b) one or more further chemotherapeutic agents other than the at least one specific integrin ligand and the one or more alkylating chemotherapeutic agents

is applied to the patient. More preferably 2 to 12 cycles and especially about 6 cycles are applied to the patient, preferably comprising one or more of the regimen (A) to (E).

[VII] Preferably, the more than one cycles comprise only one of the regimen selected from (A) to (E), i.e. the same regimen selected from (A) to (E) is applied to the patient in each of the cycles. More preferably the same regimen selected from (A) to (E) is applied to the patient in each of the about 6 cycles.

[VIII] Alternatively preferably, the more than one cycles comprise two or more of the regimen selected from (A) to (E), i.e. in different cycles different regimen selected from (A) to (E) are applied to the patient.

[IX] Thus, in cases wherein more than one cycle with respect to the

a) one or more alkylating chemotherapeutic agents, and/or
b) one or more further chemotherapeutic agents other than the at least one specific integrin ligand and the one or more alkylating chemotherapeutic agents is applied to the patient, combinations of one or more of the dosings or regimen (A) to (E) are also or preferred in this respect:

(F) Regimen (C) is applied to the patient for the first cycle, followed by regimen (A) for 1 to 11 cycles and especially about 5 cycles. Preferably, during the regimen (A), the weekly administration consists of about 500 mg.

(G) Regimen (D) is applied to the patient for the first cycle, followed by regimen (A) for 1 to 11 cycles and especially about 5 cycles. Preferably, during the regimen (A), the weekly administration consists of about 2000 mg.

[X] Preferably in this respect and especially with respect to one or more of the regimen (A) to (G), the duration of one cycle, preferably each cycle, is about three weeks (about 21 days) or about four weeks (about 28 days), more preferably about three weeks (about 21 days).

[XI] However, due to the extremely low toxicity of the cyclo-(Arg-Gly-Asp-DPhe-NMe-Val), the pharmaceutically acceptable dervatives, solvates and/or salts thereof and preferably cyclo-(Arg-Gly-Asp-DPhe-NMe-Val), it can be also applied to the patient outside the cycles with respect to a) one or more alkylating chemotherapeutic agents, and/or

b) one or more further chemotherapeutic agents other than the at least one specific integrin ligand and the one or more alkylating chemotherapeutic agents, preferably in a dosing or regimen as described above and/or below. This is especially advantageous as a maintenance therapy consisting of or comprising, preferably consisting of the administration of the cyclo-(Arg-Gly-Asp-DPhe-NMe-Val), the pharmaceutically acceptable dervatives, solvates and/or salts thereof and preferably cyclo-(Arg-Gly-Asp-DPhe-NMe-Val) for one or months, for example for up to 24 months, even substantially without a pause. Cisplatin, carboplatin, oxaliplatin, cyclo-(Arg-Gly-Asp-DPhe-NMe-Val), cetuximab, matuzumab, doxorubicine, irinotecane, vincristine, cyclophamide, gemcitabine, paclitaxel, docetaxel, pemetrexed and/or 5-fluorouracil are typically administered as an i. V. infusion.

Etoposide, cyclophosphamide and vinorelbine are typically administered either orally or as an i. V. infusion.
However, other administration forms can generally be applied according to the invention, if available.
[17] The use as described above and/or below,
wherein the cytostatic alkaloid is selected from the group consisting of etoposide, Irinotecan and vincristine, preferably etoposide, and wherein the cytostatic antibiotic is selected from the group consisting of doxorubicine and idarubicine, preferably doxorubicine;
and the pharmaceutically acceptable dervatives, salts and/or solvates thereof.
Generally, the etoposide, Irinotecan, vincristine, doxorubicine and idarubicine can be administered to the patient as it is known in the art.
Preferably, etoposide is administered to the patient in an amount of 300 mg to 1000 mg, more preferably 500 to 900 mg, within a time period of 2 to 4 weeks and preferably within a time period of about three weeks, which time periods are preferably to be regarded as one cycle. More preferably, the amount of etoposide administered to the patient is given in mg per square metre of the by the surface of the patient, i.e. in mg/m$^2$. Accordingly, more preferably the etoposide is administered to the patient in an amount of 200 mg/m$^2$ to 600 mg/m$^2$, more preferably 250 mg/m$^2$ to 450 mg/m$^2$, for example in an amount of about 300 mg/m$^2$, within a time period of 2 to 4 weeks and preferably within a time period of about three weeks, which time periods are preferably to be regarded as one cycle. Even more preferably, the amount of etoposide to be administered to the patient is divided into three about equal portions that are administered to the patient on three different days, preferably three consecutive days and more preferably three consecutive days at the beginning of one cycle with respect to the etoposide. Especially preferably, the etoposide is administered to the patient in an amount of about 100 mg/m$^2$ per day on the days 1, 2 and 3 of a cycle consisting of about 21 days. Preferably, 2 to 12 cycles, more preferably 4 to 8 cycles and especially about 6 cycles are applied to the patient with respect to etoposide, preferably substantially without a pause. The whole procedure/regimen described above with respect to the etoposide can be repeated one or more times, preferably one to 12 times and especially 2 to 6 times, for example about 5 times, preferably with a pause in between each repetition of the procedure/regimen.

Preferably, vincristine is administered to the patient in an amount of 1 mg to 50 mg, more preferably 2 to 10 mg, within a time period of 2 to 4 weeks and preferably within a time period of about three weeks, which time periods are preferably to be regarded as one cycle. More preferably, the amount of vincristine administered to the patient is given in mg per square metre of the by the surface of the patient, i.e. in mg/m$^2$. Accordingly, more preferably the vincristine is administered to the patient in an amount of 1 mg/m$^2$ to 10 mg/m$^2$, more preferably 1 mg/m$^2$ to 2 mg/m$^2$, for example in an amount of about 1.4 mg/m$^2$, within a time period of 2 to 4 weeks and preferably within a time period of about three weeks, which time periods are preferably to be regarded as one cycle. Especially preferably, the vincristine is administered to the patient in an amount of about 1.4 mg/m$^2$ per day, preferably on day 1 of a cycle consisting of about 21 days. Preferably, 2 to 12 cycles, more preferably 4 to 8 cycles and especially about 6 cycles are applied to the patient with respect to vincristine, preferably substantially without a pause. The whole procedure/regimen described above with respect to the vincristine can be repeated one or more times, preferably one to 12 times and especially 2 to 6 times, for example about 5 times, preferably with a pause in between each repetition of the procedure/regimen.

Preferably, doxorubicine is administered to the patient in an amount of 20 mg to 300 mg, more preferably 40 to 200 mg, within a time period of 2 to 4 weeks and preferably within a time period of about three weeks, which time periods are preferably to be regarded as one cycle. More preferably, the amount of doxorubicine administered to the patient is given in mg per square metre of the by the surface of the patient, i.e. in mg/m$^2$. Accordingly, more preferably the doxorubicine is administered to the patient in an amount of 30 mg/m$^2$ to 100 mg/m$^2$, more preferably 40 mg/m$^2$ to 60 mg/m$^2$, for example in an amount of about 50 mg/m$^2$, within a time period of 2 to 4 weeks and preferably within a time period of about three weeks, which time periods are preferably to be regarded as one cycle. Even more preferably, the amount of doxorubicine to be administered to the patient is administered to the patient on one day, preferably at the beginning of one cycle with respect to the doxorubicine. Especially preferably, the doxorubicine is administered to the patient in an amount of about 40 mg/m$^2$ to 60 mg/m$^2$ per day on day 1 of a cycle consisting of about 21 days. Preferably, 2 to 12 cycles, more preferably 4 to 8 cycles and especially about 6 cycles are applied to the patient with respect to doxorubicine, preferably substantially without a pause. The whole procedure/regimen described above with respect to the doxorubicine can be repeated one or more times, preferably one to 12 times and especially 2 to 6 times, for example about 5 times, preferably with a pause in between each repetition of the procedure/regimen.

Preferably, Irinotecan is administered to the patient in an amount of 20 mg to 300 mg, more preferably 40 to 200 mg, within a time period of 2 to 4 weeks and preferably within a time period of about three weeks, which time periods are preferably to be regarded as one cycle. More preferably, the amount of Irinotecan administered to the patient is given in mg per square metre of the by the surface of the patient, i.e. in mg/m$^2$. Accordingly, more preferably the Irinotecan is administered to the patient in an amount of 30 mg/m$^2$ to 100 mg/m$^2$, more preferably 50 mg/m$^2$ to 70 mg/m$^2$, for example in an amount of about 60 mg/m$^2$, within a time period of 2 to 4 weeks and preferably within a time period of about three weeks, which time periods are preferably to be regarded as one cycle. Even more preferably, the amount of Irinotecan to be administered to the patient is administered to the patient on one day, preferably at the beginning of one cycle with respect to the Irinotecan. Especially preferably, the Irinotecan is administered to the patient in an amount of about 40 mg/m$^2$ to 60 mg/m$^2$ per day on days 1 of a cycle consisting of about 21 days. Preferably, 2 to 12 cycles, more preferably 4 to 8 cycles and especially about 6 cycles are applied to the patient with respect to Irinotecan, preferably substantially without a pause. The whole procedure/regimen described above with respect to the Irinotecan can be repeated one or more times, preferably one to 12 times and especially 2 to 6 times, for example about 5 times, preferably with a pause in between each repetition of the procedure/regimen.

Etoposide is especially preferred in this aspect.

The use as described above and/or below, wherein

i) the one or more alkylating chemotherapeutic agents (a) are selected from the group consisting of the platinum containing chemotherapeutic agents cisplatin, carboplatin and oxaliplatin, more preferably consisting of cisplatin and carboplatin,
ii) the one or more further chemotherapeutic agents other than the at least one specific integrin ligand and the one or more alkylating chemotherapeutic agents (b) are selected from the group consisting of the podophylotoxines etoposide, vinblastine and teniposide, preferably etposide;

and the pharmaceutically acceptable dervatives, salts and/or solvates thereof.

Preferably, the cisplatin, carboplatin, oxaliplatin, etoposide, vinblastine and teniposide are administered to the patient as it is known in the art and even more preferably as it is described above and/or below and especially as described in one or more of the paragraphs related to and given below the paragraph numbered [17]. More preferably, the cisplatin, carboplatin and/or oxaliplatin is administered to the patient as it is described in the paragraphs following the paragraph numbered [14] and preferably before the paragraph numbered[16].

The use as described above and/or below, wherein

i) the at least one specific integrin ligand is selected from the group consisting of cyclo-(Arg-Gly-Asp-DPhe-NMe-Val), the pharmaceutically acceptable solvates and/or salts thereof,
ii) the one or more alkylating chemotherapeutic agents (a) are selected from the group consisting of the platinum containing chemotherapeutic agents cisplatin, carboplatin and oxaliplatin, preferably cisplatin and carboplatin, and
iii) the one or more further chemotherapeutic agents other than the at least one specific integrin ligand and the one or more alkylating chemotherapeutic agents (b) is selected from the group consisting of etoposide, vinblastine and vincristine, preferably etoposide;

and the pharmaceutically acceptable dervatives, salts and/or solvates thereof.

The use as described above and/or below, wherein the at least one specific integrin ligand selected from the group consisting of cyclo-(Arg-Gly-Asp-DPhe-NMe-Val), the pharmaceutically acceptable solvates and/or salts thereof is administered to a patient in an amount of 800 mg to 8000 mg per week.

The use as described above and/or below, , wherein the at least one specific integrin ligand selected from the group consisting of cyclo-(Arg-Gly-Asp-DPhe-NMe-Val), the pharmaceutically acceptable solvates and/or salts thereof is administered to a patient in an amount of 1500 mg to 7000 mg per week.

The use as described above and/or below, , wherein the at least one specific integrin ligand selected from the group consisting of cyclo-(Arg-Gly-Asp-DPhe-NMe-Val), the pharmaceutically acceptable solvates and/or salts thereof is administered to a patient in a twice weekly to four times weekly administration scheme consisting of about 500 mg or about 2000 mg per administration.

More preferably, cyclo-(Arg-Gly-Asp-DPhe-NMe-Val), the pharmaceutically acceptable solvates and/or salts thereof is administered to the patient as described in one or more of the paragraphs numbered [I] to [XI].

The use as described above and/or below, wherein

ii) the one or more alkylating chemotherapeutic agents (a) selected from the group consisting of the platinum containing chemotherapeutic agents cisplatin, carboplatin and oxaliplatin are administered to the patient in an amount of 100 to 1000 mg in one or more portions within a time period of 2 to 4 weeks, and
iii) the one or more further chemotherapeutic agents other than the at least one specific integrin ligand and the one or more alkylating chemotherapeutic agents (b) selected from the group consisting of etoposide, vinblastine and vincristine are administered to the patient in an amount of 50 to 1000 mg in one or more portions within a time period of 2 to 4 weeks.

The time period of 2 to 4 weeks referred to in the paragraph numbered [23], wherein the platinum containing chemotherapeutic agents cisplatin, carboplatin and oxaliplatin are administered to the patient in an amount of 100 to 1000 mg in one or more portions (within said time period of 2 to 4 weeks) is preferably to be regarded as one cycle. More preferably, time period or cycle, wherein the platinum containing therapeutic agent is administered is about three weeks (about 21 days). With respect to oxaliplatin, following administration is also preferred: oxaliplatin is preferably administered to the patient in an amount of 50 to 500 mg in one or more portions, preferably one portion, within a time period of about two weeks. Accordingly, the duration of a cycle with respect to this oxaliplatin regimen is preferably about two weeks.

Generally, the cisplatin can be administered to the patient as is known in the art.

Generally, the carboplatin can be administered to the patient as is known in the art.

Generally, the etoposide can be administered to the patient as it is known in the art.

Preferably, vinblastine and vincristine are administered to the patient as it is known in the art and even more preferably as it is described above and/or below and especially as described in one or more of the paragraphs related to and given below the paragraph numbered [17].

Generally, the at least one specific integrin ligand as described herein, the one or more alkylating chemotherapeutic agents (a) as described herein, and/or the one or more further chemotherapeutic agents other than the at least one specific integrin ligand and the one or more alkylating chemotherapeutic agents (b) as described herein can be administered in an amount and/or a regimen as it is known in the art for the respective compound.

Preferably, the at least one specific integrin ligand as described herein, the one or more alkylating chemotherapeutic agents (a) as described herein, and/or the one or more further chemotherapeutic agents other than the at least one specific integrin ligand and the one or more alkylating chemotherapeutic agents (b) as described herein are administered in an amount and/or a regimen as it is described above and/or below for the respective compound.

Alkylating chemotherapeutic agents in this respect are selected from the Platin derivatives Cisplatin, Carboplatin and Oxaliplatin;
and pharmaceutically acceptable dervatives, salts and/or solvates thereof.

Antimetabolites in this respect are selected from:

the antifolates Methotrexate, Raltitrexed, and Pemetrexed; and
the pyrimidine antagonists 5-Fluorouracil, Capecitabine, Cytosinarabinoside and Difluorodesoxycytidine; and pharmaceutically acceptable dervatives, salts and/or solvates thereof.

Cytostatic alkaloids in this respect are selected from:

the podophyllotoxin-derivatives Etoposide and Teniposide;
the vinca alkaloids Vinblastine, Vincristine, Vindesine and Vinorelbine;
the taxanes Docetaxel and Paclitaxel; and
the Camptothecin derivatives Irinotecane and Topotecane;

and pharmaceutically acceptable dervatives, salts and/or solvates thereof.

EGFR inhibitors in this respect are preferably selected from the group consisting of:

Anti-EGFR biologicals, more preferably from the anti-EGFR biologicals cetuximab, panitumumab, zalutumumab, nimotuzumab and matuzumab; and
anti-EGFR chemically derived compounds, more preferably from the anti-EGFR chemically derived compounds gefitinib, erlotinib and lapatinib;

and pharmaceutically acceptable dervatives, salts and/or solvates thereof.

EGFR inhibitors in this respect are more preferably selected from the group consisting of cetuximab, panitumumab, zalutumumab, nimotuzumab and matuzumab;
and pharmaceutically acceptable dervatives, salts and/or solvates thereof.

EGFR inhibitors in this respect are especially preferably selected from the group consisting of cetuximab and matuzumab;
and pharmaceutically acceptable dervatives, salts and/or solvates thereof.

Preferably, the cisplatin, carboplatin and/or oxaliplatin are administered to the patient as it is known in the art and even more preferably as it is described above and/or below. More preferably, the cisplatin, carboplatin and/or oxaliplatin is administered to the patient as it is described in the paragraphs following the paragraph numbered [14] and preferably before the paragraph numbered [15] and/or as it is described in the paragraphs following the paragraph numbered [23] and preferably before the paragraph numbered [24].

The use as described above and/or below, ;
and the paragraphs directly related thereto,
wherein

i) the platinum containing chemotherapeutic agent is selected from the group consisting of cisplatin, carboplatin and oxaliplatin,
ii) the antimetabolite is selected from the group consisting of antifolates and pyrimidine antagonists, and
iii) the cytostatic alkaloid is selected from the group consisting of vinca alkaloids, podophylotoxines and taxanes,
iv) the EGFR inhibitor is selected from the group consisting of anti-EGFR biologicals and chemically derived compounds;

and the pharmaceutically acceptable dervatives, salts and/or solvates thereof.

The use as described above and/or below,
wherein the EGFR inhibitor is selected from the group consisting of cetuximab, panitumumab, zalutumumab, nimotuzumab and matuzumab and/or the group consisting of gefitinib, erlotinib and lapatinib, the cytostatic alkaloid is selected from the group consisting of vinorelbine and vincristine and/or the group consisting of paclitaxel and docetaxel, and the antimetabolite is selected from the group consisting of gemcitabine and pemetrexed.

In this regard,

i) the platinum containing chemotherapeutic agent is selected from the group consisting of cisplatin, carboplatin and oxaliplatin,

ii) the antimetabolite is selected from the group consisting of the antifolates Methotrexate, Raltitrexed and Pemetrexed and pharmaceutically acceptable salts and/or solvates thereof, and the pyrimidine antagonists 5-Fluorouracil, Capecitabine, Cytosinarabinoside and Difluorodesoxycytidine and pharmaceutically acceptable salts and/or solvates thereof, and

iii) the cytostatic alkaloid is selected from the group consisting of the vinca alkaloids vinblastine, Vincristine, Vindesine and Vinorelbine and pharmaceutically acceptable salts and/or solvates thereof, the podophylotoxines Etoposide and Teniposide and pharmaceutically acceptable salts and/or solvates thereof, and the taxanes Docetaxel and Paclitaxel and pharmaceutically acceptable salts and/or solvates thereof,

iv) the EGFR inhibitor is selected from the group consisting of the anti-EGFR biologicals cetuximab, panitumumab, zalutumumab, nimotuzumab and matuzumab and pharmaceutically acceptable salts and/or solvates thereof, and the chemically derived compounds gefitinib, erlotinib and lapatinib and pharmaceutically acceptable salts and/or solvates thereof.

Generally, the EGFR inhibitors selected from the group consisting of cetuximab, panitumumab, zalutumumab, nimotuzumab and matuzumab and/or the group consisting of gefitinib, erlotinib and lapatinib, can be administered to the patient as it is known in the art.

Preferably, cetuximab is administered to the patient in an amount of 500 mg to 3000 mg, more preferably 800 to 2500 mg, within a time period of 2 to 4 weeks and preferably within a time period of about three weeks or about four weeks weeks, which time periods are preferably to be regarded as one cycle. More preferably, the amount of cetuximab administered to the patient is given in mg per square metre of the by the surface of the patient, i.e. in $mg/m^2$. Accordingly, more preferably the cetuximab is administered to the patient in an amount of 500 $mg/m^2$ to 2000 $mg/m^2$, more preferably 750 $mg/m^2$ to 1500 $mg/m^2$, and especially 750 $mg/m^2$ to 1000 $mg/m^2$, for example in an amount of about 750 $mg/m^2$, about 1000 $mg/m^2$, about 900 $mg/m^2$, about 1000 $mg/m^2$, about 1150 $mg/m^2$ or about 1600 $mg/m^2$, within a time period of 2 to 4 weeks and preferably within a time period of about three weeks or about four weeks, more preferably three weeks, which time periods are preferably to be regarded as one cycle. Even more preferably, the amount of cetuximab to be administered to the patient is divided into three or four portions that are administered to the patient on three or four different days, preferably selected from one day within one week for three or four consecutive weeks and more preferably on each day 1 of three or four consecutive weeks, preferably beginning with day 1 within the first week of one cycle with respect to the cetuximab. Especially preferably, the amount of cetuximab to be administered to the patient is divided into three or four portions comprising or consisting of 200 to 500 $mg/m^2$ that are administered to the patient on three or four different days, preferably selected from one day within one week for three or four consecutive weeks and more preferably on each day 1 of three or four consecutive weeks, preferably beginning with day 1 within the first week of one cycle with respect to the cetuximab. Especially preferably in this regimen, the cetuximab is administered to the patient in an amount of about 250 $mg/m^2$ or about 400 $mg/m^2$ per day on a day one during the first week of the three or four consecutive weeks consecutive, followed by an administration of about 250 $mg/m^2$ per day on a day during each of the consecutively following two or three further weeks of a cycle consisting of about three weeks (about 21 days) or consisting of about four weeks (about 28 days). Preferably the cycle starts with the first administration on day 1 of the first week.

Even more preferably, the cetuximab is administered to the patient in an amount of about 400 $mg/m^2$ per day on day 1 and in an amount of about 250 $mg/m^2$ per day on days 8 and 15 of a cycle consisting of about 21 days.

Alternatively, the cetuximab is administered to the patient in an amount of about 250 $mg/m^2$ per day on the days 1, 8 and 15.

Preferably, matuzumab is administered to the patient in an amount of 500 mg to 3000 mg, more preferably 800 to 2500 mg, within a time period of 2 to 4 weeks and preferably within a time period of about three weeks or about four weeks weeks, which time periods are preferably to be regarded as one cycle. More preferably, the amount of matuzumab administered to the patient is given in mg per square metre of the by the surface of the patient, i.e. in $mg/m^2$. Accordingly, more preferably the matuzumab is administered to the patient in an amount of 500 $mg/m^2$ to 2000 $mg/m^2$, more preferably 750 $mg/m^2$ to 1750 $mg/m^2$, and especially 800 $mg/m^2$ to 1600 $mg/m^2$, for example in an amount of about 600 $mg/m^2$, about 800 $mg/m^2$, about 1000 $mg/m^2$, about 1200 $mg/m^2$ or about 1600 $mg/m^2$, within a time period of 2 to 4 weeks and preferably within a time period of about three weeks or about four weeks, more preferably three weeks, which time periods are preferably to be regarded as one cycle. Even more preferably, the amount of matuzumab to be administered to the patient is either divided into two or three portions that are administered to the patient on two or three different days, preferably selected from one day within one week for two or three consecutive weeks and more preferably on each day 1 of two or three consecutive weeks, preferably beginning with day 1 within the first week of one cycle with respect to the matuzumab, or the whole amount to be administerd within a time period of about three weeks or about four weeks is administered on one day within one first week of said time period, preferablly on day 1 of said first week. Especially preferably, the amount of matuzumab to be administered to the patient is divided into two portions comprising or consisting of 600 to 1000 mg/m$^2$, for example about 800 mg/m$^2$, that are administered to the patient on two different days, preferably selected from one day within one week for two consecutive weeks (i.e. on one day within one first week and on one day within one second week) and more preferably on each day 1 two consecutive weeks, preferably beginning with day 1 within the first week of one cycle with respect to the matuzumab. Alternatively preferably the matuzumab is administered to the patient in an amount of about 1600 mg/m$^2$ per day on a day one during the first week of three or four consecutive weeks. Thus, a cycle with respect to matuzumab preferably consists of about three weeks (about 21 days) or about four weeks (about 28 days), more preferably about three weeks (about 21 days). Preferably, the cycle starts with the first administration on day 1 of the first week.

Even more preferably, the matuzumab is administered to the patient in an amount of about 800 mg/m$^2$ per day on days 1 and 8 of a cycle consisting of about 21 days.

Alternatively more preferably, the matuzumab is administered to the patient in an amount of of 1600 mg/m$^2$, per day on the day 1 of a cycle consisting of about 21 days.

Generally, cytostatic alkaloids, especially cytostatic alkaloids selected from the group consisting of vinorelbine, vincristine, paclitaxel and docetaxel, can can be administered to the patient as it is known in the art.

Preferably, vinorelbine is administered to the patient in an amount of 25 mg to 250 mg, more preferably 50 to 150 mg, within a time period of 2 to 4 weeks and preferably within a time period of about three weeks, which time periods are preferably to be regarded as one cycle. More preferably, the amount of vinorelbine administered to the patient is given in mg per square meter of the by the surface of the patient, i.e. in mg/m$^2$. Accordingly, more preferably the vinorelbine is administered to the patient in an amount of 20 mg/m$^2$ to 100 mg/m$^2$, more preferably 40 mg/m$^2$ to 60 mg/m$^2$, for example in an amount of about 25 mg/m$^2$, within a time period of 2 to 4 weeks and preferably within a time period of about three weeks, which time periods are preferably to be regarded as one cycle. Even more preferably, the amount of vinorelbine to be administered to the patient is divided into two about equal portions that are administered to the patient on two different days, preferably one day within one first week and one day within one second week, preferably day 1 of one first week and day 1 of one second week, e.g. on day 1 and day 8 of one cycle with respect to the vinorelbine. Especially preferably, the vinorelbine is administered to the patient in an amount of about 25 mg/m$^2$ per day on the drays 1 and 8 of a cycle consisting of about 21 days. Preferably, 2 to 12 cycles, more preferably 4 to 8 cycles and especially about 6 cycles are applied to the patient with respect to vinorelbine, preferably substantially without a pause. The whole procedure/regimen described above with respect to the vinorelbine can be repeated one or more times, preferably one to 12 times and especially 2 to 6 times, for example about 5 times, preferably with a pause in between each repetition of the procedure/regimen.

Preferably, docetaxel is administered to the patient in an amount of 50 mg to 500 mg, more preferably 100 to 250 mg, within a time period of 2 to 4 weeks and preferably within a time period of about three weeks, which time periods are preferably to be regarded as one cycle. More preferably, the amount of docetaxel administered to the patient is given in mg per square metre of the by the surface of the patient, i.e. in mg/m$^2$. Accordingly, more preferably the docetaxel is administered to the patient in an amount of 25 mg/m$^2$ to 150 mg/m$^2$, more preferably 50 mg/m$^2$ to 100 mg/m$^2$, for example in an amount of about 75 mg/m$^2$, within a time period of 2 to 4 weeks and preferably within a time period of about three weeks, which time periods are preferably to be regarded as one cycle. Even more preferably, the amount of docetaxel to be administered to the patient is administered on one day, preferably on day 1 within one first week, more preferably day 1 of one first week of one cycle with respect to the docetaxel. Especially preferably, the docetaxel is administered to the patient in an amount of about 75 mg/m$^2$ per day on day 1 of a cycle consisting of about 21 days. Preferably, 2 to 12 cycles, more preferably 4 to 8 cycles and especially about 6 cycles are applied to the patient with respect to docetaxel, preferably substantially without a pause. The whole procedure/regimen described above with respect to the docetaxel can be repeated one or more times, preferably one to 12 times and especially 2 to 6 times, for example about 5 times, preferably with a pause in between each repetition of the procedure/regimen.

Preferably, paclitaxel is administered to the patient in an amount of 100 mg to 1000 mg, more preferably 200 to 800 mg, within a time period of 2 to 4 weeks and preferably within a time period of about three weeks or about four weeks weeks, which time periods are preferably to be regarded as one cycle. More preferably, the amount of paclitaxel administered to the patient is given in mg per square metre of the by the surface of the patient, i.e. in mg/m$^2$. Accordingly, more preferably the paclitaxel is administered to the patient in an amount of 100 mg/m$^2$ to 500 mg/m$^2$, more preferably 120 mg/m$^2$ to 350 mg/m$^2$, for example in an amount of about 135 mg/m$^2$, about 150 mg/m$^2$, about 175 mg/m$^2$, about 250 mg/m$^2$, about 270 mg/m$^2$ or about 300 mg/m$^2$, within a time period of 2 to 4 weeks and preferably within a time period of about three weeks or about four weeks, which time periods are preferably to be regarded as one cycle. Even more preferably, the amount of paclitaxel to be administered to the patient is administered on one day, preferably on day 1 within one first week, more preferably day 1 of one first week of one cycle with respect to the paclitaxel.

Alternatively and also preferably, the amount of paclitaxel to be administered to the patient is divided into three about equal portions that are administered to the patient on three different days, preferably selected from one day within one week for three consecutive weeks and more preferably on each day 1 of three consecutive weeks, preferably beginning with day 1 within the first week of one cycle with respect to the paclitaxel. Especially preferably in this regimen, the paclitaxel is administered to the patient in an amount of 80 mg/m$^2$ to 100 mg/m$^2$ per day on the days 1 of three consecutive weeks of a cycle consisting of about three weeks (about 28 days), preferably starting the administration on day 1 of the first week of the cycle of about four weeks, and ending the cycle with the fourth week without an administration.

Especially preferably, the paclitaxel is administered to the patient in an amount of about 250 mg/m$^2$ per day on day 1 of a cycle consisting of about 21 days, in an amount of 135 mg/m$^2$ to 175 mg/m$^2$ per day on day 1 of a cycle consisting of about 21 days, or in an amount of 80 mg/m$^2$ to 100 mg/m$^2$ per day on day 1, day 8 and day 15 of a cycle consisting of about 28 days.

For example, the paclitaxel is administered to the patient in an amount of about 250 mg/m$^2$ per day on day 1 of a cycle consisting of about 21 days as an i.V. infusion over 16 to 26 h (hours) on the respective day, preferably over about 24 h, in an amount of 135 mg/m$^2$ to 175 mg/m$^2$ per day on day 1 of a cycle consisting of about 21 days as an i. V. infusion over 1 to 6 hours, preferably over about 3 h on the respective day, or in an amount of 80 mg/m$^2$ to 100 mg/m$^2$ per day on day 1, day 8 and day 15 of a cycle consisting of about 28 days as an i. V. infusion over 1 to 6 hours, preferably over about 3 h, on the respective days.

Preferably, 2 to 12 cycles, more preferably 4 to 8 cycles and especially about 6 cycles are applied to the patient with respect to paclitaxel, preferably substantially without a pause. The whole procedure/regimen described above with respect to the paclitaxel can be repeated one or more times, preferably one to 12 times and especially 2 to 6 times, for example about 5 times, preferably with a pause in between each repetition of the procedure/regimen.

Generally, cytostatic alkaloids, especially cytostatic alkaloids selected from the group consisting of podophyllotoxinderivatives, and especially the podophyllotoxinderivative etoposide, can can be administered to the patient as it is known in the art.

Preferably, etoposide is administered to the patient in an amount of 300 mg to 1000 mg, more preferably 500 to 900 mg, within a time period of 2 to 4 weeks and preferably within a time period of about three weeks, which time periods are preferably to be regarded as one cycle. More preferably, the amount of etoposide administered to the patient is given in mg per square metre of the by the surface of the patient, i.e. in mg/m$^2$. Accordingly, more preferably the etoposide is administered to the patient in an amount of 200 mg/m$^2$ to 600 mg/m$^2$, more preferably 250 mg/m$^2$ to 450 mg/m$^2$, for example in an amount of about 300 mg/m$^2$, within a time period of 2 to 4 weeks and preferably within a time period of about three weeks, which time periods are preferably to be regarded as one cycle. Even more preferably, the amount of etoposide to be administered to the patient is divided into three about equal portions that are administered to the patient on three different days, preferably three consecutive days and more preferably three consecutive days at the beginning of one cycle with respect to the etoposide. Especially preferably, the etoposide is administered to the patient in an amount of about 100 mg/m$^2$ per day on the days 1, 2 and 3 or on the days 3, 4 or 5 of a cycle consisting of about 21 days. Preferably, 2 to 12 cycles, more preferably 4 to 8 cycles and especially about 6 cycles are applied to the patient with respect to etoposide, preferably substantially without a pause. The whole procedure/regimen described above with respect to the etoposide can be repeated one or more times, preferably one to 12 times and especially 2 to 6 times, for example about 5 times, preferably with a pause in between each repetition of the procedure/regimen. If the etoposide is administered to the patient in an amount of about 100 mg/m$^2$ per day on the days 3, 4 or 5 of a cycle consisting of about 21 days, the beginning of the cycle with respect to the etoposide is preferably triggered by the administration, preferably the first administration of another chemotherapeutic agent according to the invention and especially preferably triggered by the administration of an alkylating chemotherapeutic agent and/or the administration of the specific integrin ligand as described herein.

Generally, antimetabolites, especially antimetabolites selected from the group consisting of gemcitabine and pemetrexed, can can be administered to the patient as it is known in the art.

Preferably, gemcitabine is administered to the patient in an amount of 800 mg to 8000 mg, more preferably 1200 to 6000 mg, within a time period of 2 to 4 weeks and preferably within a time period of about three weeks, which time periods are preferably to be regarded as one cycle. More preferably, the amount of gemcitabine administered to the patient is given in mg per square metre of the by the surface of the patient, i.e. in mg/m$^2$. Accordingly, more preferably the gemcitabine is administered to the patient in an amount of 1000 mg/m$^2$ to 5000 mg/m$^2$, more preferably 2000 mg/m$^2$ to 3000 mg/m$^2$, for example in an amount of about 2000 mg/m$^2$, within a time period of 2 to 4 weeks and preferably within a time period of about three weeks, which time periods are preferably to be regarded as one cycle. Even more preferably, the amount of gemcitabine to be administered to the patient is divided into two about equal portions that are administered to the patient on two different days, preferably one day within one first week and one day within one second week, preferably day 1 of one first week and day 1 of one second week, e.g. on day 1 and day 8 of one cycle with respect to the gemcitabine. Especially preferably, the gemcitabine is administered to the patient in an amount of about 1000 mg/m$^2$ per day on the days 1 and 8 of a cycle consisting of about 21 days. Preferably, 2 to 12 cycles, more preferably 4 to 8 cycles and especially about 6 cycles are applied to the patient with respect to gemcitabine, preferably substantially without a pause. The whole procedure/regimen described above with respect to the gemcitabine can be repeated one or more times, preferably one to 12 times and especially 2 to 6 times, for example about 5 times, preferably with a pause in between each repetition of the procedure/regimen.

Preferably, pemetrexed is administered to the patient in an amount of 500 mg to 2000 mg, more preferably 800 to 1500 mg, within a time period of 2 to 4 weeks and preferably within a time period of about three weeks, which time periods are preferably to be regarded as one cycle. More preferably, the amount of pemetrexed administered to the patient is given in mg per square metre of the by the surface of the patient, i.e. in mg/m$^2$. Accordingly, more preferably the pemetrexed is administered to the patient in an amount of 300 mg/m$^2$ to 700 mg/m$^2$, more preferably 400 mg/m$^2$ to 600 mg/m$^2$, for example in an amount of about 500 mg/m$^2$, within a time period of 2 to 4 weeks and preferably within a time period of about three weeks, which time periods are preferably to be regarded as one cycle. Even more preferably, the amount of pemetrexed to be administered to the patient is administered to the patient on one day within one first week, preferably day 1 of one first week, e.g. on day 1 of one cycle with respect to the pemetrexed. Especially preferably, the pemetrexed is administered to the patient in an amount of about 500 mg/m$^2$ per day on day 1 of a cycle consisting of about 21 days. Preferably, 2 to 12 cycles, more preferably 4 to 8 cycles and especially about 6 cycles are applied to the patient with respect to pemetrexed, preferably substantially without a pause. The whole procedure/regimen described above with respect to the pemetrexed can be repeated one or more times, preferably one to 12 times and especially 2 to 6 times, for example about 5 times, preferably with a pause in between each repetition of the procedure/regimen.

The use as described above and/or below, wherein

    i) the one or more alkylating chemotherapeutic agents (a) are selected from the group consisting of the platinum containing chemotherapeutic agents cisplatin, carboplatin and oxaliplatin,
    ii) the one or more further chemotherapeutic agents other than the at least one specific integrin ligand and the one or more alkylating chemotherapeutic agents (b) are selected from the group consisting of anti-EGFR biologicals cetuximab, panitumumab, zalutumumab, nimotuzumab and matuzumab and the vinca alkaloids vinorelbine and vincristine.

The use as described above and/or below, wherein

    i) the at least one specific integrin ligand is selected from the group consisting of cyclo-(Arg-Gly-Asp-DPhe-NMe-Val), the pharmaceutically acceptable solvates and/or salts thereof,
    ii) the one or more alkylating chemotherapeutic agents (a) are selected from the group consisting of the platinum containing chemotherapeutic agents cisplatin, carboplatin and oxaliplatin, and
    iii) the one or more further chemotherapeutic agents other than the at least one specific integrin ligand and the one or more alkylating chemotherapeutic agents (b) comprise:

        α) one or more anti-EGFR biologicals, selected from the group consisting of cetuximab, panitumumab, zalutumumab, nimotuzumab and matuzumab, and optionally
        β) one or more compounds, selected from the group consisting of the cytostatic alkaloids vinorelbine and vincristine.

The specific integrin ligand, comprising cyclo-(Arg-Gly-Asp-DPhe-NMe-Val), the pharmaceutically acceptable solvates and/or salts thereof, for use as described herein, wherein the at least one specific integrin ligand selected from the group consisting of cyclo-(Arg-Gly-Asp-DPhe-NMe-Val), the pharmaceutically acceptable solvates and/or salts thereof is administered to a patient in an amount of 1500 mg to 5000 mg per week

The use as described above and/or below, wherein the at least one specific integrin ligand selected from the group consisting of cyclo-(Arg-Gly-Asp-DPhe-NMe-Val), the pharmaceutically acceptable solvates and/or salts thereof is administered to a patient in an amount of 400 mg to 6000 mg per week.

The use as described above and/or below, wherein the at least one specific integrin ligand selected from the group consisting of cyclo-(Arg-Gly-Asp-DPhe-NMe-Val), the pharmaceutically acceptable solvates and/or salts thereof is administered to a patient in an amount of 1500 mg to 5000 mg per week.

The use as described above and/or below, wherein the at least one specific integrin ligand selected from the group consisting of cyclo-(Arg-Gly-Asp-DPhe-NMe-Val), the pharmaceutically acceptable solvates and/or salts thereof is administered to a patient in a once weekly to three times weekly administration scheme consisting of about 500 mg or about 2000 mg per administration.

More preferably, cyclo-(Arg-Gly-Asp-DPhe-NMe-Val), the pharmaceutically acceptable solvates and/or salts thereof is administered to the patient as described in one or more of the paragraphs numbered [I] to [XI].

The use as described above and/or below, wherein

    ii) the one or more alkylating chemotherapeutic agents (a) selected from the group consisting of the platinum containing chemotherapeutic agents cisplatin, carboplatin and oxaliplatin are administered to the patient in an amount of 100 to 1000 mg in one or more portions within a time period of 2 to 4 weeks, and

iiii) the one or more further chemotherapeutic agents other than the at least one specific integrin ligand and the one or more alkylating chemotherapeutic agents (b) comprise:

α) one or more anti-EGFR biologicals, selected from the group consisting of cetuximab, panitumumab, zalutumumab, nimotuzumab and matuzumab, administered to the patient in an amount of 200 to 2000 mg in one or more portions within a time period of 2 to 4 weeks, and optionally

β) one or more compounds, selected from the group consisting of the cytostatic alkaloids vinorelbine and vincristine, the group consisting of paclitaxel and docetaxel, and/or the group consisting of the antimetabolites gemcitabine and pemetrexed, administered to the patient in an amount of 25 to 6000 mg in one or more portions within a time period of 2 to 4 weeks.

Thus, a preferred subject of the instant invention is a treatment, comprising one or more cycles, preferably 2 to 12 cycles, more preferably about 6 cycles, each cycle consisting of about 21 days or about 28 days, preferably about 21 days,
wherein in each cycle:

a) cyclo-(Arg-Gly-Asp-DPhe-NMe-Val), the pharmaceutically acceptable solvates and/or salts thereof, preferably cyclo-(Arg-Gly-Asp-DPhe-NMe-Val), is administered to the patient
a1) in an amount of about 2000 mg per day on one day within each week of the cycle, preferably on day 1 of each week of the cycle, or a2) in an amount of 2000 mg per day on two different days within each week of the cycle, preferably on the days 1 and 4 or 1 and 5 within each week,
b) cisplatin, the pharmaceutically acceptable dervatives, solvates and/or salts thereof, preferably cisplatin, is administered to the patient
b1) in an amount of 60 to 120 mg/m$^2$, more preferably in an amount of about 80 mg/m$^2$ or about 100 mg/m$^2$, per day on one day within the first week of the cycle, preferably on day 1 of the first week of the cycle,
b2) preferably, no more cisplatin is administered to the patient during the subsequent weeks of said cycle;
c) cetuximab, the pharmaceutically acceptable dervatives, solvates and/or salts thereof, preferably cetuximab is administered to the patient
c1) in an amount of about 200 to 600 mg/m$^2$, preferably about 250 mg/m$^2$ or about 400 mg/m$^2$, more preferably about 400 mg/m$^2$, per day on one day within the first week, preferably on day 1 of the first week,
a2) in an amount of 200 to 400 mg/m$^2$, preferably about 250 mg/m$^2$, per day on one day during each week of the subsequent weeks of said cycle, preferably on day 1 of each week and more preferably on days 8 and 15 of said cycle; and optionally
e) Vinorelbine, the pharmaceutically acceptable dervatives, solvates and/or salts thereof, preferably vinorelbine, is administered to the patient
e1) in an amount of 10 to 50 mg/m$^2$, preferably about 25 mg/m$^2$, per day on one day within the first week, preferably day 1 of the first week, and on one day within the second week, preferably on day 1 of the second week,
e2) preferably, no more vinorelbine is administered to the patient during the subsequent weeks of said cycle.

Thus, another preferred subject of the instant invention is a treatment, comprising one or more cycles, preferably 2 to 12 cycles, more preferably about 6 cycles, each cycle consisting of about 21 days or about 28 days, preferably about 21 days,
wherein in each cycle:

a) cyclo-(Arg-Gly-Asp-DPhe-NMe-Val), the pharmaceutically acceptable solvates and/or salts thereof, preferably cyclo-(Arg-Gly-Asp-DPhe-NMe-Val), is administered to the patient
a1) in an amount of about 2000 mg per day on one day within each week of the cycle, preferably on day 1 of each week of the cycle, or a2) in an amount of 2000 mg per day on two different days within each week of the cycle, preferably on the days 1 and 4 or 1 and 5 within each week,
b) carboplatin, the pharmaceutically acceptable dervatives, solvates and/or salts thereof, preferably carboplatin, is administered to the patient
b1) in an amount as described herein, preferably as described herein as AUC 5-7 and more preferably described herein as AUC 6, per day on one day within the first week of the cycle, preferably on day 1 of the first week of the cycle,
b2) preferably, no more carboplatin is administered to the patient during the subsequent weeks of said cycle;
c) cetuximab, the pharmaceutically acceptable dervatives, solvates and/or salts thereof, preferably cetuximab is administered to the patient
c1) in an amount of about 200 to 600 mg/m$^2$, preferably about 250 mg/m$^2$ or about 400 mg/m$^2$, more preferably

about 400 mg/m$^2$, per day on one day within the first week, preferably on day 1 of the first week,

a2) in an amount of 200 to 400 mg/m$^2$, preferably about 250 mg/m$^2$, per day on one day during each week of the subsequent weeks of said cycle, preferably on day 1 of each week and more preferably on days 8 and 15 of said cycle; and optionally

e) Vinorelbine, the pharmaceutically acceptable dervatives, solvates and/or salts thereof, preferably vinorelbine, is administered to the patient

e1) in an amount of 10 to 50 mg/m$^2$, preferably about 25 mg/m$^2$, per day on one day within the first week, preferably day 1 of the first week, and on one day within the second week, preferably on day 1 of the second week,

e2) preferably, no more vinorelbine is administered to the patient during the subsequent weeks of said cycle.

Thus, an especially preferred subject of the instant invention is a treatment, comprising one or more cycles, preferably 2 to 12 cycles, more preferably about 6 cycles, each cycle consisting of about 21 days,
wherein in each cycle:

a) cyclo-(Arg-Gly-Asp-DPhe-NMe-Val), the pharmaceutically acceptable solvates and/or salts thereof, preferably cyclo-(Arg-Gly-Asp-DPhe-NMe-Val), is administered to the patient

a1) in an amount of about 2000 mg per day on one day within each week of the cycle, preferably on day 1 of each week of the cycle, or

a2) in an amount of 2000 mg per day on two different days within each week of the cycle, preferably on the days 1 and 4 or 1 and 5 within each week,

b) cisplatin, the pharmaceutically acceptable dervatives, solvates and/or salts thereof, preferably cisplatin, is administered to the patient

b1) in an amount of 60 to 120 mg/m$^2$, more preferably in an amount of about 80 mg/m$^2$, per day on one day within the first week of the cycle, preferably on day 1 of the first week of the cycle,

b2) preferably, no more cisplatin is administered to the patient during the subsequent weeks of said cycle;

c) cetuximab, the pharmaceutically acceptable dervatives, solvates and/or salts thereof, preferably cetuximab is administered to the patient

c1) in an amount of about 200 to 600 mg/m$^2$, preferably about 250 mg/m$^2$ or about 400 mg/m$^2$, more preferably about 400 mg/m$^2$, per day on one day within the first week, preferably on day 1 of the first week,

a2) in an amount of 200 to 400 mg/m$^2$, preferably about 250 mg/m$^2$, per day on one day during each week of the subsequent weeks of said cycle, preferably on day 1 of each week and more preferably on days 8 and 15 of said cycle; and optionally

e) Vinorelbine, the pharmaceutically acceptable dervatives, solvates and/or salts thereof, preferably vinorelbine, is administered to the patient

e1) in an amount of 10 to 50 mg/m$^2$, preferably about 25 mg/m$^2$, per day on one day within the first week, preferably day 1 of the first week, and on one day within the second week, preferably on day 1 of the second week,

e2) preferably, no more vinorelbine is administered to the patient during the subsequent weeks of said cycle.

Thus, another especially preferred subject of the instant invention is a treatment, comprising one or more cycles, preferably 2 to 12 cycles, more preferably about 6 cycles, each cycle consisting of about 21 days, wherein in each cycle:

a) cyclo-(Arg-Gly-Asp-DPhe-NMe-Val), the pharmaceutically acceptable solvates and/or salts thereof, preferably cyclo-(Arg-Gly-Asp-DPhe-NMe-Val), is administered to the patient

a1) in an amount of about 2000 mg per day on one day within each week of the cycle, preferably on day 1 of each week of the cycle, or

a2) in an amount of 2000 mg per day on two different days within each week of the cycle, preferably on the days 1 and 4 or 1 and 5 within each week,

b) carboplatin, the pharmaceutically acceptable dervatives, solvates and/or salts thereof, preferably carboplatin, is administered to the patient

b1) in an amount as described herein, preferably as described herein as AUC 5-7 and more preferably described herein as AUC 6, per day on one day within the first week of the cycle, preferably on day 1 of the first week of the cycle,

b2) preferably, no more carboplatin is administered to the patient during the subsequent weeks of said cycle;

c) cetuximab, the pharmaceutically acceptable dervatives, solvates and/or salts thereof, preferably cetuximab is administered to the patient

c1) in an amount of about 200 to 600 mg/m$^2$, preferably about 250 mg/m$^2$ or about 400 mg/m$^2$, more preferably about 400 mg/m$^2$, per day on one day within the first week, preferably on day 1 of the first week,

a2) in an amount of 200 to 400 mg/m$^2$, preferably about 250 mg/m$^2$, per day on one day during each week of the subsequent weeks of said cycle, preferably on day 1 of each week and more preferably on days 8 and 15 of said cycle;

and optionally

e) Vinorelbine, the pharmaceutically acceptable dervatives, solvates and/or salts thereof, preferably vinorelbine, is administered to the patient

e1) in an amount of 10 to 50 mg/m$^2$, preferably about 25 mg/m$^2$, per day on one day within the first week, preferably day 1 of the first week, and on one day within the second week, preferably on day 1 of the second week,

e2) preferably, no more vinorelbine is administered to the patient during the subsequent weeks of said cycle.

Thus, an even more preferred subject of the instant invention is a treatment, comprising one or more cycles, preferably 2 to 12 cycles, more preferably about 6 cycles, each cycle consisting of about 21 days, wherein in each cycle:

a) cyclo-(Arg-Gly-Asp-DPhe-NMe-Val), the pharmaceutically acceptable solvates and/or salts thereof, preferably cyclo-(Arg-Gly-Asp-DPhe-NMe-Val), is administered to the patient

a1) in an amount of about 2000 mg per day on one day within each week of the cycle, preferably on days 1, 8 and 15 of the cycle,

b) one platinum platinum containing chemotherapeutic agent,

either

b') cisplatin, the pharmaceutically acceptable dervatives, solvates and/or salts thereof, preferably cisplatin, is administered to the patient b1) in an amount of 60 to 120 mg/m$^2$, more preferably in an amount of about 100 mg/m$^2$, per day on one day within the first week of the cycle, preferably on day 1 of the first week of the cycle,

b'2) preferably, no more cisplatin is administered to the patient during the subsequent weeks of said cycle;

or

b") carboplatin, the pharmaceutically acceptable dervatives, solvates and/or salts thereof, preferably carboplatin, is administered to the patient b"1) in an amount as described herein, preferably as described herein as AUC 5-7 and more preferably described herein as AUC 6, per day on one day within the first week of the cycle, preferably on day 1 of the first week of the cycle,

b"2) preferably, no more carboplatin is administered to the patient during the subsequent weeks of said cycle;

c) cetuximab, the pharmaceutically acceptable dervatives, solvates and/or salts thereof, preferably cetuximab is administered to the patient

c1) in an amount of about 200 to 600 mg/m$^2$, preferably about 250 mg/m$^2$ or about 400 mg/m$^2$, more preferably about 400 mg/m$^2$, per day on one day within the first week, preferably on day 1 of the first week,

a2) in an amount of 200 to 400 mg/m$^2$, preferably about 250 mg/m$^2$, per day on one day during each week of the subsequent weeks of said cycle, preferably on day 1 of each week and more preferably on days 8 and 15 of said cycle; and optionally

e) Vinorelbine, the pharmaceutically acceptable dervatives, solvates and/or salts thereof, preferably vinorelbine, is administered to the patient

e1) in an amount of 10 to 50 mg/m$^2$, preferably about 25 mg/m$^2$, per day on one day within the first week, preferably day 1 of the first week, and on one day within the second week, preferably on day 1 of the second week,

e2) preferably, no more vinorelbine is administered to the patient during the subsequent weeks of said cycle.

In the treatments described above, the one or more cycles preferably mean one or more cycles substantially without a pause.

In the treatments described above, the administration of the cisplatin and/or the carboplatin can be substituted by the administration of oxaliplatin, preferably the administration of oxaliplatin as described herein.

Another especially preferred subject of the instant invention relates to the use of cyclo-(Arg-Gly-Asp-DPhe-NMe-Val), the pharmaceutically acceptable solvates and/or salts thereof, preferably cyclo-(Arg-Gly-Asp-DPhe-NMe-Val), for the manufacture of a medicament to be used in the methods of treatment described above.

A further subject of the instant invention is:

Generally, the at least one specific integrin ligand as described herein, the one or more alkylating chemotherapeutic agents (a) as described herein, and/or the one or more further chemotherapeutic agents other than the at least one specific integrin ligand and the one or more alkylating chemotherapeutic agents (b) as described herein can be administered in an amount and/or a regimen as it is known in the art for the respective compound.

Preferably, the at least one specific integrin ligand, the one or more alkylating chemotherapeutic agents (a), and/or the one or more further chemotherapeutic agents other than the at least one specific integrin ligand and the one or more alkylating chemotherapeutic agents (b) are administered in an amount and/or a regimen as it is described above and/or below for the respective compound.

Alkylating chemotherapeutic agents in this respect are selected from:

the Platin derivatives Cisplatin, Carboplatin and Oxaliplatin;
and pharmaceutically acceptable dervatives, salts and/or solvates thereof.

EGFR inhibitors in this respect are selected from the group consisting of:

the anti-EGFR biologicals cetuximab, panitumumab, zalutumumab, nimotuzumab and matuzumab; and
the anti-EGFR chemically derived compounds gefitinib, erlotinib and lapatinib;

and pharmaceutically acceptable dervatives, salts and/or solvates thereof.

Cytostatic alkaloids in this respect are selected from:

the podophyllotoxinderivatives Etoposide and Teniposide;
the vinca alkaloids Vinblastine, Vincristine, Vindesine and Vinorelbine;
the taxanes Docetaxel and Paclitaxel; and
Camptothecin derivatives, more preferably from the Camptothecin derivatives Irinotecane and Topotecane;

and pharmaceutically acceptable dervatives, salts and/or solvates thereof.

Antimetabolites in this respect are selected from:

the antifolates Methotrexate, Raltitrexed, and Pemetrexed; and
the pyrimidine antagonists 5-Fluorouracil, Capecitabine, Cytosinarabinoside and Difluorodesoxycytidine; and pharmaceutically acceptable dervatives,
salts and/or solvates thereof.

The use as described above and/or below
wherein

i) the platinum containing chemotherapeutic agent is selected from the group consisting of cisplatin, carboplatin and oxaliplatin,
ii) the antimetabolite is selected from the group consisting of antifolates and pyrimidine antagonists, and
iii) the cytostatic alkaloid is selected from the group consisting of vinca alkaloids and taxanes, and
iv) the EGFR inhibitor is selected from the group consisting of anti-EGFR biologicals and chemically derived compounds.

Antifolates in this respect are selected from Methotrexate, Raltitrexed, and Pemetrexed;
and pharmaceutically acceptable dervatives, salts and/or solvates thereof.
Pyrimidine antagonists in this respect are selected from 5-Fluorouracil, Capecitabine, Cytosinarabinoside and Difluorodesoxycytidine, more preferably 5-Fluorouracil;
and pharmaceutically acceptable dervatives, salts and/or solvates thereof.
Vinca alkaloids in this respect are selected from Vinblastine, Vincristine, Vindesine and Vinorelbine, more preferably Vinorelbine;
and pharmaceutically acceptable dervatives, salts and/or solvates thereof.
Taxanes in this respect are selected from Docetaxel and Paclitaxel, more preferably Paclitaxel;
and pharmaceutically acceptable dervatives, salts and/or solvates thereof.
Anti-EGFR biologicalsin this respect are selected from cetuximab, panitumumab, zalutumumab, nimotuzumab and matuzumab, more preferably from cetuximab and matuzumab;
and pharmaceutically acceptable dervatives, salts and/or solvates thereof.
Anti-EGFR chemically derived compounds in this respect are selected from gefitinib, erlotinib and lapatinib;
and pharmaceutically acceptable dervatives, salts and/or solvates thereof.
The use as described above and/or below, wherein
the EGFR inhibitor is selected from the group consisting of cetuximab, panitumumab, zalutumumab, nimotuzumab and matuzumab and/or the group consisting of gefitinib, erlotinib and lapatinib, the cytostatic alkaloid is selected from the group consisting of vinorelbine and vincristine and/or the group consisting of paclitaxel and docetaxel, and the antimetabolite is selected from the group consisting of 5-fluorouracil and pemetrexed.
Preferably,

i) the platinum containing chemotherapeutic agent is selected from the group consisting of cisplatin, carboplatin and oxaliplatin,

ii) the antimetabolite is selected from the group consisting of the antifolates Methotrexate, Raltitrexed and Pemetrexed and pharmaceutically acceptable salts and/or solvates thereof, and the pyrimidine antagonists 5-Fluorouracil, Capecitabine, Cytosinarabinoside and Difluorodesoxycytidine and pharmaceutically acceptable salts and/or solvates thereof, and

iii) the cytostatic alkaloid is selected from the group consisting of the vinca alkaloids vinblastine, Vincristine, Vindesine and Vinorelbine and pharmaceutically acceptable salts and/or solvates thereof, and the taxanes Docetaxel and Paclitaxel and pharmaceutically acceptable salts and/or solvates thereof, and

iv) the EGFR inhibitor is selected from the group consisting of the anti-EGFR biologicals cetuximab, panitumumab, zalutumumab, nimotuzumab and matuzumab and pharmaceutically acceptable salts and/or solvates thereof and the chemically derived compounds gefitinib, erlotinib and lapatinib and pharmaceutically acceptable salts and/or solvates thereof.

Preferably, the cisplatin, carboplatin and/or oxaliplatin are administered to the patient as it is known in the art and even more preferably as it is described above and/or below. More preferably, the cisplatin, carboplatin and/or oxaliplatin is administered to the patient as it is described in the paragraphs following the paragraph numbered [14] and preferably before the paragraph numbered [16] and/or as it is described in the paragraphs following the paragraph numbered [23] and preferably before the paragraph numbered [24].

Generally, the cetuximab, panitumumab, zalutumumab, nimotuzumab matuzumab, gefitinib, erlotinib, lapatinib, vinorelbine, vincristine, paclitaxel, docetaxel, 5-fluorouracil and pemetrexed can be administered to the patient as it is known in the art and/or as described herein.

Preferably, cetuximab is administered to the patient in an amount of 500 mg to 3000 mg, more preferably 800 to 2500 mg, within a time period of 2 to 4 weeks and preferably within a time period of about three weeks or about four weeks weeks, which time periods are preferably to be regarded as one cycle. More preferably, the amount of cetuximab administered to the patient is given in mg per square metre of the by the surface of the patient, i.e. in $mg/m^2$. Accordingly, more preferably the cetuximab is administered to the patient in an amount of 500 $mg/m^2$ to 2000 $mg/m^2$, more preferably 750 $mg/m^2$ to 1500 $mg/m^2$, and especially 750 $mg/m^2$ to 1000 $mg/m^2$, for example in an amount of about 750 $mg/m^2$, about 1000 $mg/m^2$, about 900 $mg/m^2$, about 1000 $mg/m^2$, about 1150 $mg/m^2$ or about 1600 $mg/m^2$, within a time period of 2 to 4 weeks and preferably within a time period of about three weeks or about four weeks, more preferably three weeks, which time periods are preferably to be regarded as one cycle. Even more preferably, the amount of cetuximab to be administered to the patient is divided into three or four portions that are administered to the patient on three or four different days, preferably selected from one day within one week for three or four consecutive weeks and more preferably on each day 1 of three or four consecutive weeks, preferably beginning with day 1 within the first week of one cycle with respect to the cetuximab. Especially preferably, the amount of cetuximab to be administered to the patient is divided into three or four portions comprising or consisting of 200 to 500 $mg/m^2$ that are administered to the patient on three or four different days, preferably selected from one day within one week for three or four consecutive weeks and more preferably on each day 1 of three or four consecutive weeks, preferably beginning with day 1 within the first week of one cycle with respect to the cetuximab. Especially preferably in this regimen, the cetuximab is administered to the patient in an amount of about 250 $mg/m^2$ or about 400 $mg/m^2$ per day on a day one during the first week of the three or four consecutive weeks consecutive, followed by an administration of about 250 $mg/m^2$ per day on a day during each of the consecutively following two or three further weeks of a cycle consisting of about three weeks (about 21 days) or consisting of about four weeks (about 28 days). Preferably the cycle starts with the first administration on day 1 of the first week.

Even more preferably, the cetuximab is administered to the patient in an amount of about 400 $mg/m^2$ per day on day 1 and in an amount of about 250 $mg/m^2$ per day on days 8 and 15 of a cycle consisting of about 21 days.

Alternatively, the cetuximab is administered to the patient in an amount of about 250 $mg/m^2$ per day on the days 1, 8 and 15.

Preferably, matuzumab is administered to the patient in an amount of 500 mg to 3000 mg, more preferably 800 to 2500 mg, within a time period of 2 to 4 weeks and preferably within a time period of about three weeks or about four weeks weeks, which time periods are preferably to be regarded as one cycle. More preferably, the amount of matuzumab administered to the patient is given in mg per square metre of the by the surface of the patient, i.e. in $mg/m^2$. Accordingly, more preferably the matuzumab is administered to the patient in an amount of 500 $mg/m^2$ to 2000 $mg/m^2$, more preferably 750 $mg/m^2$ to 1750 $mg/m^2$, and especially 800 $mg/m^2$ to 1600 $mg/m^2$, for example in an amount of about 600 $mg/m^2$, about 800 $mg/m^2$, about 1000 $mg/m^2$, about 1200 $mg/m^2$ or about 1600 $mg/m^2$, within a time period of 2 to 4 weeks and preferably within a time period of about three weeks or about four weeks, more preferably three weeks, which time periods are preferably to be regarded as one cycle. Even more preferably, the amount of matuzumab to be administered to the patient is either divided into two or three portions that are administered to the patient on two or three different days, preferably selected from one day within one week for two or three consecutive weeks and more preferably on each day 1 of two or three consecutive weeks, preferably beginning with day 1 within the first week of one cycle with respect to the matuzumab, or the whole amount to be administerd within a time period of about three weeks or about four weeks is administered on one day within one first week of said time period, preferably on day 1 of said first week. Especially preferably, the amount of matuzumab to be administered to the patient is divided into two portions comprising or consisting of 600 to 1000 mg/m$^2$, for example about 800 mg/m$^2$, that are administered to the patient on two different days, preferably selected from one day within one week for two consecutive weeks (i.e. on one day within one first week and on one day within one second week) and more preferably on each day 1 two consecutive weeks, preferably beginning with day 1 within the first week of one cycle with respect to the matuzumab. Alternatively preferably the matuzumab is administered to the patient in an amount of about 1600 mg/m$^2$ per day on a day one during the first week of three or four consecutive weeks. Thus, a cycle with respect to matuzumab preferably consists of about three weeks (about 21 days) or about four weeks (about 28 days), more preferably about three weeks (about 21 days). Preferably, the cycle starts with the first administration on day 1 of the first week.

Even more preferably, the matuzumab is administered to the patient in an amount of about 800 mg/m$^2$ per day on days 1 and 8 of a cycle consisting of about 21 days.

Alternatively more preferably, the matuzumab is administered to the patient in an amount of of 1600 mg/m$^2$, per day on the day 1 of a cycle consisting of about 21 days.

Preferably, paclitaxel is administered to the patient in an amount of 100 mg to 1000 mg, more preferably 200 to 800 mg, within a time period of 2 to 4 weeks and preferably within a time period of about three weeks or about four weeks weeks, which time periods are preferably to be regarded as one cycle. More preferably, the amount of paclitaxel administered to the patient is given in mg per square metre of the by the surface of the patient, i.e. in mg/m$^2$. Accordingly, more preferably the paclitaxel is administered to the patient in an amount of 100 mg/m$^2$ to 500 mg/m$^2$, more preferably 120 mg/m$^2$ to 350 mg/m$^2$, for example in an amount of about 135 mg/m$^2$, about 150 mg/m$^2$, about 175 mg/m$^2$, about 250 mg/m$^2$, about 270 mg/m$^2$ or about 300 mg/m$^2$, within a time period of 2 to 4 weeks and preferably within a time period of about three weeks or about four weeks, which time periods are preferably to be regarded as one cycle. Even more preferably, the amount of paclitaxel to be administered to the patient is administered on one day, preferably on day 1 within one first week, more preferably day 1 of one first week of one cycle with respect to the paclitaxel.

Alternatively and also preferably, the amount of paclitaxel to be administered to the patient is divided into three about equal portions that are administered to the patient on three different days, preferably selected from one day within one week for three consecutive weeks and more preferably on each day 1 of three consecutive weeks, preferably beginning with day 1 within the first week of one cycle with respect to the paclitaxel. Especially preferably in this regimen, the paclitaxel is administered to the patient in an amount of 80 mg/m$^2$ to 100 mg/m$^2$ per day on the days 1 of three consecutive weeks of a cycle consisting of about three weeks (about 28 days), preferably starting the administration on day 1 of the first week of the cycle of about four weeks, and ending the cycle with the fourth week without an administration.

Especially preferably, the paclitaxel is administered to the patient in an amount of about 250 mg/m$^2$ per day on day 1 of a cycle consisting of about 21 days, in an amount of 135 mg/m$^2$ to 175 mg/m$^2$ per day on day 1 of a cycle consisting of about 21 days, or in an amount of 80 mg/m$^2$ to 100 mg/m$^2$ per day on day 1, day 8 and day 15 of a cycle consisting of about 28 days.

For example, the paclitaxel is administered to the patient in an amount of about 250 mg/m$^2$ per day on day 1 of a cycle consisting of about 21 days as an i.V. infusion over 16 to 26 h (hours) on the respective day, preferably over about 24 h, in an amount of 135 mg/m$^2$ to 175 mg/m$^2$ per day on day 1 of a cycle consisting of about 21 days as an i. V. infusion over 1 to 6 hours, preferably over about 3 h on the respective day, or in an amount of 80 mg/m$^2$ to 100 mg/m$^2$ per day on day 1, day 8 and day 15 of a cycle consisting of about 28 days as an i. V. infusion over 1 to 6 hours, preferably over about 3 h, on the respective days.

Preferably, 2 to 12 cycles, more preferably 4 to 8 cycles and especially about 6 cycles are applied to the patient with respect to paclitaxel, preferably substantially without a pause. The whole procedure/regimen described above with respect to the paclitaxel can be repeated one or more times, preferably one to 12 times and especially 2 to 6 times, for example about 5 times, preferably with a pause in between each repetition of the procedure/regimen.

Generally, the 5-fluorouracil can be administered to the patient as it is known in the art.

Preferably, 5-fluorouracil is administered to the patient in an amount of 2000 mg to 15000 mg, more preferably 3000 to 10000 mg, within a time period of 2 to 4 weeks and preferably within a time period of about three weeks, which time periods are preferably to be regarded as one cycle. More preferably, the amount of 5-fluorouracil administered to the patient is given in mg per square metre of the by the surface of the patient, i.e. in mg/m$^2$. Accordingly, more preferably the 5-fluorouracil is administered to the patient in an amount of 1500 mg/m$^2$ to 8000 mg/m$^2$, more preferably 2500 mg/m$^2$ to 7500 mg/m$^2$, for example in an amount of about 5000 mg/m$^2$, within a time period of 2 to 4 weeks and preferably within a time period of about three weeks, which time periods are preferably to be regarded as one cycle. Even more preferably, the amount of 5-fluorouracil to be administered to the patient is divided into five about equal portions that are administered to the patient on five different days, preferably five consecutive days and more preferably five consecutive days at the beginning of one cycle with respect to the 5-fluorouracil. Especially preferably, the 5-fluorouracil is administered to the patient in an amount of about 1000 mg/m$^2$ per day on the days 1, 2, 3, 4 and 5 of a cycle consisting of about 21 days. Preferably, 2 to 12 cycles, more preferably 4 to 8 cycles and especially about 6 cycles are applied to the patient with respect to 5-fluorouracil, preferably substantially without a pause. The whole procedure/regimen described above with respect to the 5-fluorouracil can be repeated one or more times, preferably one to 12 times and especially 2 to 6 times, for example about 5 times, preferably with a pause in between each repetition of the procedure/regimen.

Preferably, vinorelbine is administered to the patient in an amount of 25 mg to 250 mg, more preferably 50 to 150 mg, within a time period of 2 to 4 weeks and preferably within a time period of about three weeks, which time periods are preferably to be regarded as one cycle. More preferably, the amount of vinorelbine administered to the patient is given in mg per square meter of the by the surface of the patient, i.e. in mg/m$^2$, Accordingly, more preferably the vinorelbine is administered to the patient in an amount of 20 mg/m$^2$ to 100 mg/m$^2$, more preferably 40 mg/m$^2$ to 60 mg/m$^2$, for example in an amount of about 25 mg/m$^2$, within a time period of 2 to 4 weeks and preferably within a time period of about three weeks, which time periods are preferably to be regarded as one cycle. Even more preferably, the amount of vinorelbine to be administered to the patient is divided into two about equal portions that are administered to the patient on two different days, preferably one day within one first week and one day within one second week, preferably day 1 of one first week and day 1 of one second week, e.g. on day 1 and day 8 of one cycle with respect to the vinorelbine. Especially preferably, the vinorelbine is administered to the patient in an amount of about 25 mg/m$^2$ per day on the days 1 and 8 of a cycle consisting of about 21 days. Preferably, 2 to 12 cycles, more preferably 4 to 8 cycles and especially about 6 cycles are applied to the patient with respect to vinorelbine, preferably substantially without a pause. The whole procedure/regimen described above with respect to the vinorelbine can be repeated one or more times, preferably one to 12 times and especially 2 to 6 times, for example about 5 times, preferably with a pause in between each repetition of the procedure/regimen.

The use as described above and/or below, wherein

 i) the one or more alkylating chemotherapeutic agents (a) are selected from the group consisting of the platinum containing chemotherapeutic agents cisplatin, carboplatin and oxaliplatin,
 ii) the one or more further chemotherapeutic agents other than the at least one specific integrin ligand and the one or more alkylating chemotherapeutic agents (b) are selected from the group consisting of anti-EGFR biologicals cetuximab, panitumumab, zalutumumab, nimotuzumab and matuzumab, the antimetabolites 5-fluorouracil and pemetrexed, and the taxanes docetaxel and paclitaxel.

The use as described above and/or below, wherein

 i) the at least one specific integrin ligand is selected from the group consisting of cyclo-(Arg-Gly-Asp-DPhe-NMe-Val), the pharmaceutically acceptable solvates and/or salts thereof,
 ii) the one or more alkylating chemotherapeutic agents (a) are selected from the group consisting of the platinum containing chemotherapeutic agents cisplatin, carboplatin and oxaliplatin, and
 iii) the one or more further chemotherapeutic agents other than the at least one specific integrin ligand and the one or more alkylating chemotherapeutic agents (b) comprise:

 α) one or more anti-EGFR biologicals, selected from the group consisting of cetuximab, panitumumab, zalutumumab, nimotuzumab and matuzumab, and optionally
 β) one or more compounds, selected from the group consisting of the antimetabolites 5-fluorouracil and pemetrexed, and/or the group consisting of the taxanes docetaxel and paclitaxel.

The use as described above and/or below, wherein
the at least one specific integrin ligand selected from the group consisting of cyclo-(Arg-Gly-Asp-DPhe-NMe-Val), the pharmaceutically acceptable solvates and/or salts thereof is administered to a patient in an amount of 400 mg to 6000 mg per week.

The use as described above and/or below wherein
the at least one specific integrin ligand selected from the group consisting of cyclo-(Arg-Gly-Asp-DPhe-NMe-Val), the pharmaceutically acceptable solvates and/or salts thereof is administered to a patient in an amount of 1500 mg to 5000 mg per week.

Preferably, the specific integrin ligand, comprising cyclo-(Arg-Gly-Asp-DPhe-NMe-Val), the pharmaceutically acceptable solvates and/or salts thereof, for use as described herein is administered to a patient in an amount of 1500 mg to 5000 mg per week.

More preferably, cyclo-(Arg-Gly-Asp-DPhe-NMe-Val), the pharmaceutically acceptable solvates and/or salts thereof is administered to the patient as described in one or more of the paragraphs numbered [I] to [XI].

The use as described above and/or below, wherein

the at least one specific integrin ligand selected from the group consisting of cyclo-(Arg-Gly-Asp-DPhe-NMe-Val), the pharmaceutically acceptable solvates and/or salts thereof is administered to a patient in a once weekly to five times weekly administration scheme consisting of about 500 mg or in a once weekly to three times weekly administration scheme consisting ofabout 2000 mg per administration.

The use as described above and/or below, wherein

ii) the one or more alkylating chemotherapeutic agents (a) selected from the group consisting of the platinum containing chemotherapeutic agents cisplatin, carboplatin and oxaliplatin are administered to the patient in an amount of 100 to 1000 mg in one or more portions within a time period of 2 to 4 weeks, and

iiii) the one or more further chemotherapeutic agents other than the at least one specific integrin ligand and the one or more alkylating chemotherapeutic agents (b) comprise:

α) one or more anti-EGFR biologicals, selected from the group consisting of cetuximab, panitumumab, zalutumumab, nimotuzumab and matuzumab, administered to the patient in an amount of 200 to 2000 mg in one or more portions within a time period of 2 to 4 weeks, and optionally

β) one or more compounds, selected from the group consisting of the antimetabolites 5-fluorouracil and pemetrexed and/or the group consisting of paclitaxel and docetaxel, administered to the patient in an amount of 150 to 7500 mg in one or more portions within a time period of 2 to 4 weeks.

Generally, the cisplatin can be administered to the patient as is known in the art.

Preferably, cisplatin is administered to the patient in an amount of 50 mg to 500 mg within one cycle, more preferably 80 mg to 300 mg within one cycle. Preferably, the amount of cisplatin is administered to the patient is given in mg per square metre of the by the surface of the patient, i.e. in $mg/m^2$. Accordingly, cisplatin is preferably administered to the patient in an amount of 50 to 150 $mg/m^2$, more preferably 80 to 120 $mg/m^2$ and especially about 100 $mg/m^2$ within one cycle.

The amount cisplatin can be administered in one or more portions, more preferably 1 to 5 portions, even more preferred 1 to 3 and especially preferably in one portion on one day. Generally, cisplatin is administered as an i. V. infusion.

Generally, the carboplatin can be administered to the patient as is known in the art.

Preferably, carboplatin is administered to the patient in an amount of 200 mg to 1000 mg within one cycle, more preferably 300 mg to 800 mg within one cycle and especially 400 to 700 mg within one cycle. Even more preferably, the carboplatin is administered to the patient in an AUC (Area Under the Curve) regimen, more specifically an AUC 4-8 regimen (4-8 mg/ml/min), preferably an AUC 5-7 regimen (5-7 mg/ml/min). The principles of the AUC regimen or dosing are known in the art. Preferably, the amounts to be administered to the patient in the AUC regimen according to the invention are calculated using the Calvert formula and/or the Chatelut formula, preferably the Calvert formula. Calvert Formula:

$$\text{Carboplatin dose (mg) = AUC x (CrCl (ml/min) + 25);}$$

wherein:

$$\text{AUC = Area Under the Curve ((mg/ml x min))}$$

x = multiplied
CrCl = Creatinin Clearence (of the respective patient)
Chatelut formula:

$$\text{Carboplatin dosage (mg) = AUC (mg/ml x min) x carboplatin clearance (ml/min);}$$

wherein:
AUC = Area Under the Curve

Formula suitable for estimation of the carboplatin clearance of a patient for use in the Chatelut formula:

$$\text{for Males} = (0.134 \times weight) + (218 \times weight \times (1-0.00457 \times age)/serum\ creat.)$$

$$\text{for Females} = (0.134 \times weight) + 0.686 \times (218 \times weight \times (1-0.00457 \times age)/serum\ creat.)$$

Age = age in years
x = multiplied
weight = weight in kg serum creat. = the serum concentration of creatinine

The amount carboplatin can be administered in one or more portions, more preferably 1 to 5 portions, even more preferred 1 to 3 and especially preferably in one portion on one day. Generally, carboplatin is administered as an i. V. infusion.

Generally, the oxaliplatin can be administered to the patient as is known in the art.

Preferably, oxaliplatin is administered to the patient in an amount of 50 mg to 500 mg within one cycle, more preferably 80 mg to 300 mg within one cycle. If the duration of the cycle is about three or about five weeks, the oxaliplatin is preferably administered to the patient in an amount of 100 to 500 mg. If the duration of the cycle is about two weeks, the oxaliplatin is preferably administered to the patient in an amount of 50 to 250 mg. Preferably, the amount of oxaliplatin is administered to the patient is given in mg per square metre of the by the surface of the patient, i.e. in mg/m$^2$. Accordingly, oxaliplatin is preferably administered to the patient in an amount of 80 to 150 mg/m$^2$ within one cycle, for example about 130 mg/m$^2$ within one cycle, especially if the duration of the cycle is about three or about four weeks. Alternatively, the oxaliplatin is preferably administered to the patient in an amount of 50 to 100 mg/m$^2$ within one cycle, for example about 85 mg/m$^2$ within one cycle, especially if the duration of the cycle is about two weeks.

The amount oxaliplatin can be administered in one or more portions, more preferably 1 to 5 portions, even more preferred 1 to 3 and especially preferably in one portion on one day. Generally, oxaliplatin is administered as an i. V. infusion.

Thus, a preferred subject of the instant invention is a treatment comprising one or more cycles, preferably 2 to 12 cycles, more preferably about 6 cycles, each cycle consisting of about 21 days or about 28 days, preferably about 21 days,
wherein in each cycle:

a) cyclo-(Arg-Gly-Asp-DPhe-NMe-Val), the pharmaceutically acceptable solvates and/or salts thereof, preferably cyclo-(Arg-Gly-Asp-DPhe-NMe-Val), is administered to the patient
a1) in an amount of about 500 mg per day on 1 to 5 days, preferably 5 consecutive days within the first week of the cycle, more preferably on day 1, 2, 3, 4 and 5 of the first week of the cycle, and additionally in an amount of about 500 mg per day on one day within the second and of one day within the third week and more preferably on day 8 and day 15 of the cycle, or alternatively
a2) in an amount of 2000 mg per day on one or two different days within each week of the cycle, preferably on two different days within each week of the cycle, and more preferably on the days 1 and 4 or 1 and 5 within each week of the cycle;
b) cisplatin, the pharmaceutically acceptable dervatives, solvates and/or salts thereof, preferably cisplatin, is administered to the patient
b1) in an amount of 60 to 120 mg/m$^2$, more preferably in an amount of about 80 mg/m$^2$ or about 100 mg/m$^2$, per day on one day within the first week of the cycle, preferably on day 1 of the first week of the cycle,
b2) preferably, no more cisplatin is administered to the patient during the subsequent weeks of said cycle;
c) cetuximab, the pharmaceutically acceptable dervatives, solvates and/or salts thereof, preferably cetuximab is administered to the patient
c1) in an amount of about 200 to 600 mg/m$^2$, preferably about 250 mg/m$^2$ or about 400 mg/m$^2$, more preferably about 400 mg/m$^2$, per day on one day within the first week, preferably on day 1 of the first week,
a2) in an amount of 200 to 400 mg/m$^2$, preferably about 250 mg/m$^2$, per day on one day during each week of the subsequent weeks of said cycle, preferably on day 1 of each week and more preferably on days 8 and 15 of said cycle; and optionally
e) 5-fluorouracil, the pharmaceutically acceptable dervatives, solvates and/or salts thereof, preferably 5-fluorouracil, is administered to the patient
e1) in an amount of 500 to 1500 mg/m$^2$, preferably about 1000 mg/m$^2$, per day on 2 to 5 days, preferably 4 days and more preferably 4 consecutive days, within the first week of the cycle, even more preferably on day 1, 2, 3 and 4 of the first week of the cycle,

e2) preferably, no more 5-fluorouracil is administered to the patient during the subsequent weeks of said cycle.

Thus, another preferred subject of the instant invention is a treatment comprising one or more cycles, preferably 2 to 12 cycles, more preferably about 6 cycles, each cycle consisting of about 21 days or about 28 days, preferably about 21 days,
wherein in each cycle:

a) cyclo-(Arg-Gly-Asp-DPhe-NMe-Val), the pharmaceutically acceptable solvates and/or salts thereof, preferably cyclo-(Arg-Gly-Asp-DPhe-NMe-Val), is administered to the patient
a1) in an amount of about 500 mg per day on 1 to 5 days, preferably 5 consecutive days within the first week of the cycle, more preferably on day 1, 2, 3, 4 and 5 of the first week of the cycle, and additionally in an amount of about 500 mg per day on one day within the second and on one day within the third week and more preferably on day 8 and day 15 of the cycle, or alternatively
a2) in an amount of 2000 mg per day on one or two different days within each week of the cycle, preferably on two different days within each week of the cycle, and more preferably on the days 1 and 4 or 1 and 5 within each week of the cycle;
b) carboplatin, the pharmaceutically acceptable dervatives, solvates and/or salts thereof, preferably carboplatin, is administered to the patient
b1) in an amount as described herein, preferably as described herein as AUC 5-7 and more preferably described herein as AUC 6, per day on one day within the first week of the cycle, preferably on day 1 of the first week of the cycle,
b2) preferably, no more carboplatin is administered to the patient during the subsequent weeks of said cycle;
c) cetuximab, the pharmaceutically acceptable dervatives, solvates and/or salts thereof, preferably cetuximab is administered to the patient
c1) in an amount of about 200 to 600 mg/m$^2$, preferably about 250 mg/m$^2$ or about 400 mg/m$^2$, more preferably about 400 mg/m$^2$, per day on one day within the first week, preferably on day 1 of the first week,
a2) in an amount of 200 to 400 mg/m$^2$, preferably about 250 mg/m$^2$, per day on one day during each week of the subsequent weeks of said cycle, preferably on day 1 of each week and more preferably on days 8 and 15 of said cycle; and optionally
e) 5-fluorouracil, the pharmaceutically acceptable dervatives, solvates and/or salts thereof, preferably 5-fluorouracil, is administered to the patient
e1) in an amount of 500 to 1500 mg/m$^2$, preferably about 1000 mg/m$^2$, per day on 2 to 5 days, preferably 4 days and more preferably 4 consecutive days, within the first week of the cycle, even more preferably on day 1, 2, 3 and 4 of the first week of the cycle,
e2) preferably, no more 5-fluorouracil is administered to the patient during the subsequent weeks of said cycle.

Thus, an especially preferred subject of the instant invention is a treatment comprising one or more cycles, preferably 2 to 12 cycles, more preferably about 6 cycles, each cycle consisting of about 21 days,
wherein in each cycle:

a) cyclo-(Arg-Gly-Asp-DPhe-NMe-Val), the pharmaceutically acceptable solvates and/or salts thereof, preferably cyclo-(Arg-Gly-Asp-DPhe-NMe-Val), is administered to the patient
a1) in an amount of about 500 mg per day on 1 to 5 days, preferably 5 consecutive days within the first week of the cycle, more preferably on day 1, 2, 3, 4 and 5 of the first week of the cycle, and additionally in an amount of about 500 mg per day on one day within the second and on one day within the third week and more preferably on day 8 and day 15 of the cycle, or alternatively
a2) in an amount of 2000 mg per day on one or two different days within each week of the cycle, preferably on two different days within each week of the cycle, and more preferably on the days 1 and 4 or 1 and 5 within each week of the cycle,
b) cisplatin, the pharmaceutically acceptable dervatives, solvates and/or salts thereof, preferably cisplatin, is administered to the patient
b1) in an amount of 60 to 120 mg/m$^2$, more preferably in an amount of about 100 mg/m$^2$, per day on one day within the first week of the cycle, preferably on day 1 of the first week of the cycle,
b2) preferably, no more cisplatin is administered to the patient during the subsequent weeks of said cycle;
c) cetuximab, the pharmaceutically acceptable dervatives, solvates and/or salts thereof, preferably cetuximab is administered to the patient
c1) in an amount of about 200 to 600 mg/m$^2$, preferably about 400 mg/m$^2$, per day on one day within the first week, preferably on day 1 of the first week, a2) in an amount of 200 to 400 mg/m$^2$, preferably about 250 mg/m$^2$, per day on one day during each week of the subsequent weeks of said cycle, preferably on day 1 of each week and more preferably on days 8 and 15 of said cycle;
and optionally
e) 5-fluorouracil, the pharmaceutically acceptable dervatives, solvates and/or salts thereof, preferably 5-fluorouracil, is administered to the patient
e1) in an amount of 500 to 1500 mg/m$^2$, preferably about 1000 mg/m$^2$, per day on 2 to 5 days, preferably 4 days and more preferably 4 consecutive days, within the first week of the cycle, even more preferably on day 1, 2, 3 and 4 of the first week of the cycle,
e2) preferably, no more 5-fluorouracil is administered to the patient during the subsequent weeks of said cycle.

Thus, another especially preferred subject of the instant invention is a treatment comprising one or more cycles, preferably 2 to 12 cycles, more preferably about 6 cycles, each cycle consisting of about 21 days,
wherein in each cycle:

a) cyclo-(Arg-Gly-Asp-DPhe-NMe-Val), the pharmaceutically acceptable solvates and/or salts thereof, preferably cyclo-(Arg-Gly-Asp-DPhe-NMe-Val), is administered to the patient
a1) in an amount of about 500 mg per day on 1 to 5 days, preferably 5 consecutive days within the first week of the cycle, more preferably on day 1, 2, 3, 4 and 5 of the first week of the cycle, and additionally in an amount of about 500 mg per day on one day within the second and on one day within the third week and more preferably on day 8 and day 15 of the cycle, or alternatively
a2) in an amount of 2000 mg per day on one or two different days within each week of the cycle, preferably on two different days within each week of the cycle, and more preferably on the days 1 and 4 or 1 and 5 within each week of the cycle,
b) carboplatin, the pharmaceutically acceptable dervatives, solvates and/or salts thereof, preferably carboplatin, is administered to the patient
b1) in an amount as described herein, preferably as described herein as AUC 5-7 and more preferably described herein as AUC 6, per day on one day within the first week of the cycle, preferably on day 1 of the first week of the cycle,
b2) preferably, no more carboplatin is administered to the patient during the subsequent weeks of said cycle;
c) cetuximab, the pharmaceutically acceptable dervatives, solvates and/or salts thereof, preferably cetuximab is administered to the patient c1) in an amount of about 200 to 600 mg/m$^2$, preferably about 400 mg/m$^2$, per day on one day within the first week, preferably on day 1 of the first week, a2) in an amount of 200 to 400 mg/m$^2$, preferably about 250 mg/m$^2$, per day on one day during each week of the subsequent weeks of said cycle, preferably on day 1 of each week and more preferably on days 8 and 15 of said cycle;
and optionally
e) 5-fluorouracil, the pharmaceutically acceptable dervatives, solvates and/or salts thereof, preferably 5-fluorouracil, is administered to the patient
e1) in an amount of 500 to 1500 mg/m$^2$, preferably about 1000 mg/m$^2$, per day on 2 to 5 days, preferably 4 days and more preferably 4 consecutive days, within the first week of the cycle, even more preferably on day 1, 2, 3 and 4 of the first week of the cycle,
e2) preferably, no more 5-fluorouracil is administered to the patient during the subsequent weeks of said cycle.

Thus, an even more preferred subject of the instant invention is a treatment comprising one or more cycles, preferably 2 to 12 cycles, more preferably about 6 cycles, each cycle consisting of about 21 days,
wherein in each cycle:

a) cyclo-(Arg-Gly-Asp-DPhe-NMe-Val), the pharmaceutically acceptable solvates and/or salts thereof, preferably cyclo-(Arg-Gly-Asp-DPhe-NMe-Val), is administered to the patient
a1) in an amount of about 500 mg per day on days 1, 2, 3, 4, 5, 8 and 15 of the cycle,
b) one platinum platinum containing chemotherapeutic agent,
either
b') cisplatin, the pharmaceutically acceptable dervatives, solvates and/or salts thereof, preferably cisplatin, is administered to the patient b1) in an amount of 60 to 120 mg/m$^2$, more preferably in an amount of about 100 mg/m$^2$, per day on one day within the first week of the cycle, preferably on day 1 of the first week of the cycle,
b'2) preferably, no more cisplatin is administered to the patient during the subsequent weeks of said cycle;
or
b") carboplatin, the pharmaceutically acceptable dervatives, solvates and/or salts thereof, preferably carboplatin, is administered to the patient

b"1) in an amount as described herein, preferably as described herein as AUC 5-7 and more preferably described herein as AUC 6, per day on one day within the first week of the cycle, preferably on day 1 of the first week of the cycle,

b"2) preferably, no more carboplatin is administered to the patient during the subsequent weeks of said cycle;

c) cetuximab, the pharmaceutically acceptable dervatives, solvates and/or salts thereof, preferably cetuximab is administered to the patient

c1) in an amount of about 200 to 600 mg/m$^2$, preferably about 400 mg/m$^2$, per day on one day within the first week, preferably on day 1 of the first week,

a2) in an amount of 200 to 400 mg/m$^2$, preferably about 250 mg/m$^2$, per day on one day during each week of the subsequent weeks of said cycle, preferably on day 1 of each week and more preferably on days 8 and 15 of said cycle; and optionally

e) 5-fluorouracil, the pharmaceutically acceptable dervatives, solvates and/or salts thereof, preferably 5-fluorouracil, is administered to the patient

e1) in an amount of 500 to 1500 mg/m$^2$, preferably about 1000 mg/m$^2$, per day on 2 to 5 days, preferably 4 days and more preferably 4 consecutive days, within the first week of the cycle, even more preferably on day 1, 2, 3 and 4 of the first week of the cycle,

e2) preferably, no more 5-fluorouracil is administered to the patient during the subsequent weeks of said cycle.

In the treatments described above, the one or more cycles preferably mean one or more cycles substantially without a pause.

In the treatments described above, the administration of the cisplatin and/or the carboplatin can be substituted by the administration of oxaliplatin, preferably the administration of oxaliplatin as described herein.

Another especially preferred subject of the instant invention relates to the use of cyclo-(Arg-Gly-Asp-DPhe-NMe-Val), the pharmaceutically acceptable solvates and/or salts thereof, preferably cyclo-(Arg-Gly-Asp-DPhe-NMe-Val), for the manufacture of a medicament to be used in the methods of treatment described above.

The use as described above and/or below, wherein the weekly administration scheme is applied 1 to 52 times substantially without a pause.

The use as described above and/or below, wherein said administration to the patient within a time period of 2 to 4 weeks is repeated 1 to 12 times substantially without a pause.

The use as described above and/or below, wherein

a) the weekly administration scheme regarding the specific integrin ligand and

b) the administration to the patient within a time period of 2 to 4 weeks regarding

i) the one or more alkylating chemotherapeutic agents and/or

ii) the one or more further chemotherapeutic agents other than the at least one specific integrin ligand and the one or more alkylating chemotherapeutic agents,

run in parallel for one or more weeks.

Recent in vitro results show an increase in cell death/deterioriation after combination treatment of lung cancer cell lines, such as A549, H157, H322, H460 and/or H1975, with specific integrin ligands, such as Vitaxin, Abegrin, CNT095 and cyclo-(Arg-Gly-Asp-DPhe-NMe-Val), and cancer cotherapeutic agents, such as Cisplatin, Oxaliplatin, Vinblastin, Taxol, Gemcitabine, Gleevec, Iressa, and radiotherapy, preferably external beam radiation and/or fractionated external beam radiation. The results suggest that cancer cotherapeutic agents, such as radiation, can induce expression of relevant integrins in lung cancer cells, and/or that the specific integrin ligand is acting as an amplifier of efficacy, e.g. as a radio amplifier. Moreover, combined application of at least one specific integrin ligand and at least one cancer cotherapeutic agent, preferably radiation, results in significant cell kill and thus reduced survival curves of the respective treated cells considerably. Accordingly, the combinations appear to effectively induce cell death, likely due to apoptosis and/or mitotic cell death, in endothelial cells and tumour cells, especially in lung cancer cells and especially in non-small cell lung cancer cells. The extent of effect may depend on the degree of target expression, i.e. integrin expression.

Subject of the instant invention is the use of at least one specific integrin ligand, comprising cyclo-(Arg-Gly-Asp-DPhe-NMeVal) and/or the pharmaceutically acceptable salts thereof, for the manufacture of a medicament for the treatment of tumours, wherein the medicament is to be used in combination with

a) one or more alkylating chemotherapeutic agents, and

b) one or more further chemotherapeutic agents other than the at least one specific integrin ligand and the one or more alkylating chemotherapeutic agents;

as described herein

and/or radiotherapy, preferably external beam radiation, wherein at least the specific integrin ligand cyclo-(Arg-Gly-Asp-DPhe-NMeVal) and/or the pharmaceutically acceptable salts thereof is administered to a patient in an amount of 800 mg to 7000 mg per week.

Optionally, the amount of cyclo-(Arg-Gly-Asp-DPhe-NMeVal) and/or a pharmaceutically acceptable salt thereof, preferably cyclo-(Arg-Gly-Asp-DPhe-NMeVal), to be administered to a patient per week is administered in about equal amounts of about 500 mg or about 2000 mg for each administration.

Optionally, the amount of cyclo-(Arg-Gly-Asp-DPhe-NMeVal) and/or a pharmaceutically acceptable salt thereof, preferably cyclo-(Arg-Gly-Asp-DPhe-NMeVal), is administered to a patient in an amount of about 1000 mg per week, about 1500 mg per week, about 2500 mg per week, about 4000 mg per week or about 6000 mg per week.

Optionally, the amount of about 1000 mg of cyclo-(Arg-Gly-Asp-DPhe-NMeVal) and/or a pharmaceutically acceptable salt thereof, preferably of is cyclo-(Arg-Gly-Asp-DPhe-NMeVal), per week is administered in a twice weekly administration scheme.

Optionally, the amount of about 4000 mg of cyclo-(Arg-Gly-Asp-DPhe-NMeVal) and/or a pharmaceutically acceptable salt thereof, preferably of cyclo-(Arg-Gly-Asp-DPhe-NMeVal), per week is administered in a twice weekly administration scheme, preferably in about equal amounts of about 2000 mg each.

Optionally, the amount of about 6000 mg of cyclo-(Arg-Gly-Asp-DPhe-NMeVal) and/or a pharmaceutically acceptable salt thereof, preferably of cyclo-(Arg-Gly-Asp-DPhe-NMeVal), per week is administered in a three times weekly administration scheme, preferably in about equal amounts of about 2000 mg each.

In the twice weekly administration scheme, the administration is optionally done on a day one and then on day three or a day four. Thus, the twice weekly administration scheme is optionally done either in an alternating every third day/every fourth day scheme or an alternating every fourth day/every third day scheme, such as an administration on mondays and thursdays (as an example of the 3/4 scheme) or tuesdays and fridays (as a further example of the 3/4 scheme), or on Thursdays and Mondays (as an example of the 4/3 scheme) or on Fridays and Tuesdays (as a further example of the 4/3 scheme).

Optionally, the twice weekly or three times weekly administration scheme, preferably the twice weekly or three times weekly administration scheme as described above, can be applied to the patient once or several times. Optionally, it is applied several times, preferably at least three times or at least six times. For example, the these weekly administration schemes can be applied continuously until healing, stable disease or tumor progression takes place. Optionally, the these weekly administration schemes, preferably the the weekly administration schemes as described above, are applied 4 to 156 times, such as about 4 times, about 8 times, about 16 times, about 24 times, about 35 times, about 70 times or about 104 times.

Optionally, the amount of about 1500 mg of cyclo-(Arg-Gly-Asp-DPhe-NMeVal) and/or a pharmaceutically acceptable salt thereof, preferably of cyclo-(Arg-Gly-Asp-DPhe-NMeVal), per week is administered in a three times weekly administration scheme, preferably in about equal amounts of about 500 mg each.

Optionally, the amount of about 6000 mg of cyclo-(Arg-Gly-Asp-DPhe-NMeVal) and/or a pharmaceutically acceptable salt thereof, preferably of cyclo-(Arg-Gly-Asp-DPhe-NMeVal), per week is administered in a three times weekly administration scheme, preferably in about equal amounts of about 2000 mg each.

In the three times weekly administration scheme, the administration is Optionally either done on a day one, on a day three or a day four and then on a day 6, or optionally on a day one, on a day 3 and on a day 5, then followed of two consecutive days off. The latter three times weekly administration scheme, for example, typically starts on a monday, followed by one administration on the following wednesday and one administration on friday, with saturday and sunday off of treatment.

The three times weekly administration scheme, preferably the three times weekly administration scheme as described above, can optionally be applied to the patient once or several times. Preferably, it is applied several times, even more preferably at least three times or at least six times. For example, the three times weekly administration scheme can be applied continuously till healing or tumor progression takes place. Optionally, the twice weekly administration scheme, preferably the twice weekly administration scheme as described above, is applied 4 to 156 times, such as about 4 times, about 8 times, about 16 times, about 24 times, about 35 times, about 70 times or about 104 times.

The three times weekly administration scheme can optionally be combined partially or totally with radiotherapy, preferably radiotherapy as described herein. Optionally, the three times weekly administration scheme is combined partially with radiotherapy.

Optionally, the amount of about 2500 mg of cyclo-(Arg-Gly-Asp-DPhe-NMeVal) and/or a pharmaceutically acceptable salt thereof, preferably of cyclo-(Arg-Gly-Asp-DPhe-NMeVal), per week is administered in a five times weekly administration scheme, preferably in about equal amounts of about 500 mg each. In the five times weekly administration scheme, the administration is preferably done on five consecutive days, preferably followed by 2 days in a row off. This "5 days of consecutive administration followed by 2 consecutive days off' scheme can be repeated once or several times. Preferably, this before described "5 days of consecutive administration followed by 2 consecutive days off' scheme is performed more than once but preferably less than 18 times, more preferably 2 to 12 times, even more preferably 3 to 8 times and especially 4 to 6 times, for example 2 times, 3 times, 4 times, 5 times, 6 times, 8 times or 12 times. Especially preferably, this "5 days of consecutive administration followed by 2 consecutive days off" scheme is applied 6 times.

Optionally, this "5 days of consecutive administration followed by 2 consecutive days off" scheme is combined with radiotherapy as described herein, preferably radiotherapy as described herein that is applied to the patient in an analog "5 days of consecutive application followed by 2 consecutive days off' scheme that preferably runs in parallel to the other scheme, preferably with the same two days off.

Regarding the herein described weekly administation amounts and/or schemes, the specific integrin ligand cyclo-(Arg-Gly-Asp-DPhe-NMeVal) and/or the pharmaceutically acceptable salts thereof, preferably cyclo-(Arg-Gly-Asp-DPhe-NMeVal), is optionally administered in a timed administration as described herein, generally 1,5 to 20 hours (h), preferably 2 to 16 h, more preferably 2 to 12 h, even more preferably 2 to 10 h, even more preferably 3 to 10 h and especially 2 to 8 h prior to the application of the radiotherapy. Alternatively, the specific integrin ligand cyclo-(Arg-Gly-Asp-DPhe-NMeVal) and/or the pharmaceutically acceptable salts thereof is administered in a timed administration as described herein, preferably 1 to 10 hours (h), preferably 1 to 6, more preferably 2 to 8, even more preferably 3 to 8 h, even more preferably 3 to 6 and especially 4 to 8 h prior to the application of the radiotherapy.

Optionally, the administration of the specific integrin ligand cyclo-(Arg-Gly-Asp-DPhe-NMeVal) and/or the pharmaceutically acceptable salts thereof, preferably cyclo-(Arg-Gly-Asp-DPhe-NMeVal), is combined, partially or totally, preferably partially, with the administration or delivery of focal radiotherapy, wherein 20 to 50 Gray (Gy), preferably 25 to 40 Gy, more preferably 28 to 25 Gy, for example about 28 Gy, about 30 Gy or about 35 Gy are administered or delivered to the patient, preferably in fractions of 0.5 to 5 Gy, more preferably 0.8 to 3 Gy and especially 1 to 2.5 Gy, for example about 1.0, about 1.3 Gy, about 1.6 Gy, about 1.8 Gy, about 2.0 Gy, about 2.5 Gy or about 3.0 Gy, per per administration or delivery, which is preferably also the amount of radiation per day on which the administration or delivery of the radiation takes place. Accordingly, an administration or delivery of 1.5 to 2.5 Gy and preferably 1.8 to 2.2 Gy per day for 2 or 3 days within one week is preferred. Accordingly, an administration or delivery of 0.7 to 1.3 Gy and preferably 0.9 to 1.2 Gy per day for 3 to 6 days, preferably for 5 days and more preferably 5 consecutive days, within one week, is also preferred. Generally, the administration or delivery of 1.0 to 3.0 Gy, preferably about 1.0, about 2.0 Gy or about 3.0 Gy per day for 2 or 3 days within one week is especially preferred. The kind of application of focal radiotherapy as described above is preferred in the treatment of brain metastases.

Typically, both the amounts of about 30 Gy and about 60 Gy are administered or delivered to the patient within about six consecutive weeks.

Another of the present disclosure relates to a treatment of locally advanced lung cancer, comprising administering at least one specific integrin ligand consisting of cyclo-(Arg-Gly-Asp-DPhe-NMeVal) and/or a pharmaceutically acceptable salt thereof, in combination with more than one cancer cotherapeutic agent as described herein, preferably selected from alkylating agents and antimetabolites as described herein, and radiotherapy as described herein. Preferably a combination of at least one alkylating agent and at least one antimetabolites is applied, preferably in combination with radiotherapy, preferably fractionated focal radiotherapy as described herein. Preferably, a combination of the alkylating agent cisplatin with the antimetabolite gemcitabine or a combination of the alkylating agent carboplatin and the antimetabolite paclitaxel is applied, optionally combined with fractionated focal radiotherapy, preferably consisting of about 60 Gy, preferably delivered over a period of about six weeks. Preferably, the specific integrin ligand is administered in a timed administration as described herein. If the specific integrin ligand is cyclo-(Arg-Gly-Asp-DPhe-NMeVal), it is preferably administered to the patient in a dosage and/or a weekly administration scheme as described in the treatment and/or administration schedules described herein.

Another instance of the present disclosure relates to a treatment of locally advanced head and neck cancer, comprising administering at least one specific integrin ligand consisting of cyclo-(Arg-Gly-Asp-DPhe-NMeVal) and/or a pharmaceutically acceptable salt thereof, in combination with more than one cancer cotherapeutic agent as described herein, preferably selected from alkylating agents, for example cisplatin, antimetabolites, for example 5-FU or combinations comprising 5-FU, alkaloids, for example paclitaxel or docetaxel, and compounds targeted against PDGF, PDGFR, EGFR, VEGF, VEGFR and/or VEGFR2, preferably selected from Bevacizumab (rhuMAb-VEGF, Avastin®), Cetuximab (Erbitux®), Nimotuzumab, Sorafenib (Nexavar®), Sunitinib (Sutent®) and ZD6474 (ZACTIMA™), and radiotherapy, preferably fractionated focal radiotherapy as described herein, and combinations thereof.

Preferred is a combination of at least one alkylating agent, preferably comprising cisplatin, and radiotherapy, preferably fractionated focal radiotherapy as described herein. Additionally preferred is a combination of at least one antimetabolite, comprising 5-FU, and radiotherapy, preferably fractionated focal radiotherapy as described herein. Additionally preferred is a combination of at least one alkaloid, comprising paclitaxel or docetaxel, and radiotherapy, preferably fractionated focal radiotherapy as described herein. Preferred is a combination of at least one alkylating agent, preferably comprising cisplatin, at least one antimetabolite, comprising 5-FU, and radiotherapy, preferably fractionated focal radiotherapy as described herein. Additionally preferred is a combination of at least one compound targeted against PDGF, PDGFR, EGFR, VEGF, VEGFR and/or VEGFR2, preferably selected from Bevacizumab (rhuMAb-VEGF, Avastin®), Cetuximab (Erbitux®), Nimotuzumab, Sorafenib (Nexavar®), Sunitinib (Sutent®) and ZD6474 (ZACTIMA™), and radiotherapy, preferably fractionated focal radiotherapy as described herein. The fractionated focal radiotherapy preferably consists of about 60-70 Gy, preferably delivered over a period of about six weeks, about 2 or about 3 Gy per fraction. Preferably, the specific integrin ligand is administered in a timed administration as described herein. If the specific integrin ligand is cyclo-(Arg-Gly-Asp-DPhe-NMeVal), it is preferably administered to the patient in a dosing and/or a weekly administration scheme as described in the methods of treatment and/or administration schedules described herein.

[For technical information only]: Another preferred subject of the present disclosure relates to a method of treatment of locally advanced head and neck cancer, comprising administering at least one specific integrin ligand, more preferably at least one specific integrin ligand as described herein, even more preferably a specific integrin ligand selected from the group consisting of LM609, 17E6, Vitaxin, Abegrin, Abciximab, P1F6, 14D9.F8, CNTO95 and cyclo-(Arg-Gly-Asp-DPhe-NMe-Val), more preferably Vitaxin, Abegrin, CNTO95, Abciximab and cyclo-(Arg-Gly-Asp-DPhe-NMe-Val), and especially preferably consisting of cyclo-(Arg-Gly-Asp-DPhs-NMeVal) and/or a pharmaceutically acceptable salt thereof, in combination with at least one cancer cotherapeutic agent as described herein, preferably three cancer cotherapeutic agents, selected from alkylating agents, for example cisplatin, antimetabolites, for example 5-FU or combinations comprising 5-FU, and alkaloids, for example paclitaxel or docetaxel. In metastatic head and neck cancer, the combinatin of a specific integrin ligand with the cancer cotherapeutics Cisplatin, 5-FU and Taxan, preferably paclitaxel or docetaxel, is especially preferred.

[For technical information only]: Another preferred subject of the present disclosure relates to a method of treatment of head and neck cancer, preferably locally advanced head and neck cancer, comprising administering at least one specific integrin ligand, more preferably at least one specific integrin ligand as described herein, even more preferably a specific integrin ligand selected from the group consisting of LM609, 17E6, Vitaxin, Abegrin, Abciximab, P1F6, 14D9.F8, CNTO95 and cyclo-(Arg-Gly-Asp-DPhe-NMe-Val), more preferably Vitaxin, Abegrin, CNTO95, Abciximab and cyclo-(Arg-Gly-Asp-DPhe-NMe-Val), and especially preferably consisting of cyclo-(Arg-Gly-Asp-DPhe-NMe-Val) and/or a pharmaceutically acceptable salt thereof, in combination with at least one cancer cotherapeutic agent as described herein, selected from compounds targeted against PDGF, PDGFR, EGFR, VEGF, VEGFR and/or VEGFR2, preferably selected from Bevacizumab (rhuMAb-VEGF, Avastin®), Cetuximab (Erbitux®), Nimotuzumab, Sorafenib (Nexavar®), Sunitinib (Sutent®) and ZD6474 (ZACTIMA™), and radiotherapy, preferably fractionated focal radiotherapy as described herein, more preferably 50-70 Gy, in fractions of 1.2 to 2.2 Gy, preferably about 2 Gy, preferably applied on 5 days per week. Especially preferably, a combination of a specific integrin ligand, at least one targeted compound and radiotherapy as described above is applied.

If fractionated focal radiotherapy is applied with respect to brain metastases, preferably brain metastases of other cancer types as described herein, it preferably consists of about 25 to 45 Gy, more preferably 30 to 40 gy, preferably delivered in frations of 1.5 to 3.5, more preferably 1.8 to 3, e. g. about 2 Gy or about 3 Gy, preferably over a period of about three weeks, preferably 5 days a week.

[For technical information only]: Another preferred subject of the present disclosure relates to a method of prophylactic irradiation, preferably prophylactic cranial irradiation or prophylactic mediastinal irradiation, comprising administering at least one specific integrin ligand, more preferably at least one specific integrin ligand as described herein, even more preferably a specific integrin ligand selected from the group consisting of LM609, 17E6, Vitaxin, Abegrin, Abciximab, P1F6, 14D9.F8, CNTO95 and cyclo-(Arg-Gly-Asp-DPhe-NMe-Val), more preferably Vitaxin, Abegrin, CNTO95, Abciximab and cyclo-(Arg-Gly-Asp-DPhe-NMe-Val), and especially preferably consisting of cyclo-(Arg-Gly-Asp-DPhe-NMeVal) and/or a pharmaceutically acceptable salt thereof, and radiotherapy, preferably fractionated focal radiotherapy as described herein. The method of prophylactic cranial irradiation is preferably applied with respect to lung cancer, preferably small cell lung cancer, even more preferably small cell lung cancer in complete remission, preferably after chemotherapy and/or surgical procedures. The method of prophylactic mediastinal irradiation is preferably applied with respect to lung cancer, more preferably small cell lung cancer, even more preferably small cell lung cancer in complete remission, preferably after chemotherapy and/or surgical procedures.

In all of the above given methods of treatment or methods of prophylactic irradiation, a timed administration of the at least one specific integrin ligand is preferred.

With respect to the methods of treatment, administered amounts and/or the administration schemes described herein regarding of the specific integrin ligand cyclo-(Arg-Gly-Asp-DPhe-NMeVal) and/or a pharmaceutically acceptable salt thereof, preferably of cyclo-(Arg-Gly-Asp-DPhe-NMeVal), the amounts of (about) 500 mg or (about) 1000 mg to be administered at each administration as well as the amounts of (about) 1000 mg, (about) 1500 mg, (about) 2000 mg, (about) 2500 mg, (about) 4000 mg and (about) 6000 mg given for the weekly administration schemes are preferably calculated on the compound cyclo-(Arg-Gly-Asp-DPhe-NMeVal) as such (which is also referred to as the inner or internal salt of cyclo-(Arg-Gly-Asp-DPhe-NMeVal). Accordingly, if a different form or derivative, such as the pharmacologically acceptable salts and solvates, of the specific integrin ligand cyclo-(Arg-Gly-Asp-DPhe-NMeVal) is to be administered to the patient, it is preferably administered in an amount equimolar to the amounts given above for the compound cyclo-(Arg-Gly-Asp-DPhe-NMeVal) as such.

Anothoer subject of the present disclosure is:

[45] A method for the production of a medicament for the combined use as a combination therapy for the treatment of cancer as described herein, the medicament comprising, preferably in two or more discrete therapy forms, a composition containing at least one specific integrin ligand as described herein, and a composition containing one or more alkylating chemotherapeutic agents as described herein, and at least one further cancer cotherapeutic agent different from the at least one specific integrin ligand of a) and from the one or more alkylating chemotherapeutic agents of b) as described herein; preferably as described above and/or below.

The specific integrin ligand to be used according to the invention surprisingly show an advantageously improved effect on patients that are having increased DNA methylation status, are having a partial or complete methylation of at least one promoter of at least one MGMT gene and/or are having an abnormal level of MGMT protein; especially an abnormal low level of MGMT protein. Accordingly, the invention provides medicaments and methods that can be advantageously used to treat patients associated with one or more of the aforementioned effects or defects.

Therefore, subject of the instant invention is a medicament as described herein for use in the treatment of patients colon and/or rectal cancer, wherein the medicament is for treatingpatients having an increased DNA methylation status, patients showing partial or complete methylation of at least one promotor of at least one MGMT gene and or patients having an abnormal level of MGMT protein, especially an abnormal low level of MGMT protein. Such patients are preferably referred to as "methylated patients" .

These subjects are explained and discussed in more detail below.

Methylation of the DNA-repair gene $O^6$-methylguanine-DNA methyltransferase (MGMT), more correctly called $O^6$-methylguanine-DNA methyltransferase repair gene or short MGMT repair gene, causes gene silencing. This epigenetic modification has been associated with a favourable prognosis in patients with many different cancer types, such as glioblastoma (GBM), who receive alkylating agents, for example, nitrogen mustards, ethyleneimine compounds, alkyl sulphonates and other compounds with an alkylating action, preferably selected from nitrosoureas, preferably ACNU, BCNU and CCNU, busulfan, melphalan, carboplatin, cisplatin, oxaliplatin, cyclophosphamide, dacarbazine, carmustine, ifosfamide and lomustine, temozolomide and altretamine, or campthothecin. Accordingly, there is a relationship between MGMT promoter methylation and the survival rate and sensitivity to alkylating agents, such as temozolomide. The MGMT enzyme removes alkyl groups from the 06 position of guanine, the site of a number of chemotherapy-induced DNA alkylations. These chemotherapy induced alkylations lead to DNA damage in the tumor cells, including DNA double strand breaks and mismatches, which trigger apoptosis and cytotoxicity [5,6]. The MGMT enzyme repairs DNA damage, thus interfering with the therapeutic effects of chemotherapy alkylating agents [7-10]. Methylation of discrete regions of the MGMT promoter CpG island is associated with silencing of the gene and diminished DNA-repair enzyme activity [11-13]. Previous studies have indicated that 30-40% of GBM patients have methylated MGMT promoter [1-4].

The MGMT promoter methylation and thus the methylation status of the MGMT can be advantageously determined using a 2-stage methylation specific PCR analysis on DNA extracted from tumor specimens, such as tumour specimens which have been snap frozen at surgery. The Methylation specific PCR analysis can be easily performed according to methods in the art. Preferably it can be performed by the Method by Hegi et al., NEJM, 2005, 352; 997-1003); the following method has been successfully been used in a Phase III trial assessing the methylation status of a subset of the patients (tissue available):

**DNA extraction and methylation-specific polymerase chain reaction**

Genomic DNA is isolated from one or two paraffin sections of glioblastoma tissue (Ex-Wax DNA Extraction Kit S4530, Chemicon) (proteinase digestion lasted a maximum of six hours). DNA is denatured with sodium hydroxide in a volume of 35 $\mu$l and subjected to bisulfite treatment in a volume of 360 $\mu$l (4.4 M sodium bisulfite and 20 mM hydroquinone) for five hours at 55°C and then purified (Wizard DNA Clean-Up System A7280, Promega). Unmethylated cytosine, but not its methylated counterpart, is modified into uracil by the treatment. The methylation-specific polymerase chain reaction (PCR) is performed in a two-step approach. [Palmisano WA, Divine KK, Saccomanno G, et al. Predicting lung cancer by detecting aberrant promoter methylation in sputum. Cancer Res 2000;60:5954-8.]

The results can be confirmed in an independent experiment, starting with reisolation of DNA from the tumor. The PCR products are separated on 4 percent agarose gels. The investigators who selected and analyzed the glioblastoma samples are blinded to all clinical information.

Alternatively, it can be performed according to the method described by Donson et al. in Journal Pedriatic Blood Cancer, 2006.

According to Donson et al., the MGMT promoter methylation/methylation status of the MGMT can be advantageously determined according to the following procedure:

DNA Extraction and Methylation-Specific Polymerase Chain Reaction

Genomic DNA is isolated from snap frozen tumor obtained at surgery (COMIRB 95-500) and GBM cell-lines using a DNeasy kit (Qiagen, Valencia, CA). DNA methylation patterns in the CpG island of the MGMT gene are determined by methylation specific PCR. This procedure involves chemical modification of unmethylated, but not methylated cytosines to uracil, followed by a nested, twostage PCR [17]. One microgram of DNA is denatured with sodium hydroxide (final conc. 0.3 M) in a volume of 55 ml and subjected to bisulfite treatment in a volume of 610 ml (3.3 M sodium bisulfite and 0.5mMhydroquinone) for 16 hr at 558C and then purified using the Wizard DNA Clean-Up System (Promega, Madison, WI). PCR is performed to amplify a 289-bp fragment of the MGMT gene including a portion of the CpG-rich promoter region. The primers recognize the bisulfite-modified template but do not discriminate between methylated and unmethylated alleles. Primer sequences used in the stage 1 amplification of theMGMTgene are as follows: MGMT-stage 1-Forward, 50-GGATATGTTGGGATAGTT-30; and MGMT-stage 1-Reverse, 50-CCAAAAACCCCAAACCC- 30. Master Mix (Fermentas, Hanover, MD). The PCR amplification protocol for stage 1 is as follows: 958C for 10 min, then denature at 958C for 30 sec, anneal at 528C for 30 sec, extension at 728C for 30 sec for 40 cycles followed by a 10min final extension.A25-ml volume is used in all of the PCR reactions. The stage-1 PCR products are diluted 50-fold, and 5 ml of this dilution is subjected to a stage-2 PCR in which primers specific to methylated or unmethylated template are used. Primer sequences for the stage 2 PCR for the unmethylated reaction are MGMT-stage 2-Forward, 50-TTTGTGTTTTGATGTTTGTAGGTTTTTGT-30 and MGMT-stage 2-Reverse, 50-AACTCCACACTCTTCCAAAAACAAAACA- 30 and for the methylated reaction MGMT-stage 2-forward 50-TTTCGACGTTCGTAGGTTTTCGC- 30 and MGMT-stage 2-reverse 50-GCACTCTTCCGAAAACGAAACG- 30. The PCR amplification protocol for stage 2 is as follows: 958C for 10 min, then denature at 958C for 15 sec, anneal at 628C for 15 sec, extension at 728C for 15 sec for 40 cycles followed by a 10 min final 728C extension. DNA from normal human lymphocytes treated in vitro with SssI methyltransferase (New England Biolabs, Beverly, MA) is used as positive control for methylated alleles of MGMT and untreated DNA from normal lymphocytes is used as negative control for methylated alleles of MGMT. Each PCR reaction (10 ml) is directly loaded onto 4% agarose gel, stained with ethidium bromide and visualized under UV illumination. Statistical Analysis can be performed with methods known in the art, such as the methods by Kaplan-Meier, correlation and statistical significance analyses, for example using the Prism statistical analysisprogram (GraphPad Software, Inc., San Diego, CA). Methylguanine-DNA methyltransferase promoter methylation status analysis is performed on snap frozen tissue of the patients. MGMT methylation status can regularly be determined out the tumors. In a part of the patients, the samples tested for MGMT promoter methylation status proved to be partially methylated (Fig. A). None of the samples showed complete methylation. The incomplete methylation observed may be due to tumor heterogeneity, infiltrating peripheral blood lymphocytes and/or vasculature. For comparison purposes, it can be determined whether partial methylation of the tumor MGMT promoter can be responsible for this observation by investigating the MGMT promoter methylation status of 6 GBM cell-lines, including cell-line 145 which is established from a patient who is treated with temozolomide and who's snap frozen tumor is also analyzed in the above study. In four out of the six celllines studied, partial methylation of promoter is observed (Fig. B). The results show that even in pure GBM cell-lines, partial MGMT promoter methylation can exist.

## Fig. A

Fig. A. Methylation status of the MGMT promoter in GBM biopsy specimens, as determined by a nested methylation-specific PCR assay. DNA from normal peripheral blood lymphocytes (PBL) is used as a control for the unmethylated MGMT promoter (U), enzymatically methylated DNA from PBL (MPBL) served as a positive control for the methylated MGMT promoter (M), and water is used as a negative control for the PCR. A 100-bp marker ladder is loaded to estimate molecular size, as shown on the left scale (L).

## Fig. B

| | U251 | | U118 | | G12 | | D3 | | D19 | | 145 | |
|---|---|---|---|---|---|---|---|---|---|---|---|---|
| L | U | M | U | M | U | M | U | M | U | M | U | M |
| 100 bp | | | | | | | | | | | | |
| 80 bp | | | | | | | | | | | | |

Fig. B. Methylation status of the MGMTpromoter inGBMcell-lines, as determined by a nested methylation-specific PCR assay. A 100-bp marker ladder is loaded to estimate molecular size, as shown on the left scale (L).

The MGMT analysis technique described above has been employed in the majority of recent studies showing MGMT methylation to be a successful predictor of response to alkylating agents [1-3]. This technique has superseded earlier techniques of enzyme activity measurement after it was demonstrated that MGMT methylation was the main cause of loss of MGMT enzymatic activity in GBM.

Patients that are tested as patients showing MGMT methylation or that can be tested as patients showing MGMT methylation, preferably using the above described method, an analog method thereof, or any other method which is equally suitable according to the understanding of the ones skilled in the art, are to be regarded as "methylated patients" according to the invention, more preferably as patients having an increased DNA methylation status and/or as patients showing partial or complete methylation of at least one promotor of at least one MGMT gene. They thus belong to the collective of patients that can be especially advantageously treated by the methods of treatment or the medicaments according to the invention.

However, such techniques, e.g. the method described below, can preferably be used in concordance with the instant invention with respect to the MGMT status.

Chemotherapeutic efficacy, the ability of chemotherapy to eradicate tumor cells without causing lethal host toxicity, depends on drug selectivity. One class of anticancer drugs, alkylating agents, cause cell death by binding to DNA which structurally distorts the DNA helical structure preventing DNA transcription and translation. In normal cells, the damaging action of alkylating agents can be repaired by cellular DNA repair enzymes, in particular $O^6$-methylguanine-DNA methyltransferase (MGMT) also known as $O^6$-alkylguanine-DNA-alkyltransferase (AGAT). The level of MGMT varies in tumor cells, even among tumors of the same type. The gene encoding MGMT is not commonly mutated or deleted. Rather, low levels of MGMT in tumor cells are due to an epigenetic modification; the MGMT promoter region is methylated, thus inhibiting transcription of the MGMT gene and preventing expression of MGMT.

Methylation has been shown by several lines of evidence to play a role in gene expression, cell differentiation, tumorigenesis, X-chromosome inactivation, genomic imprinting and other major biological processes. In eukaryotic cells, methylation of cytosine residues that are immediately 5' to a guanosine, occurs predominantly in cytosine-guanine (CG) poor regions. In contrast, CpG islands remain unmethylated in normal cells, except during X-chromosome inactivation and parental specific imprinting where methylation of 5' regulatory regions can lead to transcriptional repression. Expression of a tumor suppressor gene can also be abolished by de novo DNA methylation of a normally unmethylated CpG.

Hypermethylation of genes encoding DNA repair enzymes can serve as markers for predicting the clinical response to certain cancer treatments. Certain chemotherapeutic agents (including alkylating agents for example) inhibit cellular proliferation by cross-linking DNA, resulting in cell death. Treatment efforts with such agents can be thwarted and resistance to such agents develops because DNA repair enzymes remove the cross-linked structures. In view of the deleterious side effects of most chemotherapeutic drugs, and the ineffectiveness of certain drugs for various treatments, it is desirable to predict the clinical response to treatment with chemotherapeutic agents.

U.S. Pat. No. 6,773,897 discloses methods relating to chemotherapeutic treatment of a cell proliferative disorder. In particular, a method is provided for "predicting the clinical response to certain types of chemotherapeutic agents", including specific alkylating agents. The method entails determination and comparison of the methylation state of nucleic acid encoding a DNA repair enzyme from a patient in need of treatment with that of a subject not in need of treatment. Any difference is deemed "predictive" of response. The method, however, offers no suggestion of how to improve clinical outcome for any patient with an unfavorable "prediction". Temozolomide is an alkylating agent available from Schering Corp. under the trade name of Temodar® in the United States and Temodal® in Europe. Temodar® Capsules for oral administration contain temozolomide, an imidazotetrazine derivative. The chemical name of temozolomide is 3,4-dihydro-3-methyl-4-oxoimidazo[5,1-d]-as-tetrazine-8-carboxamide (see U. S. Pat. No. 5,260,291). The cytotoxicity of temozolomide or metabolite of it, MTIC, is thought to be primarily due to alkylation of DNA. Alkylation (methylation) occurs mainly at the $O^6$ and $N^7$ positions of guanine. Temodar® (temozolomide) Capsules are currently indicated in the United States for the treatment of adult patients with newly diagnosed gliobastoma multiforme as well as refractory anaplastic astrocytoma, i.e. patients at first relapse who have experienced disease progression on a drug regimen containing a nitrosourea and procarbazine. Temodal® is currently approved in Europe for the treatment of patients with malignant glioma, such as glioblastoma multiforme or anaplastic astrocytoma showing recurrence or progression after standard therapy.

According to the invention, alternatively to the method described above, the level of methylation of MGMT gene is assessed by determining the level of MGMT protein in a sample obtained from the patient. The level can be classified as being "Very Low" "Low", "Moderate", or "High", preferably as described in more detail below.

Assessing whether or not the MGMT gene is methylated can be performed using any method known to one skilled in the art. Techniques useful for detecting methylation of a gene or nucleic acid include, but are not limited to those described by Ahrendt et aL, J. Natl. Cancer Inst., 91:332-339 (1999); Belsinky etal., Proc. Natl. Acad. Sci. U.S.A., 95:11891-11896 (1998), Clark et al., NucleicAcids Res., 22:2990-2997 (1994); Herman etaL, Proc Natl Acad Sd U.S.A., 93:9821-9826 (1996); Xiong and Laird, Nucleic Acids Res., 25:2532-2534 (1997); Eads et aL, Nuc. Acids. Res., 28:e32 (2002); Cottrell et al., Nucleic Acids Res., 32:1-8 (2004). All references cited herein are incorporated herein by reference.

Methylation-specific PCR (MSP; Herman et al., Proc. Natl. Acad Sci. USA, 93(18):9821-9826 (1996); Esteller etal., Cancer Res., 59:793-797 (1999)) see also U.S. Pat. No. 5,786,146, issued Jul. 28, 1998; U.S. Pat. No. 6, 017,704, issued Jan. 25, 2000; U.S. Pat. No. 6,200,756, issued Mar. 13, 2001; and U.S. Pat. No. 6,265,171, issued Jul. 24, 2001; U.S. Pat. No. 6, 773,897 issued August 10, 2004; the entire contents of each of which is incorporated herein by reference can rapidly assess the methylation status of virtually any group of CpG sites within a CpG island, independent of the use of methylation-sensitive restriction enzymes. This assay entails initial modification of DNA by sodium bisulfite, converting all unmethylated, but not methylated, cytosines to uracil, and subsequent amplification with primers specific for methylated versus unmethylated DNA. MSP requires only small quantities of DNA, is sensitive to 0.1% methylated alleles of a given CpG island locus, and can be performed on DNA extracted from paraffin-embedded samples. MSP eliminates the false positive results inherent to previous PCR-based approaches which relied on differential restriction enzyme cleavage to distinguish methylated from unmethylated DNA. This method is very simple and can be used on small amounts of tissue or a few cells.

An illustrative example of a Western blot assay useful for this embodiment of the invention to measure the level of MGMT protein in patient samples is presented in U.S. Pat. No. 5,817,514 by Li et al., the entire disclosure of which is incorporated herein by reference. Li et al. described monoclonal antibodies able to specifically bind either to native human MGMT protein or to human MGMT protein having an active site which is alkylated. An illustrative example of an immunohistochemical technique useful for this embodiment of the invention to measure the level of MGMT protein in patient samples is presented in U.S. Pat. No. 5,407,804, the entire disclosure of which is incorporated herein by reference. Monoclonal antibodies are disclosed which are able to specifically bind to the MGMT protein in single cell preparations (immunohistochemical staining assays) and in cell-extracts (immunoassays).

The use of fluorescent read out coupled with digitization of the cell image is described and allows for quantitative measurement of MGMT levels in patient and control samples, including but not limited to tumor biopsy samples. Useful techniques for measuring the enzymatic acitivity of MGMT protein include but are not limited to methods described by: Myrnes et al., Carcinogenesis, 5:1061-1 064 (1984); Futscher et al., Cancer Comm., 1: 65-73 (1989); Kreklaw etal., J. Pharmacol. Exper. Ther., 297(2):524-530 (2001); and Nagel et al., Anal. Biochem., 321(1):38-43 (2003), the entire disclosures of which are incorporated herein in their entireties.

According to one mode of this invention, the level of MGMT protein expressed by cells of the patient is assessed by measurement of the MGMT protein, e.g., by Western blot using an antibody specific to MGMT, see for example, U.S. Pat. No. 5,817,514 (supra) by Li et al. for a description of a Western blot assay to determine MGMT level. The level is compared to that expressed by normal lymphocytes known to express MGMT.

Patient MGMT protein levels are preferably classified as follows: Very Low = 0-30% of the MGMT expressed by normal lymphocytes; Low = 31-70% of the MGMT expressed by normal lymphocytes; Moderate = 71-90% and High = 91-300% or higher of the MGMT expressed by normal lymphocytes.

Patients that are tested as patients having Moderate or less MGMT protein levels or that can be tested as patients having Moderate or less MGMT protein levels, preferably using the above described method, an analog method thereof, or any other method which is equally suitable according to the understanding of the ones skilled in the art in the art, are to be regarded as "methylated patients" according to the invention. They thus belong to the collective of patients that can be especially advantageously treated by the methods of treatment or the medicaments according to the invention.

Accordingly, patients that have or can be shown to have Moderate (= 71-90%), preferably (Low = 31-70%) and more preferably Very Low (= 0-30%), of the MGMT expressed by normal lymphocytes are preferably to be regarded as "methylated patients" according to the invention, more preferably as patients having an increased DNA methylation status and/or as patients showing partial or complete methylation of at least one promotor of at least one MGMT gene. They thus belong to the collective of patients that can be especially advantageously treated by the methods of treatment or the medicaments according to the invention.

Thus, an especially preferred subject of the invention is a method or a use as described herein, wherein the medicament is for treating patients having an increased DNA methylation status.

Thus, an especially preferred subject of the invention is a use as described herein, wherein the medicament is for treating patients showing partial or complete methylation of at least one promotor of at least one MGMT gene.

Thus, an especially preferred subject of the invention is a use as described herein, wherein the medicament is for treating patients, having a Moderate, preferably a Low and more preferably a Very Low level of MGMT protein, preferably in comparison of the MGMT expressed by normal lymphocytes.

Thus, an especially preferred subject of the invention is a use as described herein, wherein the medicament is for treating patients having an increased DNA methylation status, and wherein said method comprises the administration of one or more alkylating agents, preferably selected from, nitrogen mustards, ethyleneimine compounds, alkyl sulphonates and other compounds with an alkylating action, preferably selected from nitrosoureas, preferably ACNU, BCNU and CCNU, busulfan, melphalan, carboplatin, cisplatin, oxaliplatin, cyclophosphamide, dacarbazine, carmustine, ifosfamide and lomustine, temozolomide and altretamine, or campthothecin.

Thus, an especially preferred subject of the invention is a use as described herein, wherein the medicament is for treating patients showing partial or complete methylation of at least one promotor of at least one MGMT gene and wherein said method comprises the administration of one or more alkylating agents, preferably selected from, nitrogen mustards, ethyleneimine compounds, alkyl sulphonates and other compounds with an alkylating action, preferably selected from nitrosoureas, preferably ACNU, BCNU and CCNU, busulfan, melphalan, carboplatin, cisplatin, oxaliplatin, cyclophosphamide, dacarbazine, carmustine, ifosfamide and lomustine, temozolomide and altretamine, or campthothecin.

Thus, an especially preferred subject of the invention is a use as described herein, wherein the medicament is for treating patients, having a Moderate, preferably a Low and more preferably a Very Low level of MGMT protein, preferably in comparison of the MGMT expressed by normal lymphocytes, and wherein said method comprises the administration of one or more alkylating agents, preferably selected from, nitrogen mustards, ethyleneimine compounds, alkyl sulphonates and other compounds with an alkylating action, preferably selected from nitrosoureas, preferably ACNU, BCNU and CCNU, busulfan, melphalan, carboplatin, cisplatin, oxaliplatin, cyclophosphamide, dacarbazine, carmustine, ifosfamide and lomustine, temozolomide and altretamine, or campthothecin.

In the afore described uses with respect to MGMT, the uses comprise the administration of one or more specific integrin ligands, selected from cyclo-(Arg-Gly-Asp-DPhe-NMe-Val), the pharmaceutically acceptable solvates and salts thereof, and especially cyclo-(Arg-Gly-Asp-DPhe-NMe-Val).

Methods to assess an increased DNA methylation status and/or showing partial or complete methylation of at least one promotor of at least one MGMT gene in patients are known in the art. Accordingly, patients to be advantagously treatable by uses as described herein can readily determined by the ones skilled in the art.

A preferred subject of the instant invention is a use as described herein, wherein the medicament is to be used in the treatment of recurrent cancer, for example in a second line or subsequent treatment setting.

A more preferred subject of the instant invention is a use as described herein, wherein the medicament is to be used in the treatment of recurrent cancer, for example in a second line or subsequent treatment setting, wherein the cancer is as defined herein.

An even more preferred subject of the instant invention is a use as described herein, wherein the medicament is to be used in the treatment of newly diagnosed cancer, preferably in a first line treatment setting.

A use according to one of the preceding claims, wherein a) is preferably administered 1 to 20 hours (h), preferably 2 to 12 h, and most preferably 2 to 6 h prior to the application of b) and/or c).

A use according to one of the preceding claims, wherein the medicament is for treating patients having an increased DNA methylation status.

A use according to one of the preceding claims, wherein the medicament is for treating patients showing partial or complete methylation of at least one promotor of at least one MGMT gene.

A use according to one of the preceding claims, wherein the medicament is for treating newly diagnosed cancer, preferably in a first line chemotherapy setting.

**Examples**

The following examples are given in order to assist the skilled artisan to better understand the present invention by way of exemplification. The examples are not intended to limit the scope of protection conferred by the claims. The features, properties and advantages exemplified for the compounds and uses defined in the examples may be assigned to other compounds and uses not specifically described and/or defined in the examples, but falling under the scope of what is defined in the claims.

**Example 1 [for technical information only]: Rat orthotopic glioblastoma model radiotherapy, Cilengitide (= cyclo-(Arg-Gly-Asp-DPhe-NMe-Val)) scheduling experiments**

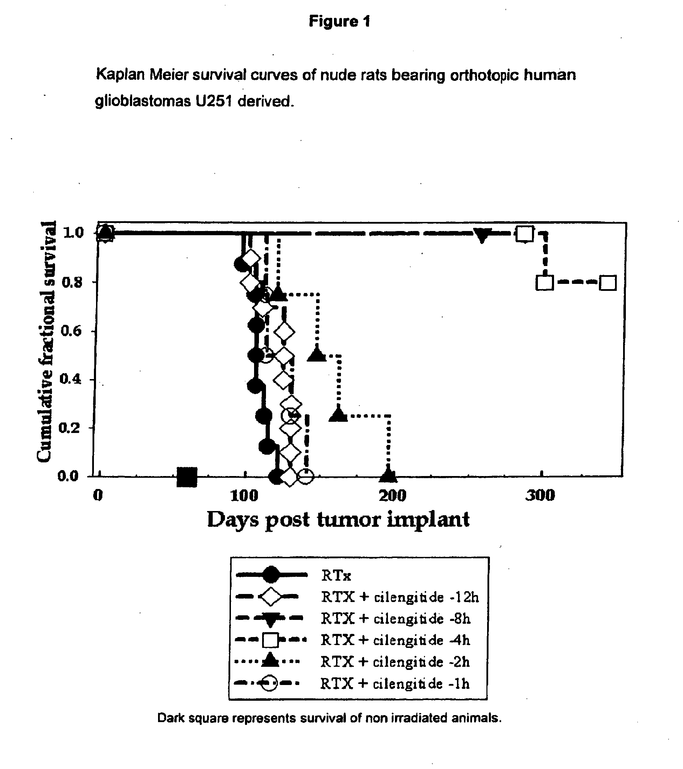

NIH rnu nude rats are anaesthetized, restrained, and injected intracerebrally 1 mm retro orbitally, 3mm to the right of the bregma and at a depth of 2.5 mm with 5x10E5 U251 human glioblastoma cells suspended in 10 ul of culture medium, using a #2701 Hamilton syringe fitted with a 26 gauge needle, essentially as previously described (Engebraaten et al., 1999). After 14 days, cilengitide (4 mg/kg) is given as an intraperitoneal bolus in PBS, at various time (8h, 4h, 2h, 1 h) prior to a single treatment with single, collimated, dorsal-ventral beam of 6 MV x-rays, so that 95-100% of the central axis dose of 25 Gy hit the tumor volume (Kim et al., 1999). Each of the 7 subsequent days the animals also received an identical i.p. bolus of cilengitide. The animals are maintained under ad libitum food and drink until they become moribund, or are sampled for tissue analysis (in the t-4 and t-8 h groups, where the animals are healthy past 230 days post tumor injection). A Kaplan-Meier survival curve is calculated and plotted (Fig.1) from the raw data (Table 1). All animals in the RT monotherapy group died by 120 d.

Reference List:

Engebraaten,O., Hjortland,G.O., Hirschberg,H., and Fodstad,O. (1999). Growth of precultured human glioma specimens in nude rat brain. J. Neurosurg. 90, 125-132.
Kim,J.H., Khil,M.S., Kolozsvary,A., Gutierrez,J.A., and Brown,S.L. (1999). Fractionated radiosurgery for 9L gliosarcoma in the rat brain. Int. J. Radiat. Oncol. Biol. Phys. 45, 1035-1040.
The Results are given in Table 1 below and Fig. 1:

**Table 1**

| 400,000 U251n Cells Inj. | | | | EMD Survival Study | | | Days Post |
|---|---|---|---|---|---|---|---|
| Group | Time Pre-Irradiation | Animal # | Trtmnt | Date of Injection | Date of Radiation | Date of Termination | Implant |
| 89 | 8 hours | G89-1 | Rt | 03.03.2005 | 17.03.2005 | (Sick) 6/7/2005 | 96 |
| 89 | 8 hours | G89-2 | Rt | 03.03.2005 | 17.03.2005 | (Sick) 6/17/2005 | 106 |
| 89 | 8 hours | G89-3 | Rt + EMD | 03.03.2005 | 17.03.2005 | (Healthy) 11/15/2005 | 257 |
| 89 | 8 hours | G89-4 | Rt + EMD | 03.03.2005 | 17.03.2005 | (Healthy) 11/15/2005 | 257 |
| 89 | 8 hours | G89-5 | Rt + EMD | 03.03.2005 | 17.03.2005 | (Alive) 12/15/2005 | 287 |
| 89 | 8 hours | G89-6 | Rt + EMD | 03.03.2005 | 17.03.2005 | (Alive) 12/15/2005 | 287 |
| 90 | 4 hours | G90-1 | Rt | 05.04.2005 | 19.04.2005 | (Sick) 7/20/2005 | 106 |
| 90 | 4 hours | G90-2 | Rt | 05.04.2005 | 19.04.2005 | (Sick) 7/29/2005 | 115 |
| 90 | 4 hours | G90-3 | Rt + EMD | 05.04.2005 | 19.04.2005 | (Healthy) 11/29/2005 | 238 |
| 90 | 4 hours | G90-4 | Rt + EMD | 05.04.2005 | 19.04.2005 | (Healthy) 11/29/2005 | 238 |

(continued)

| Group | Time Pre-Irradiation | Animal # | Trtmnt | Date of Injection | Date of Radiation | Date of Termination | Implant |
|---|---|---|---|---|---|---|---|
| 90 | 4 hours | G90-5 | Rt + EMD | 05.04.2005 | 19.04.2005 | (Alive) 12/15/2005 | 254 |
| 90 | 4 hours | G90-6 | Rt + EMD | 05.04.2005 | 19.04.2005 | (Alive) 12/15/2005 | 254 |
| | | | | | | | |
| 91 | 2 hours | G91-1 | Rt | 12.04.2005 | 26.04.2005 | (Sick) 7/26/2005 | 105 |
| 91 | 2 hours | G91-2 | Rt | 12.04.2005 | 26.04.2005 | (Sick) 8/12/2005 | 122 |
| 91 | 2 hours | G91-3 | Rt + EMD | 12.04.2005 | 26.04.2005 | (Sick) 8/10/2005 | 120 |
| 91 | 2 hours | G91-4 | Rt + EMD | 12.04.2005 | 26.04.2005 | (Sick) 9/6/2005 | 147 |
| 91 | 2 hours | G91-5 | Rt + EMD | 12.04.2005 | 26.04.2005 | (Sick) 9/21/2005 | 162 |
| 91 | 2 hours | G91-6 | Rt + EMD | 12.04.2005 | 26.04.2005 | (Sick) 10/25/2005 | 196 |
| | | | | | | | |
| 92 | 1 hour | G92-1 | Rt | 12.05.2005 | 26.05.2005 | (Sick) 8/26/2005 | 106 |
| 92 | 1 hour | G92-2 | Rt | 12.05.2005 | 26.05.2005 | (Sick) 9/1/2005 | 112 |
| 92 | 1 hour | G92-3 | Rt + EMD | 12.05.2005 | 26.05.2005 | (Sick) 9/1/2005 | 112 |
| 92 | 1 hour | G92-4 | Rt + EMD | 12.05.2005 | 26.05.2005 | (Sick) 9/2/2005 | 113 |
| 92 | 1 hour | G92-5 | Rt + EMD | 12.05.2005 | 26.05.2005 | (Sick) 9/19/2005 | 130 |
| 92 | 1 hour | G92-6 | Rt + EMD | 12.05.2005 | 26.05.2005 | (Sick) 9/30/2005 | 141 |

Sick = moribund and removed from study
Healthy = indicates sampled for tissue at date shown, but alive at this point Alive = surviving at time point shown.
Time pre-irradiation = when cilengitide 4 mg/kg is given.
Rt = radiotherapy 25 Gy
EMD = cilengitide bolus 4 mg/kg

American date convention in date of termination column, European in date of radiation column

**Example 2 [for technical information only]: Phase IIa Trial of Cilengitide ((= cyclo-(Arg-Gly-Asp-DPhe-NMe-Val))) Single Agent Therapy in Patients with Recurrent Glioblastoma**

Background: The present phase IIa study was designed to evaluate the safety, toxicity, and clinical activity of the cyclic RGD pentapeptide cilengitide((= cyclo-(Arg-Gly-Asp-DPhe-NMe-Val)), an inhibitor of integrins $av\beta3$ and $av\beta5$, as a single agent at doses of 500 and 2000 mg in patients (pts) with recurrent glioblastoma (GBM).
**Methods:** In this multicenter, open-label, randomized, uncontrolled study, pts with GBM and measurable disease that had relapsed after previous therapy with temozolomide and radiotherapy were randomized to receive cilengitide at doses of either 500 mg or 2000 mg i.v., 2x/week, until progression. Histopathology diagnosis and MRI imaging were subject to independent blinded review. The primary endpoint was Progression Free Survival (PFS) at 6 months (mths). Secondary endpoints included response, survival, time to disease progression, safety, tolerability and pharmacokinetics.

**Results:** Actual accrual; 81 pts (median Kamofsky Performance Status 80%; median age 57 yrs) at 15 sites. 41 pts received 500 mg and 40 pts to receive 2000 mg of i.v. cilengitide 2x/week. No obvious imbalance in prognostic factors was observed. Median infusions;16 [range, 4-179]. Treatment related NCI CTC grade 3 adverse events (AEs) included elevated liver enzymes (at 500 mg), arthralgia/ myalgia (at 500 mg), and weight increase/ edema (at 2000 mg) in 1 patient, respectively. No grade 4 therapy related AEs were reported by the investigators. One CTC grade 2 cerebral hemorrhage was reported, possibly related either to the drug or to the disease. The PFS rate at 6 mths was 16.1% (n= 13/81 pts). 10 of these pts (12.3 %, n=4 with 500 mg, n=6 with 2000 mg) received 12 or more cycles of therapy (1 cycle=4 weeks). Six pts (7.4%) were still progression-free and on treatment at the time when this abstract was issued. In the 500 mg arm, median Overall Survival (mOS) was 6.5 mths [95% CI: 5.2-9.3 mths], 12 mth overall survival (OS) rate was 24.4%. In the 2000 mg arm, mOS was 9.9 mths [95% CI, 6.3-15.7 mths], 12 mth OS rate was 37.5%. Although not statistically significant, there was a trend towards better tumor control in pts receiving 2000 mg 2x/week.

**Conclusion:** Cilengitide was tolerated well in single agent therapy at two dose levels. Cilengitide demonstrated advantagous single agent activity in recurrent glioblastoma, with long term disease stabilisation in a subset of pts.

**Example 3 [for technical information only]: Phase I/IIa Trial of Cilengitide (= cyclo-(Arg-Gly-Asp-DPhe-NMe-Val)) and Temozolomide with Concomitant Radiotherapy, Followed by Temozolomide and Cilengitide Maintenance Therapy in Patients With Newly Diagnosed Glioblastoma (GBM).**

**Purpose:** To evaluate safety, toxicity, and efficacy of the combination of the cyclic RGD pentapeptide Cilengitide (= cyclo-(Arg-Gly-Asp-DPhe-NMe-Val)), an inhibitor of integrins $av\beta3$ and $av\beta5$, in addition to standard temozolomide (TMZ) and radiotherapy (RT).

**Patients and methods:** Fifty-two pts (PS 0-1: 92%, 2: 8%; median age 57 yrs) after biopsy (n= 9/17%) or tumor resection (n=43/83%) were treated with standard TMZ/RT (Stupp et al. NEJM 2005). In addition Cilengitide (500 mg i.v., 2x/week) was started one week before TMZ/RT and given throughout for the duration of chemotherapy or until progression. Primary endpoint was progression free survival rate at 6 months (target: 65%). Patients were followed with MRI every 2 months. Histopathologic diagnosis and MRI imaging were independently reviewed, MGMT promotor methylation status was assessed in 45 (86.5%) pts.

**Results:** Forty-six pts (92%) completed RT, $\geq$ 90% of concomitant TMZ was received by 42 pts and cilengitide by 45 pts. 20 pts (3 ongoing) completed 6 cycles of maintenance TMZ and cilengitide. Observed haematological grade 3 and 4 toxicity was: lymphopenia (28/52, 53.8%), thrombocytopenia (7/52 pt. 13.4%) and neutropenia (5/52, 9.6%). Treatment related non-hematologic grade 3 toxicities were reported for n=3/52 (5.7%) patients: constitutional symptoms (asthenia, fatigue, anorexia, n=3); elevated liver function tests (n=1), deep venous thrombosis and pulmonary embolism (n=1). One patient with a history of sigmoid diverticulosis experienced sigmoid perforation (grade 2). In total, 34/52 (65.4% [95% CI, 50.9-78.0%]) of the patients were progression free at 6 months. Pts with $O^6$-Methylguanine-DNA methyltransferase (MGMT) gene-promotor methylation in the tumor were more likely to reach 6 months PFS endpoint. In total, 34/52 (65.4% [95% CI, 50.9-78.0%]) of the pts were progression free at 6 months. A major contribution to the overall result was provided by a subgroup of patients (23/52 subjects, with methylated MGMT promoter, silencing the DNA repair enzyme MGMT), which showed a strong increase of the PFS-6 rate compared to historical control (91% vs. 69%). The other major subgroup (22/52, unmethylated MGMT promotor) showed a less relevant difference to the historical control (40.9% vs. 40%), which is likely to be significantly improved by a higher dosing of Cilengitide in comparison to the subgroup with methylated MGMT promoter. Overall the study reached its primary endpoint (PFS-6 = 65.4%)

Conclusion: The study reached its primary endpoint. The combination of the integrin inhibitor RGD peptide Cilengitide and TMZ/RT was well tolerated, PFS at 6 months is very advantagous. MGMT gene promotor methylation provides for even better prognosis.

**Example 4: Proliferation Assays**

**1 Materials and methods**

**1.1 Test system (biological materials/animals)**

Carcinoma cell lines are grown in the following media:

A549 -DMEM containing 10% FCS (heat -inactivated) plus 2 mM glutamine, HUVEC-DMEM containing 10% FCS (heat -inactivated) plus 2 mMglutamine and 1 mM sodium pyruvate.

All media contains 100 units/ml penicillin

and 100 ug/ml streptomycin. Cells are passaged at confluence by washing once in

cation -free PBS followed by a 3 minute incubation in trypsin (0.5 ug/ml)/EDTA (0.2 ug/ml) solution in PBS at 37°C. Cells are recovered in medium, centrifuged and taken up in medium and counted.

## 1.2 Chemicals and solutions

All cell culture reagents are from GIBCO/InVitrogen with the exception of foetal calf

serum which is purchased from BioWhittaker. Dulbecco's PBS with and without cations is from GIBCO/Invitrogen Alamar Blue is from Serotech. Paclitaxel, vinblastin, and oxaliplatin are from Sigma. Cisplatin is purchased from Fluka. Gemcitabine is purchased from LGC Promochem, Heidelberg. Gefitnib from AstraZeneca and imatinib from Novartis are commercially available.

Cilengitide by Merck KGaA. Bovine serum albimun is from VWR. The extracellular matrix components vitronectin and fibronectin are purified from human serum in house according to SOP 6456; fibrinogen according to SOP 6460. Rat tail collagen I is from Serva. Antibodies for FACS analysis: 17E6, 20H9, LM609, P1F6, 11D1, P4C10 . MAb P1D6 are are commercially available, e.g. purchased from Chemicon. Goat anti-mouse IgG FITC conjugate is from Becton Dickson.

## 1.3 Methods

FACS Analysis

Cells are harvested with trypsin as described above. The required number of cells is taken up in PBS containing 0.9 mM CaCl2 and 0.5 mM MgCl2+ 0.5% BSA (= FACS Buffer)and aliquoted 1 x10e6 /tube. After centrifugation at 800 x g for 4 minutes, the cells are incubated 60 minutes on ice with anti-integrin antibodies at 10 ug/ml in FACS Buffer, 100 ul/tube. After washing to remove unbound antibody, the cells are incubated with goat anti-mouse FITC diluted 1:25 in FACS Buffer. Cells are incubated 30 minutes on ice, washed to remove unbound antibody and a final cell suspension is made in FACS Buffer 500 ul/tube. Cells are analyzed on a FACScan and the mean intensity fluorescence (MIF) is normalized to the MIF of the negative control (no primary antibody).

Attachment Assay

Attachment to extracellular matrix proteins is performed as follows:

Briefly, 2.5 x 10e4 cells/well in RPMI containing 0.5% BSA and 25 mM Hepes pH 7.4 attached to non-tissue culture treated 96-well plates coated with serially diluted vitronectin, fibronectin, fibrinogen and collagen I for 60 minutes at 37°C. After washing to remove unbound cells the relative cell number is determined by incubation with hexosaminidase substrate. The colormetric reaction is read at 405 nm in a Genios plate reader (SLT).

Proliferation assay Non-tissue cultures treated 96 well plates are coated using 100 ul/well of a 2 ug/ml vitronectin solution in PBS incubated overnight at 4 °C. Cells are plated at 5x10e3 in 100 ul cell culture medium (as described above for each cell line). After 3 hours at 37°C serially diluted chemotherapeutic agents are added alone or in the presence of a constant EC50 concentration of alpha V integrin blocker at two-fold concentration in 100ul/well in cell culture medium. Plates are incubated for 72 hours, after which relative cell number is determined by the addition of 20ul/well Alamar Blue (Resazurin) (Nakayama etal. 1997). After 4 hours of incubation at 37°C relative fluorescent intensity is read in a Genios plate reader (SLT) at 535/590nm (excitation/emission).

## 1.4 Experimental design

Points are run in triplicate. Reagent blanks, containing media plus colormetric reagent without cells, are run on each plate. Blank values are subtracted from test values and are routinely 5-10 % of uninhibited control values.

In FACS analysis 15,000 events analyzed. Single cells are gated out from debris and aggregates and the live cells based on staining with propidium iodide. Markers are set on a negative control population stained with goat anti-mouse FITC alone (no primary antibody). Cells that fell to the right of the marker (higher intensity fluorescence) are considered positively stained.

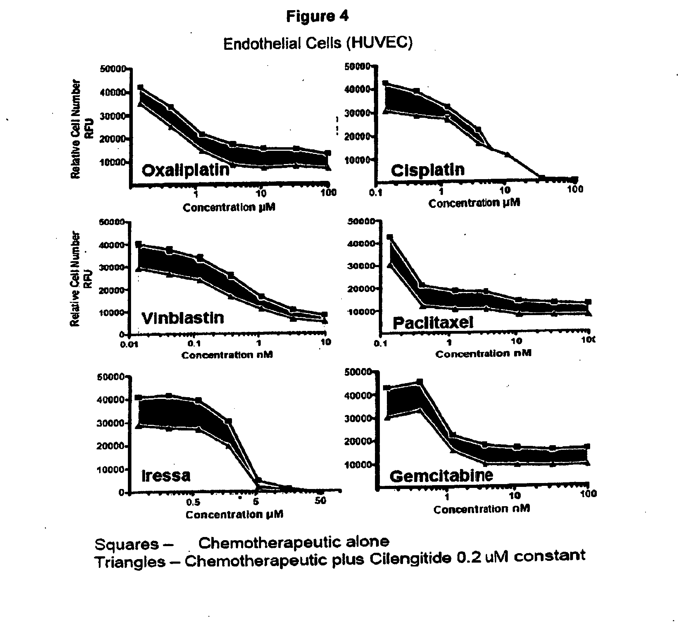

The results are shown in Figure 4 and Figure 5, respectively.

Concentration on X-Axis) refers to the respective compound (oxaliplatin, cisplatin, vinblastine, paclitaxel, Iressa (gefitinib) or gemcitabine).

Y-Axis refers to the relative cell number.

Cilengitide concentration is constant (6nM for NSCLC (A549) and 0.2 nM for Endothelial Cells (HUVEC), respectively).

## Claims

1. Specific integrin ligand, comprising cyclo-(Arg-Gly-Asp-OPhe-NMe-Val), the pharmaceutically acceptable solvates and/or salts thereof, for use in the treatment of colon and/or rectal cancer in combination with

   a) one or more alkylating chemotherapeutic agents, selected from the group consisting of the platinum containing compounds cisplatin, carboplatin and oxaliplatin, and
   b) one or more further chemotherapeutic agents other than the at least one specific integrin ligand and the one or more alkylating chemotherapeutic agents, selected from the group consisting of:

   i) EGFR inhibitors, selected from the group consisting of cetuximab, panitumumab, zalutumumab, nimotuzumab and matuzumab and/or the group consisting of gefitinib, erlotinib and lapatinib,
   ii) cytostatic alkaloids, selected from the group consisting of etoposide, vinblastine and teniposide, the group consisting of vinorelbine, vincristine and vindesine, the group consisting of docetaxel and paclitaxel, and/or the group consisting of irinotecan and topotecan,
   iii) cytostatic antibiotics, selected from the group consisting of doxorubicin, idarubicin, daunorubicin, epirubicin and valrubicin, and
   iv) antimetabolites, selected from the group consisting of 5-fluorouracil, capecitabine, cytosinarabinosid and difluorodesoxycytidin and/or the group consisting of pemetrexed, methotrexat and raltitrexed,

   and pharmaceutically acceptable salts and/or solvates thereof.

2. Specific integrin ligand for use according to claim 1, wherein the cyclo-(Arg-Gly-Asp-DPhe-NMe-Val), the pharmaceutically acceptable solvates and/or salts thereof is to be administered to a patient in an amount of 250 mg to 12500 mg per week.

3. Specific integrin ligand for use according to one of the preceding claims, wherein the EGFR inhibitor is selected from the group consisting of cetuximab, panitumumab, zalutumumab, nimotuzumab and matuzumab and/or the group consisting of gefitinib, erlotinib and lapatinib, the cytostatic alkaloid is selected from the group consisting of vinorelbine and vincristine and/or the group consisting of paclitaxel and docetaxel, and the antimetabolite is selected from the group consisting of gemcitabine and pemetrexed.

4. Specific integrin ligand for use according to one of the preceding claims, wherein

   i) the one or more alkylating chemotherapeutic agents (a) are selected from the group consisting of the platinum containing chemotherapeutic agents cisplatin, carboplatin and oxaliplatin,
   ii) the one or more further chemotherapeutic agents other than the at least one specific integrin ligand and the one or more alkylating chemotherapeutic agents (b) are selected from the group consisting of anti-EGFR biologicals cetuximab, panitumumab, zalutumumab, nimotuzumab and matuzumab and the vinca alkaloids vinorelbine and vincristine.

5. Specific integrin ligand for use according to one of the preceding claims, wherein the cyclo-(Arg-Gly-Asp-DPhe-NMe-Val), the pharmaceutically acceptable solvates and/or salts thereof, is to be combined with

   i) the one or more alkylating chemotherapeutic agents (a) selected from the group consisting of the platinum containing chemotherapeutic agents cisplatin, carboplatin and oxaliplatin, and
   ii) the one or more further chemotherapeutic agents other than the at least one specific integrin ligand and the one or more alkylating chemotherapeutic agents (b) comprise:

   α) one or more anti-EGFR biologicals, selected from the group consisting of cetuximab, panitumumab, zalutumumab, nimotuzumab and matuzumab, and
   β) one or more compounds, selected from the group consisting of the cytostatic alkaloids vinorelbine and vincristine.

6. Specific integrin ligand for use according to one of the preceding claims, wherein the cyclo-(Arg-Gly-Asp-DPhe-NMe-Val), the pharmaceutically acceptable solvates and/or salts thereof is to be administered to a patient in an amount of 400 mg to 6000 mg per week.

7. Use according to one of the preceding claims, wherein the cyclo-(Arg-Gly-Asp-DPhe-NMe-Val), the pharmaceutically acceptable solvates and/or salts thereof is to be administered to a patient in an amount of 1500 mg to 5000 mg per week.

8. Specific integrin ligand for use according to one of the preceding claims, wherein the cyclo-(Arg-Gly-Asp-DPhe-NMe-Val), the pharmaceutically acceptable solvates and/or salts thereof is to be administered to a patient in a once weekly to three times weekly administration scheme consisting of about 500 mg or about 2000 mg per administration.

9. Specific integrin ligand for use according to one of the preceding claims, wherein

ii) the one or more alkylating chemotherapeutic agents (a) selected from the group consisting of the platinum containing chemotherapeutic agents cisplatin, carboplatin and oxaliplatin are to be administered to the patient in an amount of 100 to 1000 mg in one or more portions within a time period of 2 to 4 weeks, and
iiii) the one or more further chemotherapeutic agents other than the at least one specific integrin ligand and the one or more alkylating chemotherapeutic agents (b) comprise:

α) one or more anti-EGFR biologicals, selected from the group consisting of cetuximab, panitumumab, zalutumumab, nimotuzumab and matuzumab, administered to the patient in an amount of 200 to 2000 mg in one or more portions within a time period of 2 to 4 weeks, and optionally
β) one or more compounds, selected from the group consisting of the cytostatic alkaloids vinorelbine and vincristine, the group consisting of paclitaxel and docetaxel, and/or the group consisting of the antimetabolites gemcitabine and pemetrexed, administered to the patient in an amount of 25 to 6000 mg in one or more portions within a time period of 2 to 4 weeks.

10. Specific integrin ligand for use according to one of the preceding claims, wherein

i) the platinum containing chemotherapeutic agent is selected from the group consisting of cisplatin, carboplatin and oxaliplatin,
ii) the antimetabolite is selected from the group consisting of the antifolates Methotrexate, Raltitrexed and Pemetrexed and pharmaceutically acceptable salts and/or solvates thereof, and the pyrimidine antagonists 5-Fluorouracil, Capecitabine, Cytosinarabinoside and Difluorodesoxycytidine and pharmaceutically acceptable salts and/or solvates thereof, and
iii) the cytostatic alkaloid is selected from the group consisting of the vinca alkaloids vinblastine, Vincristine, Vindesine and Vinorelbine and pharmaceutically acceptable salts and/or solvates thereof, and the taxanes Docetaxel and Paclitaxel and pharmaceutically acceptable salts and/or solvates thereof, and
iv) the EGFR inhibitor is selected from the group consisting of the anti-EGFR biologicals cetuximab, panitumumab, zalutumumab, nimotuzumab and matuzumab and pharmaceutically acceptable salts and/or solvates thereof and the chemically derived compounds gefitinib, erlotinib and lapatinib and pharmaceutically acceptable salts and/or solvates thereof.

11. Specific integrin ligand for use according to one of the preceding claims, wherein the EGFR inhibitor is selected from the group consisting of cetuximab, panitumumab, zalutumumab, nimotuzumab and matuzumab and/or the group consisting of gefitinib, erlotinib and lapatinib, the cytostatic alkaloid is selected from the group consisting of vinorelbine and vincristine and/or the group consisting of paclitaxel and docetaxel, and the antimetabolite is selected from the group consisting of 5-fluorouracil and pemetrexed.

12. Specific integrin ligand for use according to one of the preceding claims, wherein

i) the one or more alkylating chemotherapeutic agents (a) are selected from the group consisting of the platinum containing chemotherapeutic agents cisplatin, carboplatin and oxaliplatin,
ii) the one or more further chemotherapeutic agents other than the at least one specific integrin ligand and the one or more alkylating chemotherapeutic agents (b) are selected from the group consisting of anti-EGFR biologicals cetuximab, panitumumab, zalutumumab, nimotuzumab and matuzumab, the antimetabolites 5-fluorouracil and pemetrexed, and the taxanes docetaxel and paclitaxel.

**13.** Specific integrin ligand for use according to one of the preceding claims, wherein
the cyclo-(Arg-Gly-Asp-DPhe-NMe-Val), the pharmaceutically acceptable solvates and/or salts thereof, is to be combined with

i) the one or more alkylating chemotherapeutic agents (a) selected from the group consisting of the platinum containing chemotherapeutic agents cisplatin, carboplatin and oxaliplatin, and
ii) the one or more further chemotherapeutic agents other than the at least one specific integrin ligand and the one or more alkylating chemotherapeutic agents (b) comprise:

α) one or more anti-EGFR biologicals, selected from the group consisting of cetuximab, panitumumab, zalutumumab, nimotuzumab and matuzumab, and
β) one or more compounds, selected from the group consisting of the antimetabolites 5-fluorouracil and pemetrexed, and/or the group consisting of the taxanes docetaxel and paclitaxel.

**14.** Specific integrin ligand for use according to one of the preceding claims, wherein the cyclo-(Arg-Gly-Asp-DPhe-NMe-Val), the pharmaceutically acceptable solvates and/or salts thereof is to be administered to a patient in an amount of 400 mg to 6000 mg per week.

**15.** Specific integrin ligand for use according to one of the preceding claims, wherein the cyclo-(Arg-Gly-Asp-DPhe-NMe-Val), the pharmaceutically acceptable solvates and/or salts thereof is to be administered to a patient in an amount of 1500 mg to 5000 mg per week.

**16.** Specific integrin ligand for use according to one of the preceding claims, wherein the cyclo-(Arg-Gly-Asp-DPhe-NMe-Val), the pharmaceutically acceptable solvates and/or salts thereof is to be administered to a patient in a once weekly to five times weekly administration scheme consisting of about 500 mg or in a once weekly to three times weekly administration scheme consisting of about 2000 mg per administration.

**17.** Specific integrin ligand for use according to one of the preceding claims, wherein

ii) the one or more alkylating chemotherapeutic agents (a) selected from the group consisting of the platinum containing chemotherapeutic agents cisplatin, carboplatin and oxaliplatin are to be administered to the patient in an amount of 100 to 1000 mg in one or more portions within a time period of 2 to 4 weeks, and
iiii) the one or more further chemotherapeutic agents other than the at least one specific integrin ligand and the one or more alkylating chemotherapeutic agents (b) comprise:

α) one or more anti-EGFR biologicals, selected from the group consisting of cetuximab, panitumumab, zalutumumab, nimotuzumab and matuzumab, administered to the patient in an amount of 200 to 2000 mg in one or more portions within a time period of 2 to 4 weeks, and optionally
β) one or more compounds, selected from the group consisting of the antimetabolites 5-fluorouracil and pemetrexed and/or the group consisting of the taxanes paclitaxel and docetaxel, administered to the patient in an amount of 150 to 7500 mg in one or more portions within a time period of 2 to 4 weeks.

**18.** Specific integrin ligand for use according to one of the claims 9 or 17, wherein the weekly administration scheme is to be applied 1 to 52 times substantially without a pause.

**19.** Specific integrin ligand for use according to one of the claims 10 or 18, wherein said administration to the patient within a time period of 2 to 4 weeks is to be repeated 1 to 12 times substantially without a pause.

**20.** Specific integrin ligand for use according to one of the preceding claims, wherein

a) the weekly administration scheme regarding the specific integrin ligand and
b) the administration to the patient within a time period of 2 to 4 weeks regarding

i) the one or more alkylating chemotherapeutic agents and/or
ii) the one or more further chemotherapeutic agents other than the at least one specific integrin ligand and the one or more alkylating chemotherapeutic agents,

run in parallel for one or more weeks.

**21.** Specific integrin ligand comprising cyclo-(Arg-Gly-Asp-OPhe-NMeVal) and/or the pharmaceutically acceptable salts thereof for use in the treatment of colon and/or rectal cancer, wherein the specific integrin ligand is to be used in combination with

a) one or more alkylating chemotherapeutic agents according to one of the preceding claims, and
b) one or more further chemotherapeutic agents according to one of the preceding claims other than the at least one specific integrin ligand and the one or more alkylating chemotherapeutic agents;

and radiotherapy,
wherein the cyclo-(Arg-Gly-Asp-DPhe-NMeVal) and/or the pharmaceutically acceptable salts thereof is to be administered to a patient in an amount of 800 mg to 7000 mg per week.

**Patentansprüche**

**1.** Spezifischer Integrinligand, enthaltend Cyclo-(Arg-Gly-Asp-DPhe-NMe-Val), die pharmazeutisch unbedenklichen Solvate und/oder Salze davon, zur Verwendung bei der Behandlung von Dickdarm-und/oder Rektumkrebs, in Kombination mit

a) einem oder mehreren alkylierenden Chemotherapeutika, ausgewählt aus der Gruppe bestehend aus den platinhaltigen Verbindungen Cisplatin, Carboplatin und Oxaliplatin, und
b) einem oder mehreren weiteren Chemotherapeutika, außer dem mindestens einen spezifischen Integrinliganden und dem einen oder den mehreren alkylierenden Chemotherapeutika, ausgewählt aus der Gruppe bestehend aus:

i) EGFR-Inhibitoren, ausgewählt aus der Gruppe bestehend aus Cetuximab, Panitumumab, Zalutumumab, Nimotuzumab und Matuzumab und/oder der Gruppe bestehend aus Gefitinib, Erlotinib und Lapatinib,
ii) cytostatischen Alkaloiden, ausgewählt aus der Gruppe bestehend aus Etoposid, Vinblastin und Teniposid, der Gruppe bestehend aus Vinorelbin, Vincristin und Vindesin, der Gruppe bestehend aus Docetaxel und Paclitaxel und/oder der Gruppe bestehend aus Irinotecan und Topotecan,
iii) cytostatischen Antibiotika, ausgewählt aus der Gruppe bestehend aus Doxorubicin, Idarubicin, Daunorubicin, Epirubicin und Valrubicin, und
iv) Antimetaboliten, ausgewählt aus der Gruppe bestehend aus 5-Fluoruracil, Capecitabin, Cytosinarabinosid und Difluordesoxycytidin und/oder der Gruppe bestehend aus Pemetrexed, Methotrexat und Raltitrexed

und pharmazeutisch unbedenklichen Salzen und/oder Solvaten davon.

**2.** Spezifischer Integrinligand zur Verwendung nach Anspruch 1, wobei das Cyclo-(Arg-Gly-Asp-DPhe-NMe-Val), die pharmazeutisch unbedenklichen Solvate und/oder Salze davon, einem Patient in einer Menge von 250 mg bis 12500 mg pro Woche verabreicht werden soll.

**3.** Spezifischer Integrinligand zur Verwendung nach einem der vorhergehenden Ansprüche, wobei der EGFR-Inhibitor ausgewählt ist aus der Gruppe bestehend aus Cetuximab, Panitumumab, Zalutumumab, Nimotuzumab und Matuzumab und/oder aus der Gruppe bestehend aus Gefitinib, Erlotinib und Lapatinib, wobei das cytostatische Alkaloid ausgewählt ist aus der Gruppe bestehend aus Vinorelbin und Vincristin und/oder der Gruppe bestehend aus Paclitaxel und Docetaxel und der Antimetabolit ausgewählt ist aus der Gruppe bestehend aus Gemcitabin und Pemetrexed.

**4.** Spezifischer Integrinligand zur Verwendung nach einem der vorhergehenden Ansprüche, wobei

i) das eine oder die mehreren alkylierende(n) Chemotherapeutika (a) ausgewählt sind aus der Gruppe bestehend aus den platinhaltigen Chemotherapeutika Cisplatin, Carboplatin und Oxaliplatin,
ii) das eine oder die mehreren weitere(n) Chemotherapeutika, außer dem mindestens einen spezifischen Integrinliganden und dem einen oder den mehreren alkylierenden Chemotherapeutika (b) ausgewählt sind aus der Gruppe bestehend aus den biologischen anti-EGFR-Substanzen Cetuximab, Panitumumab, Zalutumumab, Nimotuzumab und Matuzumab und den Vincaalkaloiden Vinorelbin und Vincristin.

5. Spezifischer Integrinligand zur Verwendung nach einem der vorhergehenden Ansprüche, wobei das Cyclo-(Arg-Gly-Asp-DPhe-NMe-Val), die pharmazeutisch unbedenklichen Solvate und/oder Salze davon, kombiniert werden soll mit

   i) dem einen oder den mehreren alkylierenden Chemotherapeutika (a) ausgewählt aus der Gruppe bestehend aus den platinhaltigen Chemotherapeutika Cisplatin, Carboplatin und Oxaliplatin, und
   ii) dem einen oder den mehreren Chemotherapeutika, außer dem mindestens einen spezifischen Integrinliganden und dem einen oder den mehreren alkylierenden Chemotherapeutika (b), die enthalten:

   α) eine oder mehrere biologische anti-EGFR-Substanzen, ausgewählt aus der Gruppe bestehend aus Cetuximab, Panitumumab, Zalutumumab, Nimotuzumab und Matuzumab, und
   β) eine oder mehrere Verbindungen, ausgewählt aus der Gruppe bestehend aus den cytostatischen Alkaloiden Vinorelbin und Vincristin.

6. Spezifischer Integrinligand zur Verwendung nach einem der vorhergehenden Ansprüche, wobei das Cyclo-(Arg-Gly-Asp-DPhe-NMe-Val), die pharmazeutisch unbedenklichen Solvate und/oder Salze davon, einem Patienten in einer Menge von 400 mg bis 6000 mg pro Woche verabreicht werden soll.

7. Zur Verwendung nach einem der vorhergehenden Ansprüche, wobei das Cyclo-(Arg-Gly-Asp-DPhe-NMe-Val), die pharmazeutisch unbedenklichen Solvate und/oder Salze davon, einem Patienten in einer Menge von 1500 mg bis 5000 mg pro Woche verabreicht werden soll.

8. Spezifischer Integrinligand zur Verwendung nach einem der vorhergehenden Ansprüche, wobei das Cyclo-(Arg-Gly-Asp-DPhe-NMe-Val), die pharmazeutisch unbedenklichen Solvate und/oder Salze davon, einem Patienten in einem einmal wöchentlichen bis dreimal wöchentlichen Verabreichungsschema, bestehend aus etwa 500 mg oder etwa 2000 mg pro Verabreichung, verabreicht werden soll.

9. Spezifischer Integrinligand zur Verwendung nach einem der vorhergehenden Ansprüche, wobei

   ii) das eine oder die mehreren alkylierende(n) Chemotherapeutika (a), ausgewählt aus der Gruppe bestehend aus den platinhaltigen Chemotherapeutika Cisplatin, Carboplatin und Oxaliplatin, dem Patienten in einer Menge von 100 bis 1000 mg in einer oder mehreren Portionen innerhalb eines Zeitraums von 2 bis 4 Wochen verabreicht werden sollen, und
   iiii) das eine oder die mehreren weitere(n) Chemotherapeutika, außer dem mindestens einen spezifischen Integrinliganden und dem einen oder den mehreren alkylierenden Chemotherapeutika (b) enthalten:

   α) eine oder mehrere biologische anti-EGFR-Substanzen, ausgewählt aus der Gruppe bestehend aus Cetuximab, Panitumumab, Zalutumumab, Nimotuzumab und Matuzumab, dem Patienten verabreicht in einer Menge von 200 bis 2000 mg in einer oder mehreren Portionen innerhalb eines Zeitraums von 2 bis 4 Wochen, und gegebenenfalls
   β) eine oder mehrere Verbindungen, ausgewählt aus der Gruppe bestehend aus den cytostatischen Alkaloiden Vinorelbin und Vincristin, der Gruppe bestehend aus Paclitaxel und Docetaxel und/oder der Gruppe bestehend aus den Antimetaboliten Gemcitabin und Pemetrexed, dem Patienten verabreicht in einer Menge von 25 bis 6000 mg in einer oder mehreren Portionen innerhalb eines Zeitraums von 2 bis 4 Wochen.

10. Spezifischer Integrinligand zur Verwendung nach einem der vorhergehenden Ansprüche, wobei

   i) das platinhaltige Chemotherapeutikum ausgewählt ist aus der Gruppe bestehend aus Cisplatin, Carboplatin und Oxaliplatin,
   ii) der Antimetabolit ausgewählt ist aus der Gruppe bestehend aus den Antifolaten Methotrexat, Raltitrexed und Pemetrexed und pharmazeutisch unbedenklichen Salzen und/oder Solvaten davon und den Pyrimidin-Antagonisten 5-Fluoruracil, Capecitabin, Cytosinarabinosid und Difluordesoxycytidin und pharmazeutisch unbedenklichen Salzen und/oder Solvaten davon, und
   iii) das cytostatische Alkaloid ausgewählt ist aus der Gruppe bestehend aus den Vincaalkaloiden Vinblastin, Vincristin, Vindesin und Vinorelbin und pharmazeutisch unbedenklichen Salzen und/oder Solvaten davon und den Taxanen Docetaxel und Paclitaxel und pharmazeutisch unbedenklichen Salzen und/oder Solvaten davon, und
   iv) der EGFR-Inhibitor ausgewählt ist aus der Gruppe bestehend aus den biologischen anti-EGFR-Substanzen Cetuximab, Panitumumab, Zalutumumab, Nimotuzumab und Matuzumab und pharmazeutisch unbedenklichen Salzen und/oder Solvaten davon und den chemisch abgeleiteten Verbindungen Gefitinib, Erlotinib und Lapatinib und pharmazeutisch unbedenklichen Salzen und/oder Solvaten davon.

11. Spezifischer Integrinligand zur Verwendung nach einem der vorhergehenden Ansprüche, wobei der EGFR-Inhibitor ausgewählt ist aus der Gruppe bestehend aus Cetuximab, Panitumumab, Zalutumumab, Nimotuzumab und Matuzumab und/oder der Gruppe bestehend aus Gefitinib, Erlotinib und Lapatinib, wobei das cytostatische Alkaloid ausgewählt ist aus der Gruppe bestehend aus Vinorelbin und Vincristin und/oder der Gruppe bestehend aus Paclitaxel und Docetaxel und wobei der Antimetabolit ausgewählt ist aus der Gruppe bestehend aus 5-Fluoruracil und Pemetrexed.

12. Spezifischer Integrinligand zur Verwendung nach einem der vorhergehenden Ansprüche, wobei

i) das eine oder die mehreren alkylierende(n) Chemotherapeutika (a) ausgewählt sind aus der Gruppe bestehend aus den platinhaltigen Chemotherapeutika Cisplatin, Carboplatin und Oxaliplatin,
ii) das eine oder die mehreren weitere(n) Chemotherapeutika, außer dem mindestens einen spezifischen Integrinliganden und dem einen oder den mehreren alkylierenden Chemotherapeutika (b) ausgewählt sind aus der Gruppe bestehend aus den biologischen anti-EGFR-Substanzen Cetuximab, Panitumumab, Zalutumumab, Nimotuzumab und Matuzumab, den Antimetaboliten 5-Fluoruracil und Pemetrexed und den Taxanen Docetaxel und Paclitaxel.

13. Spezifischer Integrinligand zur Verwendung nach einem der vorhergehenden Ansprüche, wobei das Cyclo-(Arg-Gly-Asp-DPhe-NMe-Val), die pharmazeutisch unbedenklichen Solvate und/oder Salze davon, kombiniert werden soll mit

i) dem einen oder den mehreren alkylierenden Chemotherapeutika (a) ausgewählt aus der Gruppe bestehend aus den platinhaltigen Chemotherapeutika Cisplatin, Carboplatin und Oxaliplatin, und
ii) dem einen oder den mehreren weiteren Chemotherapeutika, außer dem mindestens einen spezifischen Integrinliganden und dem einen oder den mehreren alkylierenden Chemotherapeutika (b), die enthalten:

$\alpha$) eine oder mehrere biologische anti-EGFR-Substanzen, ausgewählt aus der Gruppe bestehend aus Cetuximab, Panitumumab, Zalutumumab, Nimotuzumab und Matuzumab, und
$\beta$) eine oder mehrere Verbindungen, ausgewählt aus der Gruppe bestehend aus den Antimetaboliten 5-Fluoruracil und Pemetrexed und/oder der Gruppe bestehend aus den Taxanen Docetaxel und Paclitaxel.

14. Spezifischer Integrinligand zur Verwendung nach einem der vorhergehenden Ansprüche, wobei das Cyclo-(Arg-Gly-Asp-DPhe-NMe-Val), die pharmazeutisch unbedenklichen Solvate und/oder Salze davon, einem Patienten in einer Menge von 400 mg bis 6000 mg pro Woche verabreicht werden soll.

15. Spezifischer Integrinligand zur Verwendung nach einem der vorhergehenden Ansprüche, wobei das Cyclo-(Arg-Gly-Asp-DPhe-NMe-Val), die pharmazeutisch unbedenklichen Solvate und/oder Salze davon, einem Patienten in einer Menge von 1500 mg bis 5000 mg pro Woche verabreicht werden soll.

16. Spezifischer Integrinligand zur Verwendung nach einem der vorhergehenden Ansprüche, wobei das Cyclo-(Arg-Gly-Asp-DPhe-NMe-Val), die pharmazeutisch unbedenklichen Solvate und/oder Salze davon, einem Patienten in einem einmal wöchentlichen bis fünfmal wöchentlichen Verabreichungsschema, bestehend aus etwa 500 mg, oder in einem einmal wöchentlichen bis dreimal wöchentlichen Verabreichungsschema, bestehend aus etwa 2000 mg pro Verabreichung, verabreicht werden soll.

17. Spezifischer Integrinligand zur Verwendung nach einem der vorhergehenden Ansprüche, wobei

ii) das eine oder die mehreren alkylierende(n) Chemotherapeutika (a), ausgewählt aus der Gruppe bestehend aus den platinhaltigen Chemotherapeutika Cisplatin, Carboplatin und Oxaliplatin, dem Patienten in einer Menge von 100 bis 1000 mg in einer oder mehreren Portionen innerhalb eines Zeitraums von 2 bis 4 Wochen verabreicht werden sollen, und
iii) das eine oder die mehreren weitere(n) Chemotherapeutika, außer dem mindestens einen spezifischen Integrinliganden und dem einen oder den mehreren alkylierenden Chemotherapeutika (b) enthalten:

α) eine oder mehrere biologische anti-EGFR-Substanzen, ausgewählt aus der Gruppe bestehend aus Cetuximab, Panitumumab, Zalutumumab, Nimotuzumab, und Matuzumab, dem Patienten verabreicht in einer Menge von 200 bis 2000 mg in einer oder mehreren Portionen innerhalb eines Zeitraums von 2 bis 4 Wochen, und gegebenenfalls

β) eine oder mehrere Verbindungen, ausgewählt aus der Gruppe bestehend aus den Antimetaboliten 5-Fluoruracil und Pemetrexed und/oder der Gruppe bestehend aus den Taxanen Paclitaxel und Docetaxel, dem Patienten verabreicht in einer Menge von 150 bis 7500 mg in einer oder mehreren Portionen innerhalb eines Zeitraums von 2 bis 4 Wochen.

18. Spezifischer Integrinligand zur Verwendung nach einem der Ansprüche 9 oder 17, wobei das wöchentliche Verabreichungsschema 1 bis 52 Mal im Wesentlichen ohne Pause angewendet werden soll.

19. Spezifischer Integrinligand zur Verwendung nach einem der Ansprüche 10 oder 18, wobei die Verabreichung an den Patienten in einem Zeitraum von 2 bis 4 Wochen 1 bis 12 Mal im Wesentlichen ohne Pause wiederholt werden soll.

20. Spezifischer Integrinligand zur Verwendung nach einem der vorhergehenden Ansprüche, wobei

a) das wöchentliche Verabreichungsschema, das den spezifischen Integrinliganden betrifft, und
b) die Verabreichung an den Patienten innerhalb eines Zeitraums von 2 bis 4 Wochen, die

i) das eine oder die mehreren alkylierende(n) Chemotherapeutika und/oder
ii) das eine oder die mehreren weitere(n) Chemotherapeutika, außer dem mindestens einen spezifischen Integrinliganden und dem einen oder den mehreren alkylierenden Chemotherapeutika betrifft,

für eine oder mehrere Wochen parallel laufen.

21. Spezifischer Integrinligand, enthaltend Cyclo-(Arg-Gly-Asp-DPhe-NMe-Val) und/oder die pharmazeutisch unbedenklichen Salze davon, zur Verwendung bei der Behandlung von Dickdarm- und/oder Rektumkrebs, wobei der spezifische Integrinligand in Kombination mit

a) einem oder mehreren alkylierenden Chemotherapeutika nach einem der vorhergehenden Ansprüche, und
b) einem oder mehreren weiteren Chemotherapeutika nach einem der vorhergehenden Ansprüche, außer dem mindestens einen spezifischen Integrinliganden und dem einen oder den mehreren alkylierenden Chemotherapeutika;

und Strahlentherapie verwendet werden soll,
wobei das Cyclo-(Arg-Gly-Asp-DPhe-NMe-Val) und/oder die pharmazeutisch unbedenklichen Salze davon einem Patienten in einer Menge von 800 mg bis 7000 mg pro Woche verabreicht werden soll.

## Revendications

1. Ligand d'intégrine spécifique, comprenant cyclo-(Arg-Gly-Asp-DPhe-NMe-Val), ses solvates et/ou sels pharmaceutiquement acceptables, pour utilisation dans le traitement du cancer du côlon et/ou du rectum en combinaison avec

a) un ou plusieurs agent(s) chimiothérapeutique(s) alkylatant(s) choisi(s) parmi le groupe constitué par les composés contenant du platine cisplatine, carboplatine et oxaliplatine ; et
b) un ou plusieurs autre(s) agent(s) chimiothérapeutique(s) autre(s) que l'au moins un ligand d'intégrine spécifique et que les un ou plusieurs agent(s) chimiothérapeutique(s) alkylatant(s), choisi(s) parmi le groupe constitué par :

i) des inhibiteurs d'EGFR choisis parmi le groupe constitué par cetuximab, panitumumab, zalutumumab, nimotuzumab et matuzumab et/ou le groupe constitué par gefitinib, erlotinib et lapatinib,
ii) des alcaloïdes cytostatiques choisis parmi le groupe constitué par etoposide, vinblastine et teniposide, le groupe constitué par vinorelbine, vincristine et vindesine, le groupe constitué par docetaxel et paclitaxel et/ou le groupe constitué par irinotecan et topotecan,
iii) des antibiotiques cytostatiques choisis parmi le groupe constitué par doxorubicine, idarubicine, daunorubicine, epirubicine et valrubicine, et

iv) des antimétabolites choisis parmi le groupe constitué par 5-fluorouracil, capecitabine, cytosinarabinoside et difluoridesoxycytidine et/ou le groupe constitué par pemetrexed, methotrexat et raltitrexed et leurs sels et ou solvates pharmaceutiquement acceptables.

2. Ligand d'intégrine spécifique pour utilisation selon la revendication 1, dans laquelle le cyclo-(Arg-Gly-Asp-DPhe-NMe-Val), ses solvates et/ou sels pharmaceutiquement acceptables, doit être administré à un patient selon une quantité de 250 mg à 12500 mg par semaine.

3. Ligand d'intégrine spécifique pour utilisation selon l'une des revendications précédentes, dans laquelle l'inhibiteur d'EGFR est choisi parmi le groupe constitué par cetuximab, panitumumab, zalutumumab, nimotuzumab et matuzumab et/ou le groupe constitué par gefitinib, erlotinib et lapatinib, l'alcaloïde cytostatique est choisi parmi le groupe constitué par vinorelbine et vincristine et/ou le groupe constitué par paclitaxel et docetaxel, et l'antimétabolite est choisi parmi le groupe constitué par gemcitabine et pemetrexed.

4. Ligand d'intégrine spécifique pour utilisation selon l'une des revendications précédentes, dans laquelle

i) les un ou plusieurs agent(s) chimiothérapeutique(s) alkylatant(s) (a) est/sont choisi(s) parmi le groupe constitué par les agents chimiothérapeutiques contenant du platine cisplatine, carboplatine et oxaliplatine,
ii) les un ou plusieurs autre(s) agent(s) chimiothérapeutique(s) autre(s) que l'au moins un ligand d'intégrine spécifique et les un ou plusieurs agent(s) chimiothérapeutique(s) alkylatant(s) (b) sont choisis parmi le groupe constitué par les produits biologiques anti-EGFR cetuximab, panitumumab, zalutumumab, nimotuzumab et matuzumab et les alcaloïdes vinca vinorelbine et vincristine.

5. Ligand d'intégrine spécifique pour utilisation selon l'une des revendications précédentes, dans laquelle le cyclo-(Arg-Gly-Asp-DPhe-NMe-Val), leurs solvates et/ou sels pharmaceutiquement acceptables doit être combiné avec

i) les un ou plusieurs agent(s) chimiothérapeutique(s) alkylatant(s) (a) choisi(s) parmi le groupe constitué par les agents chimiothérapeutiques contenant du platine cisplatine, carboplatine et oxaliplatine, et
ii) les un ou plusieurs autre(s) agent(s) chimiothérapeutique(s) autre(s) que l'au moins un ligand d'intégrine spécifique et les un ou plusieurs agent(s) chimiothérapeutique(s) alkylatant(s) (b), qui comprennent :

α) un ou plusieurs produit(s) biologique(s) anti-EGFR choisi(s) parmi le groupe constitué par cetuximab, panitumumab, zalutumumab, nimotuzumab et matuzumab, et
β) un ou plusieurs composé(s) choisi(s) parmi le groupe constitué par les alcaloïdes cytostatiques vinorelbine et vincristine.

6. Ligand d'intégrine spécifique pour utilisation selon l'une des revendications précédentes, dans laquelle le cyclo-(Arg-Gly-Asp-DPhe-NMe-Val), ses solvates et/ou sels pharmaceutiquement acceptables, doit être administré à un patient selon une quantité de 400 mg à 6000 mg par semaine.

7. Pur utilisation selon l'une des revendications précédentes, dans laquelle le cyclo-(Arg-Gly-Asp-DPhe-NMe-Val), ses solvates et/ou sels pharmaceutiquement acceptables, doit être administré à un patient selon une quantité de 1500 mg à 5000 mg par semaine.

8. Ligand d'intégrine spécifique pour utilisation selon l'une des revendications précédentes, dans laquelle le cyclo-(Arg-Gly-Asp-DPhe-NMe-Val), ses solvates et/ou sels pharmaceutiquement acceptables, doit être administré à un patient selon un schéma d'administration d'une à trois fois par semaine constitué par environ 500 mg ou environ 2000 mg par administration.

9. Ligand d'intégrine spécifique pour utilisation selon l'une des revendications précédentes, dans laquelle

ii) les un ou plusieurs agent(s) chimiothérapeutique(s) alkylatant(s) (a) choisi(s) parmi le groupe constitué par les agents chimiothérapeutiques contenant du platine cisplatine, carboplatine et oxaliplatine doit/doivent être administré(s) au patient selon une quantité de 100 à 1000 mg selon une ou plusieurs portion(s) dans une période temporelle de 2 à 4 semaines, et
iiii) les un ou plusieurs autre(s) agent(s) chimiothérapeutique(s) autre(s) que l'au moins un ligand d'intégrine spécifique et les un ou plusieurs agent(s) chimiothérapeutique(s) alkylatant(s) (b) comprennent :

α) un ou plusieurs produit(s) biologique(s) anti-EGFR choisis parmi le groupe constitué par cetuximab, panitumumab, zalutumumab, nimotuzumab et matuzumab, administré(s) au patient selon une quantité de 200 à 2000 mg selon une ou plusieurs portion(s) dans une période temporelle de 2 à 4 semaines, et en option

β) un ou plusieurs composé(s) choisi(s) parmi le groupe constitué par les alcaloïdes cytostatiques vinorelbine et vincristine, le groupe constitué par paclitaxel et docetaxel et/ou le groupe constitué par les antimétabolites gemcitabine et pemetrexed, administré(s) au patient selon une quantité de 25 à 6000 mg selon une ou plusieurs portion(s) dans une période temporelle de 2 à 4 semaines.

**10.** Ligand d'intégrine spécifique pour utilisation selon l'une des revendications précédentes, dans laquelle

i) l'agent chimiothérapeutique contenant du platine est choisi parmi le groupe constitué par cisplatine, carboplatine et oxaliplatine,
ii) l'antimétabolite est choisi parmi le groupe constitué par les antifolates methotrexate, raltitrexed et pemetrexed et leurs sels et/ou solvates pharmaceutiquement acceptables, et les antagonistes de pyrimidine 5-fluorouracil, capecitabine, cytosinarabinoside et difluoro-desoxycytidine et leurs sels et/ou solvates pharmaceutiquement acceptables, et
iii) l'alcaloïde cytostatique est choisi parmi le groupe constitué par les alcaloïdes vinca vinblastine, vincristine, vindesine et vinorelbine et leurs sels et/ou solvates pharmaceutiquement acceptables et les taxanes docetaxel et paclitaxel et leurs sels et/ou solvates pharmaceutiquement acceptables, et
iv) l'inhibiteur d'EGFR est choisi parmi le groupe constitué par les produits biologiques anti-EGFR cetuximab, panitumumab, zalutumumab, nimotuzumab et matuzumab et leurs sels et/ou solvates pharmaceutiquement acceptables, et les composés dérivés chimiquement gefitinib, erlotinib et lapatinib et leurs sels et/ou solvates pharmaceutiquement acceptables.

**11.** Ligand d'intégrine spécifique pour utilisation selon l'une des revendications précédentes, dans laquelle l'inhibiteur d'EGFR est choisi parmi le groupe constitué par cetuximab, panitumumab, zalutumumab, nimotuzumab et matuzumab et/ou le groupe constitué par gefitinib, erlotinib et lapatinib, l'alcaloïde cytostatique est choisi parmi le groupe constitué par vinorelbine et vincristine et/ou le groupe constitué par paclitaxel et docetaxel, et l'antimétabolite est choisi parmi le groupe constitué par 5-fluorouracil et pemetrexed.

**12.** Ligand d'intégrine spécifique pour utilisation selon l'une des revendications précédentes, dans laquelle

i) les un ou plusieurs agent(s) chimiothérapeutique(s) alkylatant(s) (a) sont choisis parmi le groupe constitué par les agents chimiothérapeutiques contenant du platine cisplatine, carboplatine et oxaliplatine,
ii) les un ou plusieurs autre(s) agent(s) chimiothérapeutique(s) autre(s) que l'au moins un ligand d'intégrine spécifique et les un ou plusieurs agent(s) chimiothérapeutique(s) alkylatant(s) (b) sont choisis parmi le groupe constitué par les produits biologiques anti-EGFR cetuximab, panitumumab, zalutumumab, nimotuzumab et matuzumab, les antimétabolites 5-fluorouracil et pemetrexed et les taxanes docetaxel et paclitaxel.

**13.** Ligand d'intégrine spécifique pour utilisation selon l'une des revendications précédentes, dans laquelle le cyclo-(Arg-Gly-Asp-DPhe-NMe-Val), ses solvates et/ou sels pharmaceutiquement acceptables, doit être combiné avec

i) les un ou plusieurs agent(s) chimiothérapeutique(s) alkylatant(s) (a) choisi(s) parmi le groupe constitué par les agents chimiothérapeutiques contenant du platine cisplatine, carboplatine et oxaliplatine, et
ii) les un ou plusieurs autre(s) agent(s) chimiothérapeutique(s) autre(s) que l'au moins un ligand d'intégrine spécifique et les un ou plusieurs agent(s) chimiothérapeutique(s) alkylatant(s) (b), qui comprennent :

α) un ou plusieurs produit(s) biologique(s) anti-EGFR choisi(s) parmi le groupe constitué par cetuximab, panitumumab, zalutumumab, nimotuzumab et matuzumab, et
β) un ou plusieurs composé(s) choisi(s) parmi le groupe constitué par les antimétabolites 5-fluorouracil et pemetrexed et/ou le groupe constitué par les taxanes docetaxel et paclitaxel.

**14.** Ligand d'intégrine spécifique pour utilisation selon l'une des revendications précédentes, dans laquelle le cyclo-(Arg-Gly-Asp-DPhe-NMe-Val), ses solvates et/ou sels pharmaceutiquement acceptables, doit être administré à un patient selon une quantité de 400 mg à 6000 mg par semaine.

**15.** Ligand d'intégrine spécifique pour utilisation selon l'une des revendications précédentes, dans laquelle le cyclo-(Arg-Gly-Asp-DPhe-NMe-Val), ses solvates et/ou sels pharmaceutiquement acceptables, doit être administré à un patient selon une quantité de 1500 mg à 5000 mg par semaine.

**16.** Ligand d'intégrine spécifique pour utilisation selon l'une des revendications précédentes, dans laquelle le cyclo-(Arg-Gly-Asp-DPhe-NMe-Val), ses solvates et/ou sels pharmaceutiquement acceptables, doit être administré à un patient selon un schéma d'administration d'une à cinq fois par semaine constitué par environ 500 mg ou selon un schéma d'administration d'une à trois fois par semaine constitué par environ 2000 mg par administration.

**17.** Ligand d'intégrine spécifique pour utilisation selon l'une des revendications précédentes, dans laquelle

ii) les un ou plusieurs agent(s) chimiothérapeutique(s) alkylatant(s) (a) choisi(s) parmi le groupe constitué par les agents chimiothérapeutiques contenant du platine cisplatine, carboplatine et oxaliplatine doit/doivant être administré(s) au patient selon une quantité de 100 à 1000 mg selon une ou plusieurs portion(s) dans une période temporelle de 2 à 4 semaines, et

iiii) les un ou plusieurs autre(s) agent(s) chimiothérapeutique(s) autre(s) que l'au moins un ligand d'intégrine spécifique et les un ou plusieurs agent(s) chimiothérapeutique(s) alkylatant(s) (b) comprennent :

α) un ou plusieurs produit(s) biologique(s) anti-EGFR choisis parmi le groupe constitué par cetuximab, panitumumab, zalutumumab, nimotuzumab et matuzumab, administré(s) au patient selon une quantité de 200 à 2000 mg selon une ou plusieurs portion(s) dans une période temporelle de 2 à 4 semaines, et en option β) un ou plusieurs composé(s) choisi(s) parmi le groupe constitué par les antimétabolites 5-fluorouracil et pemetrexed et/ou le groupe constitué par les taxanes paclitaxel et docetaxel, administré(s) au patient selon une quantité de 150 à 7500 mg selon une ou plusieurs portion(s) dans une période temporelle de 2 à 4 semaines.

**18.** Ligand d'intégrine spécifique pour utilisation selon l'une des revendications 9 ou 17, dans laquelle le schéma d'administration hebdomadaire doit être appliqué de 1 à 52 fois sensiblement sans pause.

**19.** Ligand d'intégrine spécifique pour utilisation selon l'une des revendications 10 ou 18, dans laquelle ladite administration au patient dans une période temporelle de 2 à 4 semaines doit être répétée 1 à 12 fois sensiblement sans pause.

**20.** Ligand d'intégrine spécifique pour utilisation selon l'une des revendications précédentes, dans laquelle

a) le schéma d'administration hebdomadaire concernant le ligand d'intégrine spécifique et
b) l'administration au patient dans une période temporelle de 2 à 4 semaines concernant

i) les un ou plusieurs agent(s) chimiothérapeutique(s) alkylatant(s) et/ou
ii) les un ou plusieurs autre(s) agent(s) chimiothérapeutique(s) autre(s) que l'au moins un ligand d'intégrine spécifique et les un ou plusieurs agent(s) chimiothérapeutique(s) alkylatant(s) fonctionnent en parallèle pendant une ou plusieurs semaines.

**21.** Ligand d'intégrine spécifique, comprenant cyclo-(Arg-Gly-Asp-DPhe-NMe-Val) et/ou ses sels pharmaceutiquement acceptables, pour utilisation dans le traitement du cancer du côlon et/ou du rectum, dans laquelle le ligand d'intégrine spécifique doit être utilisé en combinaison avec

a) un ou plusieurs agent(s) chimiothérapeutique(s) alkylatant(s) selon l'une des revendications précédentes, et
b) un ou plusieurs autre(s) agent(s) chimiothérapeutique(s) selon l'une des revendications précédentes autre(s) que l'au moins un ligand d'intégrine spécifique et que les un ou plusieurs agent(s) chimiothérapeutique(s) alkylatant(s) ;

et la radiothérapie,
dans laquelle le cyclo-(Arg-Gly-Asp-DPhe-NMe-Val) et/ou ses sels pharmaceutiquement acceptables doit être administré à un patient selon une quantité de 800 mg à 7000 mg par semaine.

**Figure 1**

Kaplan Meier survival curves of nude rats bearing orthotopic human glioblastomas U251 derived.

Dark square represents survival of non irradiated animals.

## Figure 2

**Figure 3**

NSCLC (A549)

Squares –    Chemotherapeutic alone
Triangles – Chemotherapeutic plus Cilengitide 6 uM constant

**Figure 4**

Endothelial Cells (HUVEC)

Squares —   Chemotherapeutic alone
Triangles — Chemotherapeutic plus Cilengitide 0.2 uM constant

EP 2 441 464 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4517686 A
- US 4578079 A
- US 4589881 A
- US 4614517 A
- US 4661111 A
- US 4792525 A
- EP 0770622 A
- US 5753230 A
- US 5766591 A
- WO 9532710 A
- WO 9537655 A
- WO 9701540 A
- WO 9737655 A
- WO 9745137 A
- WO 9741844 A
- WO 9808840 A
- WO 9818460 A
- WO 9818461 A
- WO 9825892 A
- WO 9831359 A
- WO 9830542 A
- WO 9915506 A
- WO 9915507 A
- WO 9931061 A
- WO 0006169 A
- EP 0853084 A
- EP 0854140 A
- EP 0854145 A
- US 5780426 A
- US 6048861 A
- WO 9600574 A
- WO 9600730 A
- WO 9606087 A
- WO 9626190 A
- WO 9724119 A
- WO 9724122 A
- WO 9724124 A
- WO 9815278 A
- WO 9905107 A
- WO 9906049 A
- WO 9915170 A
- WO 9915178 A

- WO 9734865 A
- WO 9911626 A
- WO 9915508 A
- WO 9930709 A
- WO 9930713 A
- WO 9931099 A
- WO 0009503 A
- US 5919792 A
- US 5925655 A
- US 5981546 A
- US 6017926 A
- WO 0072801 A
- WO 0038665 A
- WO 9745447 A
- WO 9937683 A
- US 4816567 A
- US 4816397 A, Cabilly
- US 5225539 A, Winter
- US 4342566 A
- WO 9316185 A
- US 5571894 A
- US 5587458 A
- US 4474893 A
- WO 9100360 A
- WO 9205793 A
- WO 9604305 A
- WO 9210209 A
- WO 9311162 A
- WO 9103493 A
- WO 9413804 A
- WO 9850431 A
- US 6001961 A
- WO 0015244 A
- US 0701446 W
- US 6773897 B
- US 5260291 A
- US 5786146 A
- US 6017704 A
- US 6200756 B
- US 6265171 B
- US 5817514 A, Li
- US 5407804 A

**Non-patent literature cited in the description**

- **DAVIS et al.** *J. Cell. Biochem.,* 1993, vol. 51, 206
- **DAVIS et al.** *J. Cell. Biol.,* 1993, vol. 51, 206

- **CHERESH et al.** *Cell,* 1989, vol. 58, 945
- **AUMAILLEY et al.** *FEBS Letts.,* 1991, vol. 291, 50

70

- Isolated liver perfusion. **K. R. AIGNER.** Hepatic Metastases. Butterworth Heinemann, 1981, 101-107
- **HOEKSTRA ; POULTER.** *Curr. Med. Chem.,* 1998, vol. 5, 195
- **KEENAN et al.** *Bioorg. Med. Chem. Lett.,* 1998, vol. 8 (22), 3171
- **WARD et al.** *Drug Metab. Dispos.,* 1999, vol. 27 (11), 1232
- **SMITH et al.** *J. Biol. Chem.,* 1990, vol. 265, 12267
- **BROOKS et al.** *Cell,* 1994, vol. 79, 1157
- **CHOTHIA ; LESK.** *J. Mol. Biol.,* 1987, vol. 196, 901-917
- **KOHLER ; MILSTEIN.** *Nature,* 1975, vol. 256, 495
- Monoclonal Antibody Technology, The Production and Characterization of Rodent and Human Hybridomas. Laboratory Techniques in Biochemistry and Molecular Biology. Elsevier Science Publishers, 1985, vol. 13
- **CLACKSON et al.** *Nature,* 1991, vol. 352, 624-628
- **MARKS et al.** *J. Mol. Biol.,* 1991, vol. 222 (58), 1-597
- **MORRISON et al.** *Proc. Nat. Acad. Sci., USA,* 1984, vol. 81, 6851-6855
- **HERMANSON.** Bioconjugate Techniques. Academic Press, 1996
- **PLÜCKTHUN.** The Pharmacology of Monoclonal Antibodies. Springer-Verlag, 1994, vol. 113, 269-315
- **HUSTON et al.** *Proc. Natl. Acad. Sci.,* 1988, vol. 85, 5879
- **SKERRA ; PLUECKTHUN.** *Science,* 1988, vol. 240, 1038
- **KRANZ et al.** *Proc. Natl. Acad. Sci. USA,* 1981, vol. 78, 5807
- **LANDSCHULZ et al.** *Science,* 1988, vol. 240, 1759
- **MANIATIS ; ABEL.** *Nature,* 1989, vol. 341, 24
- **O'SHEA et al.** *Science,* 1989, vol. 245, 646
- **KARPOVSKY et al.** *J. EXP. Med.,* 1984, vol. 160, 1686
- **LIU.** *Proc. Natl. Acad. Sci. USA,* 1985, vol. 82, 8648
- **PAULUS.** *Behring Inst. Mitt., No. 78,* 1985, 118
- **BRENNAN.** *Science 30 Method,* 1985, vol. 30, 81
- **GLENNIE et al.** *J. Immunol.,* 1987, vol. 139, 2367
- Burger's Medicinal Chemistry And Drug Discovery. Principles and Practice. vol. 1
- **GATZERNEIER.** *ASCO,* 2003
- **GREEN.** *Am J Med,* 1969
- **HEGI et al.** *NEJM,* 2005, vol. 352, 997-1003
- **PALMISANO WA ; DIVINE KK, ; SACCOMANNO G et al.** Predicting lung cancer by detecting aberrant promoter methylation in sputum. *Cancer Res,* 2000, vol. 60, 5954-8
- **DONSON et al.** *Journal Pedriatic Blood Cancer,* 2006
- **AHRENDT et al.** *J. Natl. Cancer Inst.,* 1999, vol. 91, 332-339
- **BELSINKY et al.** *Proc. Natl. Acad. Sci. U.S.A.,* vol. 95, 11891-11896
- **CLARK et al.** *NucleicAcids Res.,* 1994, vol. 22, 2990-2997
- **HERMAN et al.** *Proc Natl Acad Sd U.S.A.,* 1996, vol. 93, 9821-9826
- **XIONG ; LAIRD.** *Nucleic Acids Res.,* 1997, vol. 25, 2532-2534
- **EADS et al.** *Nuc. Acids. Res.,* 2002, vol. 28, 32
- **COTTRELL et al.** *Nucleic Acids Res.,* 2004, vol. 32, 1-8
- **HERMAN et al.** *Proc. Natl. Acad Sci. USA,* 1996, vol. 93 (18), 9821-9826
- **ESTELLER et al.** *Cancer Res.,* 1999, vol. 59, 793-797
- **MYRNES et al.** *Carcinogenesis,* 1984, vol. 5, 1061-1064
- **FUTSCHER et al.** *Cancer Comm.,* 1989, vol. 1, 65-73
- **KREKLAW et al.** *J. Pharmacol. Exper. Ther.,* 2001, vol. 297 (2), 524-530
- **NAGEL et al.** *Anal. Biochem.,* 2003, vol. 321 (1), 38-43
- **ENGEBRAATEN,O. ; HJORTLAND,G.O. ; HIRSCHBERG,H. ; FODSTAD,O.** Growth of precultured human glioma specimens in nude rat brain. *J. Neurosurg.,* 1999, vol. 90, 125-132
- **KIM,J.H. ; KHIL,M.S. ; KOLOZSVARY,A. ; GUTIERREZ,J.A. ; BROWN,S.L.** Fractionated radiosurgery for 9L gliosarcoma in the rat brain. *Int. J. Radiat. Oncol. Biol. Phys.,* 1999, vol. 45, 1035-1040